(19) Europäisches Patentamt
European Patent Office
Office européen des brevets

(11) EP 0 656 950 B1

(12) **EUROPEAN PATENT SPECIFICATION**

(45) Date of publication and mention of the grant of the patent: **04.11.1998 Bulletin 1998/45**

(21) Application number: **93920231.3**

(22) Date of filing: **19.08.1993**

(51) Int Cl.6: **C12N 15/49**, C12N 15/87, C12N 15/37, C12N 15/62, C12N 15/31, C12N 9/02, C12N 9/22, A61K 39/21, C07K 14/00, C12N 5/10, C12N 1/11

(86) International application number: **PCT/US93/07833**

(87) International publication number: **WO 94/04686 (03.03.1994 Gazette 1994/06)**

(54) **TAT-DERIVED TRANSPORT POLYPEPTIDES**

VON TAT ABGELEITETE TRANSPORTPOLYPEPTIDE

POLYPEPTIDES DE TRANSPORT DERIVES DE LA PROTEINE TAT

(84) Designated Contracting States: **AT BE CH DE DK ES FR GB GR IE IT LI LU MC NL PT SE**

(30) Priority: **21.08.1992 US 934375**

(43) Date of publication of application: **14.06.1995 Bulletin 1995/24**

(60) Divisional application: **98105625.2**

(73) Proprietor: **BIOGEN, INC.**
**Cambridge Massachusetts 02142 (US)**

(72) Inventors:
- **BARSOUM, James, G.**
  **Lexington, MA 02173 (US)**
- **FAWELL, Stephen, E.**
  **Winchester, MA 01890 (US)**
- **PEPINSKY, R., Blake**
  **Arlington, MA 02174 (US)**

(74) Representative: **VOSSIUS & PARTNER**
**Postfach 86 07 67**
**81634 München (DE)**

(56) References cited:
**WO-A-91/09958**

- **JOURNAL OF CELLULAR BIOCHEMISTRY vol. SUP 0, no. 17 E , 29 March 1993 page 242 FARHOOD, H. ET AL. 'Regulated gene transfer by co-delivery of a cis-acting DNA element and a trans-acting protein factor to mammalian cells with cationic liposomes'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This application is a continuation-in-part of copending application Serial No. 07/934,375, filed August 21, 1992.

### TECHNICAL FIELD OF THE INVENTION

This invention relates to delivery of biologically active cargo molecules, such as polypeptides and nucleic acids, into the cytoplasm and nuclei of cells in vitro and in vivo. Intracellular delivery of cargo molecules according to this invention is accomplished by the use of novel transport polypeptides which comprise one or more portions of HIV tat protein and which are covalently attached to cargo molecules. The transport polypeptides of this invention are characterized by the presence of the tat basic region (amino acids 49-57), the absence of the tat cysteine-rich region (amino acids 22-36) and the absence of the tat exon 2-encoded carboxy-terminal domain (amino acids 73-86) of the naturally-occurring tat protein. By virtue of the absence of the cysteine-rich region found in conventional tat proteins, the transport polypeptides of this invention solve the problems of spurious trans-activation and disulfide aggregation. The reduced size of the transport polypeptides of this invention also minimizes interference with the biological activity of the cargo molecule.

### BACKGROUND OF THE INVENTION

Biological cells are generally impermeable to macromolecules, including proteins and nucleic acids. Some small molecules enter living cells at very low rates. The lack of means for delivering macromolecules into cells in vivo has been an obstacle to the therapeutic, prophylactic and diagnostic use of a potentially large number of proteins and nucleic acids having intracellular sites of action. Accordingly, most therapeutic, prophylactic and diagnostic candidates produced to date using recombinant DNA technology are polypeptides that act in the extracellular environment or on the target cell surface.

Various methods have been developed for delivering macromolecules into cells in vitro. A list of such methods includes electroporation, membrane fusion with liposomes, high velocity bombardment with DNA-coated microprojectiles, incubation with calcium-phosphate-DNA precipitate, DEAE-dextran mediated transfection, infection with modified viral nucleic acids, and direct micro-injection into single cells. These in vitro methods typically deliver the nucleic acid molecules into only a fraction of the total cell population, and they tend to damage large numbers of cells. Experimental delivery of macromolecules into cells in vivo has been accomplished with scrape loading, calcium phosphate precipitates and liposomes. However, these techniques have, to date, shown limited usefulness for in vivo cellular delivery. Moreover, even with cells in vitro, such methods are of extremely limited usefulness for delivery of proteins.

General methods for efficient delivery of biologically active proteins into intact cells, in vitro and in vivo, are needed. (L.A. Sternson, "Obstacles to Polypeptide Delivery", Ann. N.Y Acad. Sci, 57, pp. 19-21 (1987)). Chemical addition of a lipopeptide (P. Hoffmann et al., "Stimulation of Human and Murine Adherent Cells by Bacterial Lipoprotein and Synthetic Lipopeptide Analogues", Immunobiol., 177, pp. 158-70 (1988)) or a basic polymer such as polylysine or polyarginine (W-C. Chen et al., "Conjugation of Poly-L-Lysine Albumin and Horseradish Peroxidase: A Novel Method of Enhancing the Cellular Uptake of Proteins", Proc. Natl. Acad. Sci. USA, 75, pp. 1872-76 (1978)) have not proved to be highly reliable or generally useful (see Example 4 infra,). Folic acid has been used as a transport moiety (C.P. Leamon and Low, Delivery of Macromolecules into Living Cells: A Method That Exploits Folate Receptor Endocytosis", Proc. Natl. Acad. Sci USA, 88, pp. 5572-76 (1991)). Evidence was presented for internalization of folate conjugates, but not for cytoplasmic delivery. Given the high levels of circulating folate in vivo, the usefulness of this system has not been fully demonstrated. Pseudomonas exotoxin has also been used as a transport moiety (T.I. Prior et al., "Barnase Toxin: A New Chimeric Toxin Composed of Pseudomonas Exotoxin A and Barnase", Cell, 64, pp. 1017-23 (1991)). The efficiency and general applicability of this system is not clear from the published work, however.

The tat protein of human immunodeficiency virus type-1 ("HIV") has demonstrated potential for delivery of cargo proteins into cells (published PCT application WO 91/09958). However, given the chemical properties of the full-length tat protein, generally applicable methods for its efficient use in delivery of biologically active cargo are not taught in the art.

Tat is an HIV-encoded protein that transactivates certain HIV genes and is essential for viral replication. The full-length HIV-1 tat protein has 86 amino acid residues. The HIV tat gene has two exons. Tat amino acids 1-72 are encoded by exon 1, and amino acids 73-86 are encoded by exon 2. The full-length tat protein is characterized by a basic region which contains two lysines and six arginines (amino acids 49-57) and a cysteine-rich region which contains seven cysteine residues (amino acids 22-37). Purified tat protein is taken up from the surrounding medium by human cells growing in culture (A.D. Frankel and C.O. Pabo, "Cellular Uptake of the Tat Protein from Human Immunodeficiency Virus", Cell, 55, pp. 1189-93 (1988)). The art does not teach whether the cysteine-rich region of tat protein (which causes aggregation and insolubility) is required for cellular uptake of tat protein.

PCT patent application WO 91/09958 ("the '958 application") discloses that a heterologous protein consisting of amino acids 1-67 of HIV tat protein genetically fused to a papillomavirus E2 trans-activation repressor polypeptide is taken up by cultured cells. However, preservation of the cargo polypeptide's biological activity (repression of E2 trans-activation) is not demonstrated therein.

The use of tat protein, as taught in the '958 application, potentially involves practical difficulties when used for cellular delivery of cargo proteins. Those practical difficulties include protein aggregation and insolubility involving the cysteine-rich region of tat protein. Furthermore, the '958 application provides no examples of chemical cross-linking of tat protein to cargo proteins, which may be critical in situations where genetic fusion of tat to the cargo protein interferes with proper folding of the tat protein, the cargo protein, or both. In addition, both the '958 application and Frankel and Pabo (supra) teach the use of tat transport proteins in conjunction with chloroquine, which is cytotoxic. The need exists, therefore, for generally applicable means for safe, efficient delivery of biologically active cargo molecules into the cytoplasm and nuclei of living cells.

## SUMMARY OF THE INVENTION

This invention solves the problems set forth above by providing processes and products for the efficient cytoplasmic and nuclear delivery of biologically active non-tat proteins, nucleic acids and other molecules that are (1) not inherently capable of entering target cells or cell nuclei, or (2) not inherently capable of entering target cells at a useful rate. Intracellular delivery of cargo molecules according to this invention is accomplished by the use of novel transport proteins which comprise one or more portions of HIV tat protein and which are covalently attached to the cargo molecules. More particularly, this invention relates to novel transport polypeptides, methods for making those transport polypeptides, transport polypeptide-cargo conjugates, pharmaceutical, prophylactic and diagnostic compositions comprising transport polypeptide-cargo conjugates and methods for delivery of cargo into cells by means of tat-related transport polypeptides.

The transport polypeptides of this invention are characterized by the presence of the tat basic region amino acid sequence (amino acids 49-57 of naturally-occurring tat protein); the absence of the tat cysteine-rich region amino acid sequence (amino acids 22-36 of naturally-occurring tat protein) and the absence of the tat exon 2-encoded carboxy-terminal domain (amino acids 73-86 of naturally-occurring tat protein). Preferred embodiments of such transport polypeptides are: tat37-72 (SEQ ID NO:2), tat37-58 (SEQ ID NO:3), tat38-58GGC (SEQ ID NO:4), tatCGG47-58 (SEQ ID NO:5) tat47-58GGC (SEQ ID NO:6), and tatΔcys (SEQ ID NO:7). It will be recognized by those of ordinary skill in the art that when the transport polypeptide is genetically fused to the cargo moiety, an amino-terminal methionine must be added, but the spacer amino acids (e.g., CysGlyGly or GlyGlyCys) need not be added. By virtue of the absence of the cysteine-rich region present in conventional tat proteins, transport polypeptides of this invention solve the problem of disulfide aggregation, which can result in loss of the cargo's biological activity, insolubility of the transport polypeptide-cargo conjugate, or both. The reduced size of the transport polypeptides of this invention also advantageously minimizes interference with the biological activity of the cargo. A further advantage of the reduced transport polypeptide size is enhanced uptake efficiency in embodiments of this invention involving attachment of multiple transport polypeptides per cargo molecule.

Transport polypeptides of this invention may be advantageously attached to cargo molecules by chemical cross-linking or by genetic fusion. According to preferred embodiments of this invention, the transport polypeptide and the cargo molecule are chemically cross-linked. A unique terminal cysteine residue is a preferred means of chemical cross-linking. According to other preferred embodiments of this invention, the carboxy terminus of the transport moiety is genetically fused to the amino terminus of the cargo moiety. A particularly preferred embodiment of the present invention is JB106, which consists of an amino-terminal methionine followed by tat residues 47-58, followed by HPV-16 E2 residues 245-365.

In many cases, the novel transport polypeptides of this invention advantageously avoid chloroquine-associated toxicity. According to one preferred embodiment of this invention, a biologically active cargo is delivered into the cells of various organs and tissues following introduction of a transport polypeptide-cargo conjugate into a live human or animal. By virtue of the foregoing features, this invention opens the way for biological research and disease therapy involving proteins, nucleic acids and other molecules with cytoplasmic or nuclear sites of action.

## BRIEF DESCRIPTION OF THE DRAWINGS

Figure 1 depicts the amino acid sequence of HIV-1 tat protein (SEQ ID NO:1).

Figure 2 summarizes the results of cellular uptake experiments with transport polypeptide-Pseudomonas exotoxin ribosylation domain conjugates (shaded bars, unconjugated; diagonally-hatched bars, conjugated).

Figure 3 summarizes the results of cellular uptake experiments with transport polypeptide-ribonuclease conjugates (closed squares, ribonuclease-SMCC without transport moiety; closed circles, tat37-72-ribonuclease; closed triangles tat38-58GGC-ribonuclease; closed diamonds, tatCGG38-58-ribonuclease; open squares, tatCGG47-58-ribonuclease).

Figure 4 schematically depicts the construction of plasmid pAHE2.

Figure 5 schematically depicts the construction of plasmid pET8c123.

Figure 6 schematically depicts the construction of plasmid pET8c123CCSS.

Figure 7 summarizes the results of cellular uptake experiments with transport polypeptide-E2 repressor conjugates (open diamonds, E2.123 cross-linked to tat37-72, without chloroquine; closed diamonds, E2.123 cross-linked to tat37-72, with chloroquine; open circles, E2.123CCSS cross-linked to tat37-72, without chloroquine; closed circles, E2.123CCSS cross-linked to tat37-72, with chloroquine).

Figure 8 schematically depicts the construction of plasmid pTATΔcys.

Figure 9 schematically depicts the construction of plasmid pFTE501.

Figure 10 schematically depicts the construction of plasmid pTATΔcys-249.

Figure 11 schematically depicts the construction of plasmid pJB106.

Figure 12 depicts the complete amino acid sequence of protein JB106.

Figure 13 summarizes the results of E2 repression assays involving JB106 (squares), TxHE2CCSS (diamonds) and HE2.123 (circles). The assays were carried out in COS7 cells, without chloroquine, as described in Example 14.

## DETAILED DESCRIPTION OF THE INVENTION

In order that the invention herein described may be more fully understood, the following detailed description is set forth.

In the description, the following terms are employed:

Amino acid -- A monomeric unit of a peptide, polypeptide or protein. The twenty protein amino acids (L-isomers) are: alanine ("Ala" or "A"), arginine ("Arg" or "R"), asparagine ("Asn" or "N"), aspartic acid ("Asp" or "D"), cysteine ("Cys" or "C"), glutamine ("Gln" or "Q"), glutamic acid ("Glu" or "E"), glycine ("Gly" or "G"), histidine ("His" or "H"), isoleucine ("Ile" or "I"), leucine ("Leu" or "L"), lysine ("Lys" or "K"), methionine ("Met" or "M"), phenylalanine ("Phe" or "F"), proline ("Pro" or "P"), serine ("Ser" or "S"), threonine ("Thr" or "T"), tryptophan ("Trp" or "W"), tyrosine ("Tyr" or "Y") and valine ("Val" or "V"). The term amino acid, as used herein, also includes analogs of the protein amino acids, and D-isomers of the protein amino acids and their analogs.

Cargo -- A molecule that is not a tat protein or a fragment thereof, and that is either (1) not inherently capable of entering target cells, or (2) not inherently capable of entering target cells at a useful rate. ("Cargo", as used in this application, refers either to a molecule, per se, i.e., before conjugation, or to the cargo moiety of a transport polypeptide-cargo conjugate.) Examples of "cargo" include, but are not limited to, small molecules and macromolecules, such as polypeptides, nucleic acids and polysaccharides.

Chemical cross-linking -- Covalent bonding of two or more pre-formed molecules.

Cargo conjugate -- A molecule comprising at least one transport polypeptide moiety and at least one cargo moiety, formed either through genetic fusion or chemical cross-linking of a transport polypeptide and a cargo molecule.

Genetic fusion -- Co-linear, covalent linkage of two or more proteins via their polypeptide backbones, through genetic expression of contiguous DNA sequences encoding the proteins.

Macromolecule -- A molecule, such as a peptide, polypeptide, protein or nucleic acid.

Polypeptide -- Any polymer consisting essentially of any of the 20 protein amino acids (above), regardless of its size. Although "protein" is often used in reference to relatively large polypeptides, and "peptide" is often used in reference to small polypeptides, usage of these terms in the art overlaps and varies. The term "polypeptide" as used herein refers to peptides, polypeptides and proteins, unless otherwise noted.

Reporter gene -- A gene the expression of which depends on the occurrence of a cellular event of interest, and the expression of which can be conveniently observed in a genetically transformed host cell.

Reporter plasmid -- A plasmid vector comprising one or more reporter genes.

Small molecule -- A molecule other than a macromolecule.

Spacer amino acid -- An amino acid (preferably having a small side chain) included between a transport moiety and an amino acid residue used for chemical cross-linking (e.g., to provide molecular flexibility and avoid steric hindrance).

Target cell -- A cell into which a cargo is delivered by a transport polypeptide. A "target cell" may be any cell, including human cells, either in vivo or in vitro.

Transport moiety or transport polypeptide -- A polypeptide capable of delivering a covalently attached cargo into a target cell.

This invention is generally applicable for therapeutic, prophylactic or diagnostic intracellular delivery of small molecules and macromolecules, such as proteins, nucleic acids and polysaccharides, that are not inherently capable of entering target cells at a useful rate. It should be appreciated, however, that alternate embodiments of this invention are not limited to clinical applications. This invention may be advantageously applied in medical and biological research. In research applications of this invention, the cargo may be a drug or a reporter molecule. Transport polypeptides of this invention may be used as research laboratory reagents, either alone or as part of a transport polypeptide conjugation kit.

The target cells may be in vivo cells, i.e., cells composing the organs or tissues of living animals or humans, or microorganisms found in living animals or humans. The target cells may also be in vitro cells, i.e., cultured animal cells, human cells or microorganisms.

Wide latitude exists in the selection of drugs and reporter molecules for use in the practice of this invention. Factors to be considered in selecting reporter molecules include, but are not limited to, the type of experimental information sought, non-toxicity, convenience of detection, quantifiability of detection, and availability. Many such reporter molecules are known to those skilled in the art.

As will be appreciated from the examples presented below, we have used enzymes for which colorimetric assays exist, as model cargo to demonstrate the operability and useful features of the transport polypeptides of this invention. These enzyme cargos provide for sensitive, convenient, visual detection of cellular uptake. Furthermore, since visual readout occurs only if the enzymatic activity of the cargo is preserved, these enzymes provide a sensitive and reliable test for preservation of biological activity of the cargo moiety in transport polypeptide-cargo conjugates according to this invention. A preferred embodiment of this invention comprises horseradish peroxidase ("HRP") as the cargo moiety of the transport polypeptide-cargo conjugate. A particularly preferred model cargo moiety for practice of this invention is β-galactosidase.

Model cargo proteins may also be selected according to their site of action within the cell. As described in Examples 6 and 7, below, we have used the ADP ribosylation domain from Pseudomonas exotoxin ("PE") and pancreatic ribonuclease to confirm cytoplasmic delivery of a properly folded cargo proteins by transport polypeptides according to this invention.

Full-length Pseudomonas exotoxin is itself capable of entering cells, where it inactivates ribosomes by means of an ADP ribosylation reaction, thus killing the cells. A portion of the Pseudomonas exotoxin protein known as the ADP ribosylation domain is incapable of entering cells, but it retains the ability to inactivate ribosomes if brought into contact with them. Thus, cell death induced by transport polypeptide-PE ADP ribosylation domain conjugates is a test for cytoplasmic delivery of the cargo by the transport polypeptide.

We have also used ribonuclease to confirm cytoplasmic delivery of a properly folded cargo protein by transport polypeptides of this invention. Protein synthesis, an RNA-dependent process, is highly sensitive to ribonuclease, which digests RNA. Ribonuclease is, by itself, incapable of entering cells, however. Thus, inhibition of protein synthesis by a transport polypeptide-ribonuclease conjugate is a test for intracellular delivery of biologically active ribonuclease.

Of course, delivery of a given cargo molecule to the cytoplasm may be followed by further delivery of the same cargo molecule to the nucleus. Nuclear delivery necessarily involves traversing some portion of the cytoplasm.

Papillomavirus E2 repressor proteins are examples of macromolecular drugs that may be delivered into the nuclei of target cells by the transport polypeptides of this invention. Papillomavirus E2 protein, which normally exists as a homodimer, regulates both transcription and replication of the papillomavirus genome. The carboxy-terminal domain of the E2 protein contains DNA binding and dimerization activities. Transient expression of DNA sequences encoding various E2 analogs or E2 carboxy-terminal fragments in transfected mammalian cells inhibits trans-activation by the full-length E2 protein (J. Barsoum et al., "Mechanism of Action of the Papillomavirus E2 Repressor: Repression in the Absence of DNA Binding", J. Virol., 66, pp. 3941-3945 (1992)). E2 repressors added to the growth medium of cultured mammalian cells do not enter the cells, and thus do not inhibit E2 trans-activation in those cells. However, conjugation of the transport polypeptides of this invention to E2 repressors results in translocation of the E2 repressors from the growth medium into the cultured cells, where they display biological activity, repressing E2-dependent expression of a reporter gene.

The rate at which single-stranded and double-stranded nucleic acids enter cells, in vitro and in vivo, may be advantageously enhanced, using the transport polypeptides of this invention. As shown in Example 11 (below), methods for chemical cross-linking of polypeptides to nucleic acids are well known in the art. In a preferred embodiment of this invention, the cargo is a single-stranded antisense nucleic acid. Antisense nucleic acids are useful for inhibiting cellular expression of sequences to which they are complementary. In another embodiment of this invention, the cargo is a double-stranded nucleic acid comprising a binding site recognized by a nucleic acid-binding protein. An example of such a nucleic acid-binding protein is a viral trans-activator.

Naturally-occurring HIV-1 tat protein (Figure 1) has a region (amino acids 22-37) wherein 7 out of 16 amino acids are cysteine. Those cysteine residues are capable of forming disulfide bonds with each other, with cysteine residues in the cysteine-rich region of other tat protein molecules and with cysteine residues in a cargo protein or the cargo moiety of a conjugate. Such disulfide bond formation can cause loss of the cargo's biological activity. Furthermore, even if there is no potential for disulfide bonding to the cargo moiety (for example, when the cargo protein has no cysteine residues), disulfide bond formation between transport polypeptides leads to aggregation and insolubility of the transport polypeptide, the transport polypeptide-cargo conjugate, or both. The tat cysteine-rich region is potentially a source of serious problems in the use of naturally-occurring tat protein for cellular delivery of cargo molecules.

The cysteine-rich region is required for dimerization of tat in vitro, and is required for trans-activation of HIV DNA sequences. Therefore, removal of the tat cysteine-rich region has the additional advantage of eliminating the natural activity of tat, i.e., induction of HIV transcription and replication. However, the art does not teach whether the cysteine-rich region of the tat protein is required for cellular uptake.

The present invention includes embodiments wherein the problems associated with the tat cysteine-rich region are solved, because that region is not present in the transport polypeptides described herein. In those embodiments, cellular uptake of the transport polypeptide or transport polypeptide-cargo molecule conjugate still occurs. In one group of preferred embodiments of this invention, the sequence of amino acids preceding the cysteine-rich region is fused directly to the sequence of amino acids following the cysteine-rich region. Such transport polypeptides are called tat–Δcys, and have the general formula (tat1-21)-(tat38-n), where n is the number of the carboxy-terminal residue, i.e., 49-86. Preferably, n is 58-72. As will be appreciated from the examples below, the amino acid sequence preceding the cysteine-rich region of the tat protein is not required for cellular uptake. A preferred transport polypeptide (or transport moiety) consists of amino acids 37-72 of tat protein, and is called tat37-72 (SEQ ID NO:2). Retention of tat residue 37, a cysteine, at the amino terminus of the transport polypeptide is preferred, because it is useful for chemical cross-linking.

The advantages of the tatΔcys polypeptides, tat37-72 and other embodiments of this invention include the following:

a) The natural activity of tat protein, i.e., induction of HIV transcription, is eliminated;
b) Dimers, and higher multimers of the transport polypeptide are avoided;
c) The level of expression of tatΔcys genetic fusions in E.coli may be improved;
d) Some transport polypeptide conjugates display increased solubility and superior ease of handling; and
e) Some fusion proteins display increased activity by the cargo moiety, as compared with fusions containing the cysteine-rich region.

Numerous chemical cross-linking methods are known and potentially applicable for conjugating the transport polypeptides of this invention to cargo macromolecules. Many known chemical cross-linking methods are non-specific, i.e., they do not direct the point of coupling to any particular site on the transport polypeptide or cargo macromolecule. As a result, use of non-specific cross-linking agents may attack functional sites or sterically block active sites, rendering the conjugated proteins biologically inactive.

A preferred approach to increasing coupling specificity in the practice of this invention is direct chemical coupling to a functional group found only once or a few times in one or both of the polypeptides to be cross-linked. For example, in many proteins, cysteine, which is the only protein amino acid containing a thiol group, occurs only a few times. Also, for example, if a polypeptide contains no lysine residues, a cross-linking reagent specific for primary amines will be selective for the amino terminus of that polypeptide. Successful utilization of this approach to increase coupling specificity requires that the polypeptide have the suitably rare and reactive residues in areas of the molecule that may be altered without loss of the molecule's biological activity.

As demonstrated in the examples below, cysteine residues may be replaced when they occur in parts of a polypeptide sequence where their participation in a cross-linking reaction would likely interfere with biological activity. When a cysteine residue is replaced, it is typically desirable to minimize resulting changes in polypeptide folding. Changes in polypeptide folding are minimized when the replacement is chemically and sterically similar to cysteine. For these reasons, serine is preferred as a replacement for cysteine. As demonstrated in the examples below, a cysteine residue may be introduced into a polypeptide's amino acid sequence for cross-linking purposes. When a cysteine residue is introduced, introduction at or near the amino or carboxy terminus is preferred. Conventional methods are available for such amino acid sequence modifications, whether the polypeptide of interest is produced by chemical synthesis or expression of recombinant DNA.

Cross-linking reagents may be homobifunctional, i.e., having two functional groups that undergo the same reaction. A preferred homobifunctional cross-linking reagent is bismaleimidohexane ("BMH"). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of polypeptides that contain cysteine residues.

Cross-linking reagents may also be heterobifunctional. Heterobifunctional cross-linking agents have two different functional groups, for example an amine-reactive group and a thiol-reactive group, that will cross-link two proteins having free amines and thiols, respectively. Examples of heterobifunctional cross-linking agents are succinimidyl 4-(N-maleimidomethyl)cyclohexane-1-carboxylate ("SMCC"), m-maleimidobenzoyl-N-hydroxysuccinimide ester ("MBS"), and succinimide 4-(p-maleimidophenyl)butyrate ("SMPB"), an extended chain analog of MBS. The succinimidyl group of these crosslinkers reacts with a primary amine, and the thiol-reactive maleimide, forms a covalent bond with the thiol of a cysteine residue.

Cross-linking reagents often have low solubility in water. A hydrophilic moiety, such as a sulfonate group, may be added to the cross-linking reagent to improve its water solubility. Sulfo-MBS and sulfo-SMCC are examples of cross-linking reagents modified for water solubility.

Many cross-linking reagents yield a conjugate that is essentially non-cleavable under cellular conditions. However, some cross-linking reagents contain a covalent bond, such as a disulfide, that is cleavable under cellular conditions. For example, dithiobis(succinimidylpropionate) ("DSP"), Traut's reagent and N-succinimidyl 3-(2-pyridyldithio) propionate ("SPDP") are well-known cleavable crosslinkers. The use of a cleavable cross-linking reagent permits the cargo moiety to separate from the transport polypeptide after delivery into the target cell. Direct disulfide linkage may also be useful.

Some new cross-linking reagents such as n-γ-maleimidobutyryloxy-succinimide ester ("GMBS") and sulfo-GMBS, have reduced immunogenicity. In some embodiments of the present invention, such reduced immunogenicity may be advantageous.

Numerous cross-linking reagents, including the ones discussed above, are commercially available. Detailed instructions for their use are readily available from the commercial suppliers. A general reference on protein cross-linking and conjugate preparation is: S.S. Wong, Chemistry of Protein Conjugation and Cross-Linking, CRC Press (1991).

Chemical cross-linking may include the use of spacer arms. Spacer arms provide intramolecular flexibility or adjust intramolecular distances between conjugated moieties and thereby may help preserve biological activity. A spacer arm may be in the form of a polypeptide moiety comprising spacer amino acids. Alternatively, a spacer arm may be part of the cross-linking reagent, such as in "long-chain SPDP" (Pierce Chem. Co., Rockford, IL, cat. No. 21651 H).

The pharmaceutical compositions of this invention may be for therapeutic, prophylactic or diagnostic applications, and may be in a variety of forms. These include, for example, solid, semi-solid, and liquid dosage forms, such as tablets, pills, powders, liquid solutions or suspensions, aerosols, liposomes, suppositories, injectable and infusible solutions and sustained release forms. The preferred form depends on the intended mode of administration and the therapeutic, prophylactic or diagnostic application. The transport polypeptide-cargo molecule conjugates of this invention may be administered by conventional routes of administration, such as parenteral, subcutaneous, intravenous, intramuscular, intralesional or aerosol routes. The compositions also preferably include conventional pharmaceutically acceptable carriers and adjuvants that are known to those of skill in the art.

Generally, the pharmaceutical compositions of the present invention may be formulated and administered using methods and compositions similar to those used for pharmaceutically important polypeptides such as, for example, alpha interferon. It will be understood that conventional doses will vary depending upon the particular cargo involved.

The processes and compositions of this invention may be applied to any organism, including humans. The processes and compositions of this invention may also be applied to animals and humans in utero.

For many pharmaceutical applications of this invention, it is necessary for the cargo molecule to be translocated from body fluids into cells of tissues in the body, rather than from a growth medium into cultured cells. Therefore, in addition to examples below involving cultured cells, we have provided examples demonstrating delivery of model cargo proteins into cells of various mammalian organs and tissues, following intravenous injection of transport polypeptide-cargo protein conjugates into live animals. These cargo proteins display biological activity following delivery into the cells in vivo.

As demonstrated in the examples that follow, using the amino acid and DNA sequence information provided herein, the transport polypeptides of this invention may be chemically synthesized or produced by recombinant DNA methods. Methods for chemical synthesis or recombinant DNA production of polypeptides having a known amino acid sequence are well known. Automated equipment for polypeptide or DNA synthesis is commercially available. Host cells, cloning vectors, DNA expression control sequences and oligonucleotide linkers are also commercially available.

Using well-known techniques, one of skill in the art can readily make minor additions, deletions or substitutions in the preferred transport polypeptide amino acid sequences set forth herein. It should be understood, however, that such variations are within the scope of this invention.

Furthermore, tat proteins from other viruses, such as HIV-2 (M. Guyader et al., "Genome Organization and Transactivation of the Human Immunodeficiency Virus Type 2", Nature, 326, pp. 662-669 (1987)), equine infectious anemia virus (R. Carroll et al., "Identification of Lentivirus Tat Functional Domains Through Generation of Equine Infectious Anemia Virus/Human Immunodeficiency Virus Type 1 tat Gene Chimeras", J. Virol., 65, pp. 3460-67 (1991)), and simian immunodeficiency virus (L. Chakrabarti et al., "Sequence of Simian Immunodeficiency Virus from Macaque and Its Relationship to Other Human and Simian Retroviruses", Nature, 328, pp. 543-47 (1987); S.K. Arya et al., "New Human and Simian HIV-Related Retroviruses Possess Functional Transactivator (tat) Gene", Nature, 328, pp. 548-550 (1987)) are known. It should be understood that polypeptides derived from those tat proteins and characterized by the presence of the tat basic region and the absence of the tat cysteine-rich region fall within the scope of the present invention.

In order that the invention described herein may be more fully understood, the following examples are set forth. It should be understood that these examples are for illustrative purposes only and are not to be construed as limiting this invention in any manner. Throughout these examples, all molecular cloning reactions were carried out according to methods in J. Sambrook et al., Molecular Cloning: A Laboratory Manual, 2nd Edition, Cold Spring Harbor Laboratory (1989), except where otherwise noted.

EXAMPLE 1

Production and Purification of Transport Polypeptides

Recombinant DNA

Plasmid pTat72 was a starting clone for bacterial production of tat-derived transport polypeptides and construction of genes encoding transport polypeptide-cargo protein fusions. We obtained plasmid pTat72 (described in Frankel and Pabo, supra) from Alan Frankel (The Whitehead Institute for Biomedical Research, Cambridge, MA). Plasmid pTat72, was derived from the pET-3a expression vector of F.W. Studier et al. ("Use of T7 RNA Polymerase to Direct Expression of Cloned Genes", Methods Enzymol., 185, pp. 60-90 (1990)) by insertion of a synthetic gene encoding amino acids 1 to 72 of HIV-1 tat. The tat coding region employs E.coli codon usage and is driven by the bacteriophage T7 polymerase promoter inducible with isopropyl beta-D-thiogalactopyranoside ("IPTG"). Tat protein constituted 5% of total E.coli protein after IPTG induction.

Purification of Tat1-72 from Bacteria

We suspended E.coli expressing tat1-72 protein in 10 volumes of 25 mM Tris-HCl (pH 7.5), 1 mM EDTA. We lysed the cells in a French press and removed the insoluble debris by centrifugation at 10,000 x g for 1 hour. We loaded the supernatant onto a Q Sepharose Fast Flow (Pharmacia LKB, Piscataway, NJ) ion exchange column (20 ml resin/60 ml lysate). We treated the flow-through fraction with 0.5 M NaCl, which caused the tat protein to precipitate. We collected the salt-precipitated protein by centrifugation at 35,000 rpm, in a 50.2 rotor, for 1 hour. We dissolved the pelleted precipitate in 6 M guanidine-HCl and clarified the solution by centrifugation at 35,000 rpm, in a 50.2 rotor, for 1 hour. We loaded the clarified sample onto an A.5 agarose gel filtration column equilibrated with 6 M guanidine-HCl, 50 mM sodium phosphate (pH 5.4), 10 mM DTT, and then eluted the sample with the same buffer. We loaded the tat protein-contain gel filtration fractions onto a $C_4$ reverse phase HPLC column and eluted with a gradient of 0-75% acetonitrile, 0.1% trifluoroacetic acid. Using this procedure, we produced about 20 mg of tat1-72 protein per liter of E.coli culture (assuming 6 g of cells per liter). This represented an overall yield of about 50%.

Upon SDS-PAGE analysis, the tat1-72 polypeptide migrated as a single band of 10 kD. The purified tat1-72 polypeptide was active in an uptake/transactivation assay. We added the polypeptide to the culture medium of human hepatoma cells containing a tat-responsive tissue plasminogen activator ("tPA") reporter gene. In the presence of 0.1 mM chloroquine, the purified tat1-72 protein (100 ng/ml) induced tPA expression approximately 150-fold.

Chemical Synthesis of Transport Polypeptides

For chemical synthesis of the various transport polypeptides, we used a commercially-available, automated system (Applied Biosystems Model 430A synthesizer) and followed the system manufacturer's recommended procedures. We removed blocking groups by HF treatment and isolated the synthetic polypeptides by conventional reverse phase HPLC methods. The integrity of all synthetic polypeptides was confirmed by mass spectrometer analysis.

EXAMPLE 2

β-Galactosidase Conjugates

Chemical Cross-Linking with SMCC

For acetylation of β-galactosidase (to block cysteine sulfhydryl groups) we dissolved 6.4 mg of commercially obtained β-galactosidase (Pierce Chem. Co., cat. no. 32101G) in 200 μl of 50 mM phosphate buffer (pH 7.5). To the 200 μl of β-galactosidase solution, we added 10 μl of iodoacetic acid, prepared by dissolving 30 mg of iodoacetic acid in 4 ml of 50 mM phosphate buffer (pH 7.5). (In subsequent experiments we found iodoacetamide to be a preferable substitute for iodoacetic acid.) We allowed the reaction to proceed for 60 minutes at room temperature. We then separated the acetylated β-galactosidase from the unreacted iodoacetic acid by loading the reaction (Pharmacia) mixture on a small G-25 (Pharmacia LKB, Piscataway, NJ) gel filtration column and collecting the void volume.

Prior to SMCC activation of the amine groups of the acetylated β-galactosidase, we concentrated 2 ml of the enzyme collected from the G-25 column to 0.3 ml in a Centricon 10 (Amicon, Danvers, MA) ultrafiltration apparatus.

To the concentrated acetylated β-galactosidase, we added 19 μg of sulfo-SMCC (Pierce Chem. Co., cat. no. 22322G) dissolved in 15 μl of dimethylformamide ("DMF"). We allowed the reaction to proceed for 30 minutes at room temperature. We then separated the β-galactosidase-SMCC from the DMF and unreacted SMCC by passage over a small G-25 gel filtration column.

For chemical cross-linking of transport polypeptides to β-galactosidase, we mixed the solution of β-galactosidase-SMCC with 100 μg of transport polypeptide (tat1-72, tat37-72, tat38-58GGC, tat37-58, tat47-58GGC or tatCGG47-58) dissolved in 200 μl of 50 mM phosphate buffer (pH 7.5). We allowed the reaction to proceed for 60 minutes at room temperature. We then isolated the transport polypeptide-β-galactosidase conjugate by loading the reaction mixture on an S-200HR gel filtration column and collecting the void volume.

The transport polypeptide-β-galactosidase conjugate thus obtained yielded positive results when assayed for tat in conventional Western blot and ELISA analyses performed with rabbit anti-tat polyclonal antibodies. For a general discussion of Western blot and ELISA analysis, see E. Harlow and D. Lane, Antibodies: A Laboratory Manual, Cold Spring Harbor Laboratory (1988). Gel filtration analysis with Superose 6 (Pharmacia LKB, Piscataway, NJ) indicated the transport polypeptide-β-galactosidase conjugate to have a molecular weight of about 540,000 daltons. Specific activity of the transport polypeptide-β-galactosidase conjugate was 52% of the specific activity of the β-galactosidase starting material, when assayed with o-nitrophenyl-β-D-galactopyranoside ("ONPG"). The ONPG assay procedure is described in detail at pages 16.66-16.67 of Sambrook et al. (supra).

Cellular Uptake of β-Galactosidase Conjugates

We added the conjugates to the medium of HeLa cells (ATCC no. CCL2) at 20 μg/ml, in the presence or absence of 100 μM chloroquine. We incubated the cells for 4-18 hours at 37°C/5.5% $CO_2$. We fixed the cells with 2% formaldehyde, 0.2% glutaraldehyde in phosphate-buffered saline ("PBS") for 5 minutes at 4°C. We then washed the cells three times with 2 mM $MgCl_2$ in PBS, and stained them with X-gal, at 37°C. X-gal is a colorless β-galactosidase substrate (5-bromo-4-chloro-3-indolyl D-galactoside) that yields a blue product upon cleavage by β-galactosidase. Our X-gal staining solution contained 1 mg of X-gal (Bio-Rad, Richmond, CA, cat. no. 170-3455) per ml of PBS containing 5 mM potassium ferricyanide, 5 mM potassium ferrocyanide and 2 mM $MgCl_2$.

We subjected the stained cells to microscopic examination at magnifications up to 400 X. Such microscopic examination revealed nuclear staining, as well as cytoplasmic staining.

The cells to which the tat37-72-β-galactosidase conjugate or tat1-72-β-galactosidase conjugate was added stained dark blue. β-galactosidase activity could be seen after a development time as short as 15 minutes. For comparison, it should be noted that stain development time of at least 6 hours is normally required when β-galactosidase activity is introduced into cells by means of transfection of the β-galactosidase gene. Nuclear staining was visible in the absence of chloroquine, although the nuclear staining intensity was slightly greater in chloroquine-treated cells. Control cells treated with unconjugated β-galactosidase showed no detectable staining.

Cleavable Conjugation by Direct Disulfide

Each β-galactosidase tetramer has 12 cysteine residues that may be used for direct disulfide linkage to a transport polypeptide cysteine residue. To reduce and then protect the sulfhydryl of tat37-72, we dissolved 1.8 mg (411 nmoles) of tat37-72 in 1 ml of 50 mM sodium phosphate (pH 8.0), 150 mM NaCl, 2mM EDTA, and applied the solution to a Reduce-Imm column (Pierce Chem. Co., Rockford, IL). After 30 minutes at room temperature, we eluted the tat37-72 from the column with 1 ml aliquots of the same buffer, into tubes containing 0.1 ml of 10 mM 5,5'-dithio-bis(2-nitrobenzoic acid) ("DTNB"). We left the reduced tat37-72 polypeptide in the presence of the DTNB for 3 hours. We then removed the unreacted DTNB from the tat37-72-TNB by gel filtration on a 9 ml Sephadex G-10 column (Pharmacia LKB, Piscataway, NJ). We dissolved 5 mg β-galactosidase in 0.5 ml of buffer and desalted it on a 9 ml Sephadex G-25 column (Pharmacia LKB, Piscataway, NJ), to obtain 3.8 mg of β-galactosidase/ml buffer. We mixed 0.5 ml aliquots of desalted β-galactosidase solution with 0.25 or 0.5 ml of the tat37-72-TNB preparation, and allowed the direct disulfide cross-linking reaction to proceed at room temperature for 30 minutes. We removed the unreacted tat37-72-TNB from the β-galactosidase conjugate by gel filtration on a 9 ml Sephacryl S-200 column. We monitored the extent of the cross-linking reaction indirectly, by measuring absorbance at 412 nm due to the released TNB. The direct disulfide conjugates thus produced were taken up into cells (data not shown).

Cleavable Conjugation with SPDP

We used the heterobifunctional cross-linking reagent ("SPDP"), which contains a cleavable disulfide bond, to form a cross-link between: (1) the primary amine groups of β-galactosidase and the cysteine sulfhydryls of tat1-72 (metabolically labelled with $^{35}S$) ; or (2) the primary amine groups of rhodamine-labelled β-galactosidase and the amino terminal cysteine sulfhydryl of tat37-72.

For the tat1-72 conjugation, we dissolved 5 mg of β-galactosidase in 0.5 ml of 50 mM sodium phosphate (pH 7.5), 150 mM NaCl, 2 mM $MgCl_2$, and desalted the β-galactosidase on a 9 ml Sephadex G-25 column (Pharmacia LKB, Piscataway, NJ). We treated the desalted β-galactosidase with an 88-fold molar excess of iodoacetamide at room temperature for 2 hours, to block free sulfhydryl groups. After removing the unreacted iodoacetamide by gel filtration, we treated the blocked β-galactosidase with a 10-fold molar excess of SPDP at room temperature. After 2 hours, we exchanged the buffer, by ultrafiltration (Ultrafree 30, Millipore, Bedford, MA). We then added a 4-fold molar excess of labelled tat1-72, and allowed the cross-linking reaction to proceed overnight, at room temperature. We removed the unreacted tat1-72 by gel filtration on a 9 ml Sephacryl S-200 column. Using the known specific activity of the labelled tat1-72, we calculated that there were 1.1 tat1-72 polypeptides cross-linked per β-galactosidase tetramer. Using the ONPG assay, we found that the conjugated β-galactosidase retained 100% of its enzymatic activity. Using measurement of cell-incorporated radioactivity and X-gal staining, we demonstrated uptake of the conjugate into cultured HeLa cells.

For the tat37-72 conjugation, our procedure was as described in the preceding paragraph, except that we labelled the β-galactosidase with a 5:1 molar ratio of rhodamine maleimide at room temperature for 1 hour, prior to the iodoacetamide treatment (100:1 iodoacetamide molar excess). In the cross-linking reaction, we used an SPDP ratio of 20: 1, and a tat37-72 ratio of 10:1. We estimated the conjugated product to have about 5 rhodamine molecules (according to UV absorbance) and about 2 tat37-72 moieties (according to gel filtration) per β-galactosidase tetramer. The conjugate from this procedure retained about 35% of the initial β-galactosidase enzymatic activity. Using X-gal staining and rhodamine fluorescence, we demonstrated that the SPDP conjugate was taken up into cultured HeLa cells.

EXAMPLE 3

Animal Studies with β-Galactosidase Conjugates

For conjugate half-life determination and biodistribution analysis, we injected either 200 μg of SMCC-β-galactosidase (control) or tat1-72-β-galactosidase intravenously ("IV") into the tail veins of Balb/c mice (Jackson Laboratories), with and without chloroquine. We collected blood samples at intervals up to 30 minutes. After 30 minutes, we sacrificed the animals and removed organs and tissues for histochemical analysis.

We measured β-galactosidase activity in blood samples by the ONPG assay. The ONPG assay procedure is described in detail at pages 16.66-16.67 of Sambrook et al. (supra). β-galactosidase and tat1-72-β-galactosidase were rapidly cleared from the bloodstream. We estimated their half-lives at 3-6 minutes. These experimental comparisons indicated that attachment of the tat1-72 transport polypeptide has little or no effect on the clearance rate of β-galactosidase from the blood.

To detect cellular uptake of the transport polypeptide-β-galactosidase conjugates, we prepared thin frozen tissue sections from sacrificed animals (above), carried out fixation as described in Example 2 (above), and subjected them to a standard X-gal staining procedure. Liver, spleen and heart stained intensely. Lung, and skeletal muscle stained less intensely. Brain, pancreas and kidney showed no detectable staining. High power microscopic examination revealed strong cellular, and in some cases, nuclear staining of what appeared to be endothelial cells surrounding the blood supply to the tissues.

EXAMPLE 4

Cellular Uptake Tests with β-Galactosidase-Polyarginine and β-Galactosidase-Polylysine Conjugates

To compare the effectiveness of simple basic amino acid polymers with the effectiveness of our tat-derived transport polypeptides, we conjugated commercially available polyarginine (Sigma Chem Co., St. Louis, MO, cat. no. P-4663) and polylysine (Sigma cat. no. P-2658) to β-galactosidase, as described in Example 2, above. We added the conjugates to the medium of HeLa cells at 1-30 μg/ml, with and without chloroquine. Following incubation with the conjugates, we fixed, stained and microscopically examined the cells as described in Example 2, above.

The polylysine-β-galactosidase conjugate gave low levels of surface staining and no nuclear staining. The polyarginine-β-galactosidase conjugate gave intense overall staining, but showed less nuclear stain than the tati-72-β-galactosidase and tat37-72-β-galactosidase conjugates. To distinguish between cell surface binding and actual internalization of the polyarginine-β-galactosidase conjugate, we treated the cells with trypsin, a protease, prior to the fixing and staining procedures. Trypsin treatment eliminated most of the X-gal staining of polyarginine-β-galactosidase treated cells, indicating that the polyarginine-β-galactosidase conjugate was bound to the outside surfaces of the cells rather than actually internalized. In contrast, cells exposed to the tat1-72 or 37-72-β-galactosidase conjugates stained despite trypsin treatment, indicating that the β-galactosidase cargo was inside the cells and thus protected from trypsin digestion. Control cells treated with unconjugated β-galactosidase showed no detectable staining.

EXAMPLE 5

Horseradish Peroxidase Conjugates

Chemical Cross-Linking

To produce tat1-72-HRP and tat37-72-HRP conjugates, we used a commercially-available HRP coupling kit (Immunopure maleimide activated HRP, Pierce Chem. Co., cat. no. 31498G). The HRP supplied in the kit is in a form that is selectively reactive toward free -SH groups. (Cysteine is the only one of the 20 protein amino acids having a free -SH group.) In a transport polypeptide-HRP conjugation experiment involving tat1-72, we produced the tat1-72 starting material in E.coli and purified it by HPLC, as described in Example 1, above. We lyophilized 200 μg of the purified tat1-72 (which was dissolved in TFA/acetonitrile) and redissolved it in 100 μl of 100 mM HEPES buffer (pH7.5), 0.5 mM EDTA. We added 50 μl of the tat1-72 or tat37-72 solution to 50 μl of Immunopure HRP (750 μg of the enzyme) in 250 mM triethanolamine (pH 8.2). We allowed the reaction to proceed for 80 minutes, at room temperature. Under these conditions, approximately 70% of the HRP was chemically linked to tat1-72 molecules. We monitored the extent of the linking reaction by SDS-PAGE analysis.

Cellular Uptake of HRP Conjugates

We added the conjugates to the medium of HeLa cells at 20 μg/ml, in the presence or absence of 100 μM chloroquine. We incubated the cells for 4-18 hours at 37°C/5.5% $CO_2$. We developed the HRP stain using 4-chloro-1-naphthol (Bio-Rad, Richmond, CA, cat. no. 170-6431) and hydrogen peroxide HRP substrate. In subsequent experiments, we substituted diaminobenzidine (Sigma Chem. Co., St. Louis, MO) for 4-chloro-1-naphthol.

Cells to which we added transport polypeptide-HRP conjugates displayed cell-associated HRP activity. Short time periods of conjugate exposure resulted in staining patterns which appeared punctate, probably reflecting HRP in endocytic vesicles. Following longer incubations, we observed diffuse nuclear and cytoplasmic staining. Control cells treated with unconjugated HRP showed no detectable staining.

EXAMPLE 6

PE ADP Ribosylation Domain Conjugates

We cloned and expressed in E.coli the Pseudomonas exotoxin ("PE") both in its full length form and in the form of its ADP ribosylation domain. We produced transport polypeptide-PE conjugates both by genetic fusion and chemical cross-linking.

Plasmid Construction

To construct plasmid pTat70(ApaI), we inserted a unique ApaI site into the tat open reading frame by digesting pTat72 with BamH1 and EcoR1, and inserting a double-stranded linker consisting of the following synthetic oligonucleotides:

GATCCCAGAC CCACCAGGTT TCTCTGTCGG GCCCTTAAG (SEQ ID NO:8)

AATTCTTAAG GGCCCGACAG AGAAACCTGG TGGGTCTGG (SEQ ID NO:9).

The linker replaced the C-terminus of tat, LysGlnStop, with GlyProStop. The linker also added a unique ApaI site suitable for in-frame fusion of the tat sequence with the PE ADP ribosylation domain-encoding sequences, by means of the naturally-occurring ApaI site in the PE sequence. To construct plasmid pTat70PE (SEQ ID NO:10), we removed an ApaI-EcoRI fragment encoding the PE ADP ribosylation domain, from plasmid CD4(181)-PE(392). The construction of CD4(181)-PE(392) is described by G. Winkler et al. ("CD4-Pseudomonas-Exotoxin Hybrid Proteins: Modulation of Potency and Therapeutic Window Through Structural Design and Characterization of Cell Internalization", AIDS Research and Human Retroviruses, 7, pp. 393-401 (1991)). We inserted the ApaI-EcoRI fragment into pTat70(ApaI) digested with ApaI and EcoR1.

To construct plasmid pTat8PE (SEQ ID NO:11), we removed a 214-base pair NdeI-ApaI fragment from pTat70PE and replaced it with a double-stranded linker having NdeI and ApaI cohesive termini, encoding tat residues 1-4 and 67-70, and consisting of the following synthetic oligonucleotides:

```
TATGGAACCG GTCGTTTCTC TGTCGGGCC    (SEQ ID NO:12)
CGACAGAGAA ACGACCGGTT CCA          (SEQ ID NO:13).
```

Purification of TAT8-PE

Expression of the pTat8-PE construct yielded the PE ADP ribosylation domain polypeptide fused to amino acids 1-4 and 67-70 of tat protein. The pTat8-PE expression product ("tat8-PE") served as the PE ADP ribosylation domain moiety (and the unconjugated control) in chemical cross-linking experiments described below. Codons for the 8 tat amino acids were artifacts from a cloning procedure selected for convenience. The 8 tat amino acids fused to the PE ADP ribosylation domain had no transport activity (Figure 2).

For purification of tat8-PE, we suspended 4.5 g of pTat8-PE-transformed E.coli in 20 ml of 50 mM Tris-HCl (pH 8.0), 2mM EDTA. We lysed the cells in a French press and removed insoluble debris by centrifugation at 10,000 rpm for 1 hour, in an SA600 rotor. Most of the tat8-PE was in the supernatant. We loaded the supernatant onto a 3 ml Q-Sepharose Fast Flow (Pharmacia LKB, Piscataway, NJ) ion exchange column. After loading the sample, we washed the column with 50 mM Tris-HCl (pH 8.0), 2 mM EDTA. After washing the column, we carried out step gradient elution, using the same buffer with 100, 200 and 400 mM NaCl. The tat8-PE eluted with 200 mM NaCl. Following the ion exchange chromatography, we further purified the tat8-PE by gel filtration on a Superdex 75 FPLC column (Pharmacia LKB, Piscataway, NJ). We equilibrated the gel filtration column with 50 mM HEPES (pH 7.5). We then loaded the sample and carried out elution with the equilibration buffer at 0.34 ml/min. We collected 1.5-minute fractions and stored the tat8-PE fractions at-70°C.

Crosslinking of TAT8-PE

Since the PE ADP ribosylation domain has no cysteine residues, we used sulfo-SMCC (Pierce Chem. Co., Rockford, IL cat no. 22322 G) for transport polypeptide-tat8-PE conjugation. We carried out the conjugation in a 2-step reaction procedure. In the first reaction step, we treated tat8-PE (3 mg/ml), in 50 mM HEPES (pH 7.5), with 10 mM sulfo-SMCC, at room temperature, for 40 minutes. (The sulfo-SMCC was added to the reaction as a 100 mM stock solution in 1 M HEPES, pH 7.5.) We separated the tat8-PE-sulfo-SMCC from the unreacted sulfo-SMCC by gel filtration on a P6DG column (Bio-Rad, Richmond, CA) equilibrated with 25 mM HEPES (pH 6.0), 25 mM NaCl. In the second reaction step, we allowed the tat8-PE-sulfo-SMCC (1.5 mg/ml 100 mM HEPES (pH 7.5), 1 mM EDTA) to react with purified tat37-72 (600 μM final conc.) at room temperature, for 1 hour. To stop the cross-linking reaction, we added cysteine. We analyzed the cross-linking reaction products by SDS-PAGE. About 90% of the tat8-PE became cross-linked to the tat37-72 transport polypeptide under these conditions. Approximately half of the conjugated product had one transport poiypeptide moiety, and half had two transport polypeptide moieties.

Cell-Free Assay for PE ADP Ribosylation

To verify that the PE ribosylation domain retained its biological activity (i.e., destructive ribosome modification) following conjugation to transport polypeptides, we tested the effect of transport polypeptide-PE ADP ribosylation conjugates on in vitro (i.e., cell-free) translation. For each in vitro translation experiment, we made up a fresh translation cocktail and kept it on ice. The in vitro translation cocktail contained 200 μl rabbit reticulocyte lysate (Promega, Madison, WI), 2 μl 10 mM $ZnCl_2$ (optional), 4 μl of a mixture of the 20 protein amino acids except methionine, and 20 μl $^{35}S$-methionine. To 9 μl of translation cocktail we added from 1 to 1000 ng of transport polypeptide-PE conjugate (preferably in a volume of 1 μl) or control, and pre-incubated the mixture for 60 minutes at 30°C. We then added 0.5 μl BMV RNA to each sample and incubated for an additional 60 minutes at 30°C. We stored the samples at -70°C after adding 5 μl of 50% glycerol per sample. We analyzed the in vitro translation reaction products by SDS-PAGE techniques. We loaded 2 μl of each translation reaction mixture (plus an appropriate volume of SDS-PAGE sample buffer) per lane on the SDS gels. After electrophoresis, we visualized the $^{35}S$-containing in vitro translation products by fluorography.

Using the procedure described in the preceding paragraph, we found that the PE ADP ribosylation domain genetically fused to the tat1-70 transport polypeptide had no biological activity, i.e., did not inhibit in vitro translation. In contrast, using the same procedure, we found that the PE ADP ribosylation domain chemically cross-linked to the tat37-72 transport polypeptide had retained full biological activity, i.e., inhibited in vitro translation as well as the non-conjugated PE ADP ribosylation domain controls (Figure 2).

Cytotoxicity Assay for PE ADP Ribosylation

In a further test involving the tat37-72-PE ADP ribosylation domain conjugate, we added it to cultured HeLa cells in the presence or absence of 100 µM chloroquine. We then assayed cytotoxicity by measuring in vivo protein synthesis, as indicated by trichloroacetic acid ("TCA")-precipitable radioactivity in cell extracts.

We performed the cytotoxicity assay as follows. We disrupted HeLa cell layers, centrifuged the cells and resuspended them at a density of 2.5 x $10^4$/ml of medium. We used 0.5 ml of suspension/well when using 24 well plates, or 0.25 ml of suspension/well when using 48 well plates. We added conjugates or unconjugated controls, dissolved in 100 µl of PBS, to the wells after allowing the cells to settle for at least 4 hours. We incubated the cells in the presence of conjugates or controls for 60 minutes, at 37°C, then added 0.5 ml of fresh medium to each cell, and incubated the cells for an additional 5-24 hours. Following this incubation, we removed the medium from each well and washed the cells once with about 0.5 ml PBS. We then added 1 µCi of $^{35}$S-methionine (Amersham) per 100 µl per well in vivo cell labelling grade SJ.1015), and incubated the cells for 2 hours. After two hours, we removed the radioactive medium and washed the cells 3 times with cold 5% TCA and then once with PBS. We added 100 µl of 0.5 M NaOH to each well and allowed at least 45 minutes for cell lysis and protein dissolving to take place. We then added 50 µl 1 M HCl to each well and transferred the entire contents of each well into scintillation fluid for liquid scintillation measurement of radioactivity.

In the absence of chloroquine, there was a clear dose-dependent inhibition of cellular protein synthesis in response to treatment with the transport polypeptide-PE ADP ribosylation domain conjugate, but not in response to treatment with the unconjugated PE ADP ribosylation domain. The results are summarized in Figure 2. When conjugated to tat37-72, the PE ADP ribosylation domain appeared to be transported 3 to 10-fold more efficiently than when conjugated to tat1-72. We also conjugated transport polypeptides tat38-58GGC, tat37-58, tat47-58GGC and tatCGG-47-58 to the PE ADP ribosylation domain. All of these conjugates resulted in cellular uptake of biologically active PE ADP ribosylation domain (data not shown).

EXAMPLE 7

Ribonuclease Conjugates

Chemical Cross-Linking

We dissolved 7.2 mg of bovine pancreatic ribonuclease A, Type 12A (Sigma Chem. Co., St. Louis, MO, cat. no. R5500) in 200 µl PBS (pH 7.5). To the ribonuclease solution, we added 1.4 mg sulfo-SMCC (Pierce Chem. Co., Rockford, IL, cat. no. 22322H). After vortex mixing, we allowed the reaction to proceed at room temperature for 1 hour. We removed unreacted SMCC from the ribonuclease-SMCC by passing the reaction mixture over a 9 ml P6DG column (Bio-Rad, Richmond, CA) and collecting 0.5 ml fractions. We identified the void volume peak fractions (containing the ribonuclease-SMCC conjugate) by monitoring UV absorbance at 280 nm. We divided the pooled ribonuclease-SMCC-containing fractions into 5 equal aliquots. To each of 4 ribonuclease-SMCC aliquots, we added a chemically-synthesized transport polypeptide corresponding to tat residues: 37-72 ("37-72"); 38-58 plus GGC at the carboxy terminal ("38-58GGC"); 37-58 ("CGG37-58"); or 47-58 plus CGG at the amino terminal ("CGG47-58"). We allowed the transport polypeptide-ribonuclease conjugation reactions to proceed for 2 hours at room temperature, and then overnight at 4°C. We analyzed the reaction products by SDS-PAGE on a 10-20% gradient gel. The cross-linking efficiency was approximately 60% for transport polypeptides tat38-58GGC, tat37-58 and tatCGG47-58, and 40% for tat37-72. Of the modified species, 72% contained one, and 25% contained 2 transport polypeptide substitutions.

Cellular Uptake of Tat37-72-Ribonuclease Conjugates

We maintained cells at 37°C in a tissue culture incubator in Dulbecco's Modified Eagle Medium supplemented with 10% donor calf serum and penicillium/streptomycin. For cellular uptake assays, we plated $10^5$ cells in a 24-well plate and cultured them overnight. We washed the cells with Dulbecco's PBS and added the ribonuclease conjugate dissolved in 300 µl of PBS containing 80 µM chloroquine, at concentrations of 0, 10, 20, 40 and 80 µg/ml. After a 1.25 hour incubation at 37°C, we added 750 µl of growth medium and further incubated the cell samples overnight. After the overnight incubation, we washed the cells once with PBS and incubated them for 1 hour in Minimal Essential Medium without methionine (Flow Labs) (250 µl/well) containing $^{35}$S methionine (1 µCi/well). After the 1 hour incubation with radioactive methionine, we removed the medium and washed the cells three times 5% TCA (1 ml/well/wash). We then added 250 µl of 0.5 M NaOH per well. After 1 hour at room temperature, we pipetted 200 µl of the contents of each well into a scintillation vial, added 100 µl of 1 M HCl and 4 ml of scintillation fluid. After thorough mixing of the contents of each vial, we measured radioactivity in each sample by liquid scintillation counting.

The cellular uptake results are summarized in Figure 3. Transport polypeptide tat38-58GGC functioned as well as, or slightly better than tat37-72. Transport polypeptide tatCGG47-58 had reduced activity (data not shown). We do not know whether this polypeptide had reduced uptake activity or whether the proximity of the basic region to the ribonuclease interfered with enzyme activity.

We have used cation exchange chromatography (BioCAD perfusion chromatography system, PerSeptive Biosystems) to purify ribonuclease conjugates having one or two transport polypeptide moieties.

EXAMPLE 8

Protein Kinase A Inhibitor Conjugates

Chemical Cross-Linking

We purchased the protein kinase A inhibitor ("PKAI") peptide (20 amino acids) from Bachem California (Torrence, CA). For chemical cross-linking of PKAI to transport polypeptides, we used either sulfo-MBS (at 10 mM) or sulfo-SMPB (at 15 mM). Both of these cross-linking reagents are heterobifunctional for thiol groups and primary amine groups. Since PKAI lacks lysine and cysteine residues, both sulfo-MBS and sulfo-SMPB selectively target cross-linking to the amino terminus of PKAI. We reacted PKAI at a concentration of 2 mg/ml, in the presence of 50 mM HEPES (pH 7.5), 25 mM NaCl, at room temperature, for 50 minutes, with either cross-linking reagent. The sulfo-MBS reaction mixture contained 10 mM sulfo-MBS and 20% DMF. The sulfo-SMPB reaction mixture contained 15 mM sulfo-SMPB and 20% dimethylsulfoxide ("DMSO"). We purified the PKAI-cross-linker adducts by reverse phase HPLC, using a $C_4$ column. We eluted the samples from the $C_4$ column in a 20-75% acetonitrile gradient containing 0.1% trifluoroacetic acid. We removed the acetonitrile and trifluoroacetic acid from the adducts by lyophilization and redissolved them in 25 mM HEPES (pH 6.0); 25 mM NaCl. We added tat1-72 or tat37-72 and adjusted the pH of the reaction mixture to 7.5, by adding 1 M HEPES (pH 7.5) to 100 mM. We then allowed the cross-linking reaction to proceed at room temperature for 60 minutes.

We regulated the extent of cross-linking by altering the transport polypeptide:PKAI ratio. We analyzed the cross-linking reaction products by SDS-PAGE. With tat37-72, a single new electrophoretic band formed in the cross-linking reactions. This result was consistent with the addition of a single tat37-72 molecule to a single PKAI molecule. With tat1-72, six new products formed in the cross-linking reactions. This result is consistent with the addition of multiple PKAI molecules per tat1-72 polypeptide, as a result of the multiple cysteine residues in tat1-72. When we added PKAI to the cross-linking reaction in large molar excess, we obtained only conjugates containing 5 or 6 PKAI moieties per tat1-72.

In Vitro Phosphorylation Assay for PKAI Activity

To test the sulfo-MBS-cross-linked conjugates for retention of PKAI biological activity, we used an in vitro phosphorylation assay. In this assay, histone V served as the substrate for phosphorylation by protein kinase A in the presence or absence of PKAI (or a PKAI conjugate). We then used SDS-PAGE to monitor PKAI-dependent differences in the extent of phosphorylation. In each reaction, we incubated 5 units of the catalytic subunit of protein kinase A Sigma) with varying amounts of PKAI or PKAI conjugate, at 37°C, for 30 minutes. The assay reaction mixture contained 24 mM sodium acetate (pH 6.0), 25 mM $MgCl_2$, 100 mM DTT, 50 µCi of [$\gamma$-$^{32}$P]ATP and 2 µg of histone V, in a total reaction volume of 40 µl. Using this assay, we found that PKAI conjugated to tat1-72 or tat37-72 inhibited phosphorylation as well as unconjugated PKAI (data not shown).

Cellular Assay

To test for cellular uptake of PKAI and transport polypeptide-PKAI conjugates, we employed cultured cells containing a chloramphenicol acetyltransferase ("CAT") reporter gene under the control of a cAMP-responsive expression control sequence. We thus quantified protein kinase A activity indirectly, by measuring CAT activity. This assay has been described in detail by J. R. Grove et al. ("Probind cAMP-Related Gene Expression with a Recombinant Protein Kinase Inhibitor", Molecular Aspects of Cellular Regulation, Vol. 6, P. Cohen and J. G. Folkes, eds., Elsevier Scientific, Amsterdam, pp. 173-95 (1991)).

Using this assay, we found no activity by PKAI or any of the transport polypeptide-PKAI conjugates. This result suggested to us that the PKAI moiety might be undergoing rapid degradation upon entry into the cells.

Cross-Linking of PKAI to Tat37-72-β-Galactosidase

We had previously found cellular uptake of tat37-72-β-galactosidase to be chloroquine-independent (Example 2, above). Therefore, we cross-linked PKAI to tat37-72-β-galactosidase for possible protection of PKAI against rapid degradation.

We treated β-galactosidase with 20 mM DTT (a reducing agent) at room temperature for 30 minutes and then removed the DTT by gel filtration on a G50 column in MES buffer (pH 5). We allowed the reduced β-galactosidase to react with SMPB-activated PKAI (above), at pH 6.5, for 60 minutes. To block residual free sulfhydryl groups, we added N-ethylmaleimide or iodoacetamide. SDS-PAGE analysis showed that at least 95% of the β-galactosidase had been conjugated. About 90% of the conjugated beta-galactosidase product contained one PKAI moiety per subunit, and about 10% contained 2 PKAI moieties. We treated the PKAI-β-galactosidase conjugate with a 10-fold molar excess of sulfo-SMCC. We then reacted the PKAI-β-galactosidase-SMCC with tat1-72. According to SDS-PAGE analysis, the PKAI-β-galactosidase:tat1-72 ratio appeared to be 1:0.5. We have produced about 100 μg of the final product. Because of precipitation problems, the concentration of the final product in solution has been limited to 100 μg/ml.

EXAMPLE 9

E2 Repressor Conjugates

To test cellular uptake and E2 repressor activity of transport polypeptide-E2 repressor conjugates, we simultaneously transfected an E2-dependent reporter plasmid and an E2 expression plasmid into SV40-transformed African green monkey kidney ("COS7") cells. Then we exposed the transfected cells to transport polypeptide-E2 repressor conjugates (made by genetic fusion or chemical cross-linking) or to appropriate controls. The repression assay, described below, was essentially as described in Barsoum et al. (supra).

Repression Assay Cells

We obtained the COS7 cells from the American Type Culture Collection, Rockville, MD (ATCC No. CRL 1651). We propagated the COS7 cells in Dulbecco's modified Eagle's medium (GIBCO, Grand Island, NY) with 10% fetal bovine serum (JRH Biosciences, Lenexa, KS) and 4 mM glutamine ("growth medium"). Cell incubation conditions were 5.5% $CO_2$ at 37°C.

Repression Assay Plasmids

Our E2-dependent reporter plasmid, pXB332hGH, contained a human growth hormone reporter gene driven by a truncated SV40 early promoter having 3 upstream E2 binding sites. We constructed the hGH reporter plasmid, pXB332hGH, as described in Barsoum et al. (supra).

For expression of a full-length HPV E2 gene, we constructed plasmid pAHE2 (Figure 4). Plasmid pAHE2 contains the E2 gene from HPV strain 16, operatively linked to the adenovirus major late promoter augmented by the SV40 enhancer, upstream of the promoter. We isolated the HPV E2 gene from plasmid pHPV16 (the full-length HPV16 genome cloned into pBR322), described in M. Durst et al., "A Papillomavirus DNA from Cervical Carcinoma and Its Prevalence in Cancer Biopsy Samples from Different Geographic Regions", Proc. Natl. Acad. Sci. USA, 80, pp. 3812-15 (1983), as a Tth111I-AseI fragment. Tth111I cleaves at nucleotide 2711, and AseI cleaves at nucleotide 3929 in the HPV16 genome. We blunted the ends of the Tth111I-AseI fragment in a DNA polymerase I Klenow reaction, and ligated BamHI linkers (New England Biolabs, cat. no. 1021). We inserted this linker-bearing fragment into BamHI-cleaved plasmid pBG331, to create plasmid pAHE2.

Plasmid pBG331 is the same as pBG312 (R.L. Cate et al., "Isolation of the Bovine and Human Genes for Mullerian Inhibiting Substance and Expression of the Human Gene in Animal Cells", Cell, 45, pp. 685-98 (1986)) except that it lacks the BamHI site downstream of the SV40 polyadenylation signal, making the BamHI site between the promoter and the SV40 intron unique. We removed the unwanted BamHI site by partial BamHI digestion of pBG312, gel purification of the linearized plasmid, blunt end formation by DNA polymerase I Klenow treatment, self-ligation and screening for plasmids with the desired deletion of the BamHI site.

Bacterial Production of E2 Repressor Proteins

One of our E2 repressor proteins, E2.123, consisted of the carboxy-terminal 121 amino acids of HPV16 E2 with MetVal added at the amino terminus. We also used a variant of E2.123, called E2.123CCSS. E2.123 has cysteine residues at HPV16 E2 amino acid positions 251, 281, 300 and 309. In E2.123CCSS, the cysteine residues at positions 300 and 309 were changed to serine, and the lysine residue at position 299 was changed to arginine. We replaced the cysteine residues at positions 300 and 309, so that cysteine-dependent chemical cross-linking could take place in the amino terminal portion of the E2 repressor, but not in the E2 minimal DNA binding/dimerization domain. We considered crosslinks in the minimal DNA binding domain likely to interfere with the repressor's biological activity.

For construction of plasmid pET8c-123 (Figure 5; SEQ ID NO:14), we produced the necessary DNA fragment by standard polymerase chain reaction ("PCR") techniques, with plasmid pHPV16 as the template. (For a general discussion of PCR techniques, see Chapter 14 of Sambrook et al., supra. Automated PCR equipment and chemicals are commercially available.) The nucleotide sequence of EA52, the PCR oligonucleotide primer for the 5' end of the 374 base pair E2-123 fragment, is set forth in the Sequence Listing under SEQ ID NO:15. The nucleotide sequence of EA54, the PCR oligonucleotide primer used for the 3' end of the E2-123 fragment is set forth in the Sequence Listing under SEQ ID NO:16. We digested the PCR products with NcoI and BamHI and cloned the resulting fragment into NcoI/BamHI-digested expression plasmid pET8c (Studier et al., supra), to create plasmid pET8c-123.

By using the same procedure with a different 5' oligonucleotide PCR primer, we obtained a 260 base pair fragment ("E2-85") containing a methionine codon and an alanine codon immediately followed by codons for the carboxy-terminal 83 amino acids of HPV16 E2. The nucleotide sequence of EA57, the PCR 5' primer for producing E2-85, is set forth in the Sequence Listing under SEQ ID NO:34.

To construct plasmid pET8c-123CCSS (Figure 6; SEQ ID NO:17), for bacterial production of E2.123CCSS, we synthesized an 882 bp PstI-EagI DNA fragment by PCR techniques. The PCR template was pET8c-123. One of the PCR primers, called 374.140, encoded all three amino acid changes:
CGACACTGCA GTATACAATG TAGAATGCTT TTTAAATCTA TATCTTAAAG ATCTTAAAG (SEQ ID NO:18). The other PCR primer, 374.18, had the following sequence: GCGTCGGCCG CCATGCCGGC GATAAT (SEQ ID NO:19). We digested the PCR reaction products with PstI plus EagI and isolated the 882 bp fragment by standard methods. The final step was production of pET8c-123CCSS in a 3-piece ligation joining a 3424 bp EcoRI-EagI fragment from pET8c-123 with the 882 bp PCR fragment and a 674-bp PstI-EcoRI pET8c-123 fragment, as shown in Figure 6. We verified the construction by DNA sequence analysis. For production of E2.123 and E2.123CCSS proteins, we expressed plasmids pET8c-123 and pET8c-123CCSS in E.coli strain BL21(DE3)pLysS, as described by Studier (supra).

Purification of E2 Repressor Proteins

We thawed 3.6 grams of frozen, pET8c-123-transformed E.coli cells and suspended them in 35 ml of 25 mM Tris-HCl (pH 7.5), 0.5 mM EDTA, 2.5 mM DTT, plus protease inhibitors (1 mM PMSF, 3 mM benzamidine, 50 μg/ml pepstatin A, 10 μg/ml aprotinin). We lysed the cells by two passages through a French press at 10,000 psi. We centrifuged the lysate at 12,000 rpm, in an SA600 rotor, for 1 hour. The E2.123 protein was in the supernatant. To the supernatant, we added MES buffer (pH 6) up to 25 mM, MES buffer (pH 5) up to 10 mM, and NaCl up to 125 mM. We then applied the supernatant to a 2 ml S Sepharose Fast Flow column at 6 ml/hr. After loading, we washed the column with 50 mM Tris-HCl (pH 7.5), 1 mM DTT. We then carried out step gradient elution (2 ml/step) with 200, 300, 400, 500, 700 and 1000 mM NaCl in 50 mM Tris-HCl (pH -7.5), 1 mM DTT. The E2.123 repressor protein eluted in the 500 and 700 mM NaCl fractions. SDS-PAGE analysis indicated the E2.123 repressor purity exceeded 95%.

We thawed 3.0 grams of frozen, pET8c-123CCSS-transformed E.coli and suspended the cells in 30 ml of the same buffer used for pET8c-123-transformed cells (above). Lysis, removal of insoluble cellular debris and addition of MES buffer and NaCl was also as described for purification of E2-123. The purification procedure for E2.123CCSS diverged after addition of the MES buffer and NaCl, because a precipitate formed, with E2.123CCSS, at that point in the procedure. We removed the precipitate by centrifugation, and found that it and the supernatant both contained substantial E2 repressor activity. Therefore, we subjected both to purification steps. We applied the supernatant to a 2 ml S Sepharose Fast Flow column (Pharmacia LKB, Piscataway, NJ) at 6 ml/hr. After loading, we washed the column with 50 mM Tris-HCl (pH 7.5), 1 mM DTT. After washing the column, we carried out step gradient elution (2 ml/step), using 300, 400, 500, 700 and 1000 mM NaCl in 50 mM Tris-HCl (pH 7.5), 1 mM DTT. The E2.123CCSS protein eluted with 700 mM NaCl. SDS-PAGE analysis indicated its purity to exceed 95%. We dissolved the E2.123CCSS precipitate in 7.5 ml of 25 mM Tris-HCl (pH 7.5), 125 mM NaCl, 1 mM DTT and 0.5 mM EDTA. We loaded the dissolved material onto a 2 ml S Sepharose Fast Flow column and washed the column as described for E2.123 and non-precipitated E2.123CCSS. We carried out step gradient elution (2 ml/step), using 300, 500, 700 and 1000 mM NaCl. The E2 repressor eluted in the 500-700 mM NaCl fractions. SDS-PAGE analysis indicated its purity to exceed 98%. Immediately following purification of the E2.123 and E2.123CCSS proteins, we added glycerol to a final concentration of 15% (v/v), and stored flash-frozen (liquid $N_2$) aliquots at -70°C. We quantified the purified E2 repressor proteins by UV absorbance at 280 nm, using an extinction coefficient of 1.8 at 1 mg/ml.

Chemical Cross-Linking

We performed chemical synthesis of the transport polypeptide consisting of tat amino acids 37-72, as described in Example 1. We dissolved the polypeptide (5 mg/ml) in 10 mM MES buffer (pH 5.0), 50 mM NaCl, 0.5 mM EDTA, (extinction coefficient of 0.2 at 1 ml/ml). To the transport polypeptide solution, we added a bismaleimidohexane ("BMH") (Pierce Chemical Co., Rockford, IL, cat. no. 22319G) stock solution (6.25 mg/ml DMF) to a final concentration of 1.25 mg/ml, and a pH 7.5 HEPES buffer stock solution (1 M) to a final concentration of 100 mM. We allowed the BMH to react with the protein for 30 minutes at room temperature. We then separated the protein-BMH from unreacted BMH by gel filtration on a G-10 column equilibrated in 10 mM MES (pH 5), 50 mM NaCl, 0.5 mM EDTA. We stored aliquots of the transport polypeptide-BMH conjugate at -70°C.

For cross-linking of the transport polypeptide-BMH conjugate to the E2 repressor, we removed the E2 repressor protein from its storage buffer. We diluted the E2 repressor protein with three volumes of 25 mM MES (pH 6.0), 0.5 mM EDTA and batch-loaded it onto S Sepharose Fast Flow (Pharmacia LKB, Piscataway, NJ) at 5 mg protein per ml resin. After pouring the slurry of protein-loaded resin into a column, we washed the column with 25 mM MES (pH 6.0), 0.5 mM EDTA, 250 mM NaCl. We then eluted the bound E2 repressor protein from the column with the same buffer containing 800 mM NaCl. We diluted the E2 repressor-containing eluate to 1 mg/ml with 25 mM MES (pH 6.0), 0.5 mM EDTA. From trial cross-linking studies performed with each batch of E2 repressor protein and BMH-activated transport polypeptide, we determined that treating 1 mg of E2 repressor protein with 0.6 mg of BMH-activated transport polypeptide yields the desired incorporation of 1 transport molecule per E2 repressor homodimer. Typically, we mixed 2 ml of E2 repressor (1 mg/ml) with 300 µl of tat37-72-BMH (4 mg/ml) and 200 µl of 1 M HEPES (pH 7.5). We allowed the cross-linking reaction to proceed for 30 minutes at room temperature. We terminated the cross-linking reaction by adding 2-mercaptoethanol to a final concentration of 14 mM. We determined the extent of cross-linking by SDS-PAGE analysis. We stored aliquots of the tat37-72-E2 repressor conjugate at -70°C. We employed identical procedures to chemically cross-link the tat37-72 transport polypeptide to the HPVE2 123 repressor protein and the HPVE2 CCSS repressor protein.

Cellular Uptake of E2 Repressor Conjugates

For our E2 repression assays, we used transient expression of plasmids transfected into COS7 cells. Our E2 repression assay procedure was similar to that described in Barsoum et al. (supra). We transfected 4 x $10^6$ COS7 cells (about 50% confluent at the time of harvest) by electroporation, in two separate transfections ("EP1" and "EP2"). In transfection EP1, we used 20 µg pXB332hGH (reporter plasmid) plus 380 µg sonicated salmon sperm carrier DNA (Pharmacia LKB, Piscataway, NJ). In transfection EP2, we used 20 µg pXB332hGH plus 30 µg pAHE2 (E2 transactivator) and 350 µg salmon sperm carrier DNA. We carried out electroporations with a Bio-Rad Gene Pulser, at 270 volts, 960 µFD, with a pulse time of about 11 msec. Following the electroporations, we seeded the cells in 6-well dishes, at 2 x $10^5$ cells per well. Five hours after the electroporations, we aspirated the growth medium, rinsed the cells with growth medium and added 1.5 ml of fresh growth medium to each well. At this time, we added chloroquine ("CQ") to a final concentration of 80 µM (or a blank solution to controls). Then we added tat37-72 cross-linked E2.123 ("TxHE2") or tat37-72 cross-linked to E2.123CCSS ("TxHE2CCSS"). The final concentration of these transport polypeptide-cargo conjugates was 6, 20 or 60 µg/ml of cell growth medium (Table I).

TABLE I

| Identification of Samples | | |
|---|---|---|
| well | CQ (µM) | protein (µg/ml) |
| EP1.1 | 0 | 0 |
| EP1.2 | 80 | 0 |
| | | |
| EP2.1 | 0 | 0 |
| EP2.2 | 0 | 6 TxHE2 |
| EP2.3 | 0 | 20 TxHE2 |
| EP2.4 | 0 | 60 TxHE2 |
| EP2.5 | 0 | 6 TxHE2CCSS |
| EP2.6 | 0 | 20 TxHE2CCSS |
| EP2.7 | 0 | 60 TxHE2CCSS |
| EP2.8 | 80 | 0 |

TABLE I (continued)

| Identification of Samples | | |
|---|---|---|
| well | CQ (μM) | protein (μg/ml) |
| EP2.9 | 80 | 6 TxHE2 |
| EP2.10 | 80 | 20 TxHE2 |
| EP2.11 | 80 | 60 TxHE2 |
| EP2.12 | 80 | 6 TxHE2CCSS |
| EP2.13 | 80 | 20 TxHE2CCSS |
| EP2.14 | 80 | 60 TxHE2CCSS |

After an 18-hour incubation, we removed the medium, rinsed the cells with fresh medium, and added 1.5 ml of fresh medium containing the same concentrations of chloroquine and transport polypeptide-cargo conjugates as in the preceding 18-hour incubation. This medium change was to remove any hGH that may have been present before the repressor entered the cells. Twenty-four hours after the medium change, we harvested the cells and performed cell counts to check for viability. We then assayed for hGH on undiluted samples of growth medium according to the method of Seldon, described in Protocols in Molecular Biology, Green Publishing Associates, New York, pp. 9.7.1-9.7.2 (1987), using the Allegro Human Growth Hormone transient gene expression system kit (Nichols Institute, San Juan Capistrano, CA). We subtracted the assay background (i.e., assay components with non-conditioned medium added) from the hGH cpm, for all samples. We performed separate percentage repression calculations for a given protein treatment, according to whether chloroquine was present ("(+)CQ") or absent ("(-)CQ") in the protein uptake test. We calculated percentage repression according to the following formula:

$$\text{Repression} = \frac{(\text{ACT - BKG}) - (\text{REP - BKG})}{\text{ACT - BKG}} \times 100$$

where:

BKG = hGH cpm in the transfections of reporter alone (e.g., EP1.1 for (-)CQ and EP1.2 for (+)CQ);
ACT = hGH cpm in the transfection of reporter plus transactivator, but to which no repressor conjugate was added (e.g., EP2.1 for (-)CQ and EP2.8 for (+)CQ) ;
REP = hGH cpm in the transfection of reporter plus transactivator, to which a repressor conjugate was added (e.g., EP2.2-2.7 for (-)CQ and EP2.9-2.14 for (+)CQ).

Data from a representative E2 repression assay are shown in Table II. Table I identifies the various samples represented in Table II. Figure 7 graphically depicts the results presented in Table II.

TABLE II

| E2 Repression Assay | | | | |
|---|---|---|---|---|
| sample | hGH cpm | cpm - assay bkgd | cpm - BKG | % repression |
| EP1.1 | 3958 | 3808 | -- | -- |
| EP1.2 | 5401 | 5251 | -- | -- |
| EP2.1 | 15,161 | 15,011 | 11,203 | -- |
| EP2.2 | 12,821 | 12,671 | 8863 | 20.9 |
| EP2.3 | 10,268 | 10,118 | 6310 | 43.7 |
| EP2.4 | 8496 | 8346 | 4538 | 59.5 |
| EP2.5 | 11,934 | 11,784 | 7976 | 28.8 |
| EP2.6 | 9240 | 9090 | 5282 | 52.9 |
| EP2.7 | 7926 | 7776 | 3968 | 64.6 |
| EP2.8 | 15,120 | 14,970 | 9719 | -- |

TABLE II (continued)

| E2 Repression Assay | | | | |
|---|---|---|---|---|
| sample | hGH cpm | cpm - assay bkgd | cpm - BKG | % repression |
| EP2.9 | 12,729 | 12,579 | 7328 | 24.6 |
| EP2.10 | 9590 | 9440 | 4189 | 56.9 |
| EP2.11 | 8440 | 8290 | 3039 | 68.7 |
| EP2.12 | 11,845 | 11,695 | 6444 | 33.7 |
| EP2.13 | 8175 | 8025 | 2774 | 71.5 |
| EP2.14 | 6697 | 6547 | 1296 | 86.7 |

Transport polypeptide tat37-72 cross-linked to either E2 repressor (E2.123 or E2.123CCSS) resulted in a dose-dependent inhibition of E2-dependent gene expression in the cultured mammalian cells (Table II; Figure 7). We have repeated this experiment four times, with similar results. The effect was E2-specific, in that other tat37-72 conjugates had no effect on E2 induction of pXB332hGH (data not shown). Also, the tat37-72xHE2 conjugates had no effect on the hGH expression level of a reporter in which the expression of the hGH gene was driven by a constitutive promoter which did not respond to E2. The E2 repressor with the CCSS mutation repressed to a greater degree than the repressor with the wild-type amino acid sequence. This was as expected, because cross-linking of the transport polypeptide to either of the last two cysteines in the wild-type repressor would likely reduce or eliminate repressor activity. Chloroquine was not required for the repression activity. However, chloroquine did enhance repression in all of the tests. These results are summarized in Table II and Figure 7.

EXAMPLE 10

TATΔCYS Conjugates

Production of TatΔcys

For bacterial production of a transport polypeptide consisting of tat amino acids 1-21 fused directly to tat amino acids 38-72, we constructed expression plasmid pTATΔcys (Figure 8; SEQ ID NO:20). To construct plasmid pTATΔcys, we used conventional PCR techniques, with plasmid pTAT72 as the PCR template. One of the oligonucleotide primers used for the PCR was 374.18 (SEQ ID NO:19), which covers the EagI site upstream of the tat coding sequence. (We also used oligonucleotide 374.18 in the construction of plasmid pET8c-123CCSS. See Example 9.) The other oligonucleotide primer for the PCR, 374.28, covers the EagI site within the tat coding sequence and has a deletion of the tat DNA sequence encoding amino acids 22-37. The nucleotide sequence of 374.28 is: TTTACGGCCG TAAGAGATAC CTAGGGCTTT GGTGATGAAC GCGGT (SEQ ID NO:21). We digested the PCR products with EagI and isolated the resulting 762-base pair fragment. We inserted that EagI fragment into the 4057 base pair vector produced by EagI cleavage of pTAT72. We verified the construction by DNA sequence analysis and expressed the tatΔcys polypeptide by the method of Studier et al. (supra). SDS-PAGE analysis showed the tatΔcys polypeptide to have the correct size.

For purification of tatΔcys protein, we thawed 4.5 grams of pTATΔcys-transformed E.coli cells, resuspended the cells in 35 ml of 20 mM MES (pH 6.2), 0.5 mM EDTA. We lysed the cells by two passes through a French press, at 10,000 psi. We removed insoluble debris by centrifugation at 10,000 rpm in an SA600 rotor, for 1 hour. We applied the supernatant to a 5 ml S Sepharose Fast Flow column at 15 ml/hr. We washed the column with 50 mM Tris-HCl (pH 7.5), 0.3 mM DTT. We then carried out step gradient elution (2 ml/step) with the same buffer containing 300, 400, 500, 700 and 950 mM NaCl. The tatΔcys protein eluted in the 950 mM NaCl fraction.

We conjugated a tatΔcys transport polypeptide to rhodamine isothiocyanate and tested it by assaying directly for cellular uptake. The results were positive (similar to results in related experiments with tat1-72).

TATΔcys-249 Genetic Fusion

For bacterial expression of the tatΔcys transport polypeptide genetically fused to the amino terminus of the native E2 repressor protein (i.e., the carboxy-terminal 249 amino acids of BPV-1 E2), we constructed plasmid pTATΔcys-249 as follows. We constructed plasmid pFTE501 (Figure 9) from plasmids pTAT72 (Frankel and Pabo, supra) and pXB314 (Barsoum et al., supra). From plasmid pXB314, we isolated the NcoI-SpeI DNA fragment encoding the 249 amino acid BPV-1 E2 repressor. (NcoI cleaves at nucleotide 296, and SpeI cleaves at nucleotide 1118 of pXB314.) We blunted the ends of this fragment by DNA polymerase I Klenow treatment and added a commercially available BglII linker (New England Biolabs, cat. no. 1090). We inserted this linker-bearing fragment into BamHI-cleaved (complete digestion) plasmid pTAT72. In pTAT72, there is a BamHI cleavage site within the tat coding region, near its 3' end, and a second BamHI cleavage site slightly downstream of the tat gene. The BglII linker joined the tat and E2 coding sequences in frame to encode a fusion of the first 62 amino acids of tat protein followed by a serine residue and the last 249 amino acids of BPV-1 E2 protein. We designated this bacterial expression plasmid pFTE501 (Figure 9). To construct plasmid pTATΔcys-249 (Figure 10; SEQ ID NO:22), we inserted the 762 base pair EagI fragment from plasmid pTAT cys, which includes the portion of tat containing the cysteine deletion, into the 4812 base pair EagI fragment of plasmid pFTE501.

Purification of tatΔcys-249

We thawed 5 g of E.coli expressing tatΔcys-249 and suspended the cells in 40 ml of 25 mM Tris HCl (pH 7.5), 25 mM NaCl, 0.5 mM EDTA, 5 mM DTT, plus protease inhibitors (1.25 mM PMSF, 3 mM Benzamidine, 50 μg/ml pepstatin A, 50 μg/ml aprotinin, 4 μg/ml E64). We lysed the cells by two passages through a French pressure cell at 10,000 psi. We removed insoluble debris from the lysate by centrifugation at 12,000 rpm in an SA600 rotor, for 1 hour. We purified the tatΔcys-249 from the soluble fraction. The supernatant was loaded onto a 2 ml S Sepharose Fast Flow column (Pharmacia LKB, Piscataway, NJ) at a flow rate of 6 ml/h. The column was washed with 25 mM Tris HCl pH (7.5), 25 mM NaCl, 0.5 mM EDTA, 1 mM DTT and treated with sequential salt steps in the same buffer containing 100, 200, 300, 400, 500, 600, and 800 mM NaCl. We recovered the TatΔcys-249 in the 600-800 mM salt fractions. We pooled the peak fractions, added glycerol to 15%, and stored aliquots at -70°C.

Immunofluorescence Assay

To analyze cellular uptake of the tatΔcys-E2 repressor fusion protein, we used indirect immunofluorescence techniques. We seeded HeLa cells onto cover slips in 6-well tissue culture dishes, to 50% confluence. After an overnight incubation, we added the tatΔcys-E2 repressor fusion protein (1 μg/ml final concentration) and chloroquine (0.1 mM final concentration). After six hours, we removed the fusion protein/chloroquine-containing growth medium and washed the cells twice with PBS. We fixed the washed cells in 3.5% formaldehyde at room temperature. We permeabilized the fixed cells with 0.2% Triton X-100/2% bovine serum albumin ("BSA") in PBS containing 1 mM $MgCl_2$/0.1 mM $CaCl_2$ ("PBS+") for 5 minutes at room temperature. To block the permeabilized cells, we treated them with PBS containing 2% BSA, for 1 hour at 4°C.

We incubated the cover slips with 20 μl of a primary antibody solution in each well, at a 1:100 dilution in PBS+ containing 2% BSA, for 1 hour at 4°C. The primary antibody was either a rabbit polyclonal antibody to the BPV-1 E2 repressor (generated by injecting the purified carboxy-terminal 85 amino acids of E2), or a rabbit polyclonal antibody to tat (generated by injecting the purified amino-terminal 72 amino acids of tat protein). We added a secondary antibody at a 1:100 dilution in 0.2% Tween-20/2% BSA in PBS+ for 30 minutes at 4°C.

The secondary antibody was a rhodamine-conjugated goat anti-rabbit IgG (Cappel no. 2212-0081). Following incubation of the cells with the secondary antibody, we washed the cells with 0.2% Tween 20/2% BSA in PBS+ and mounted the cover slips in 90% glycerol, 25 mM sodium phosphate (pH 7.2), 150 mM NaCl. We examined the cells with a fluorescent microscope having a rhodamine filter.

Cellular Uptake of TatΔCys Fusions

We observed significant cellular uptake of the tatΔcys-E2 repressor fusion protein, using either the tat antibody or the E2 antibody. In control cells exposed to the unconjugated tat protein, we observed intracellular fluorescence using the tat antibody, but not the E2 antibody. In control cells exposed to a mixture of the unconjugated E2 repressor and tat protein or tatΔcys, we observed fluorescence using the tat antibody, but not the E2 antibody. This verified that tat mediates E2 repressor uptake only when linked to the tat protein. As with unconjugated tat protein, we observed the tatΔcys-E2 repressor fusion protein throughout the cells, but it was concentrated in intracellular vesicles. These results show that a tat-derived polypeptide completely lacking cysteine residues can carry a heterologous protein (i.e., transport polypeptide-cargo protein genetic fusion) into animal cells.

In a procedure similar to that described above, we produced a genetic fusion of tatΔcys to the C-terminal 123 amino acids of HPV E2. When added to the growth medium, this fusion polypeptide exhibited repression of E2-dependent gene expression in C0S7 cells (data not shown).

EXAMPLE 11

Antisense Oligodeoxynucleotide Conjugates

Using an automated DNA/RNA synthesizer (Applied Biosystems model 394), we synthesized DNA phosphorothionate analogs (4-18 nucleotides in length), with each containing a free amino group at the 5' end. The amine group was incorporated into the oligonucleotides using commercially modified nucleotides (aminolink 2, Applied Biosystems). The oligonucleotides corresponded to sense and antisense strands from regions of human growth hormone and CAT messenger RNA.

For each cross-linking reaction, we dissolved 200 µg of an oligonucleotide in 100 µl of 25 mM sodium phosphate buffer (pH 7.0). We then added 10 µl of a 50 mM stock solution of sulfo-SMCC and allowed the reaction to proceed at room temperature for 1 hour. We removed unreacted sulfo-SMCC by gel filtration of the reaction mixture on a P6DG column (Bio-Rad) in 25 mM HEPES (pH 6.0). We dried the oligonucleotide-sulfo-SMCC adduct under a vacuum. Recovery of the oligonucleotides in this procedure ranged from 58 to 95%. For reaction with a transport polypeptide, we redissolved each oligonucleotide-sulfo-SMCC adduct in 50 µl of 0.5 mM EDTA, transferred the solution to a test tube containing 50 µg of lyophilized transport polypeptide, and allowed the reaction to proceed at room temperature for 2 hours. We analyzed the reaction products by SDS-PAGE.

EXAMPLE 12

Antibody Conjugates

Anti-Tubulin conjugate 1

We obtained commercial mouse IgG1 mAb anti-tubulin (Amersham) and purified it from ascites by conventional methods, using protein A. We labelled the purified antibody with rhodamine isothiocyanate, at 1.2 moles rhodamine/mole Ab. When we exposed fixed, permeabilized HeLa cells to the labelled antibody, microscopic examination revealed brightly stained microtubules. Although the rhodamine labelling was sufficient, we enhanced the antibody signal with antimouse FITC.

In a procedure essentially as described in Example 2, (above) we allowed 250 µg of the antibody to react with a 10:1 molar excess of sulfo-SMCC. We then added 48 µg of ($^{35}$S-labelled) tat1-72. The molar ratio of tat1-71:Ab was 2.7:1. According to incorporation of radioactivity, the tat1:72 was cross-linked to the antibody in a ratio of 0.6:1.

For analysis of uptake of the tat1-72-Ab conjugate, we added the conjugate to medium (10 µg/ml) bathing cells grown on coverslips. We observed a punctate pattern of fluorescence in the cell. The punctate pattern indicated vesicular location of the conjugate, and was therefore inconclusive as to cytoplasmic delivery.

To demonstrate immunoreactivity of the conjugated antibody, we tested its ability to bind tubulin. We coupled purified tubulin to cyanogen bromide-activated Sepharose 4B (Sigma Chem. Co., St. Louis, MO). We applied a samples of the radioactive conjugate to the tubulin column (and to a Sepharose 4B control column) and measured the amount of bound conjugate. More radioactivity bound to the affinity matrix than to the control column, indicating tubulin binding activity.

Anti-Tubulin conjugate 2

In a separate cross-linking experiment, we obtained an anti-tubulin rat monoclonal antibody IgG2a (Serotec), and purified it from ascites by conventional procedures, using protein G. We eluted the antibody with Caps buffer (pH 10). The purified antibody was positive in a tubulin-binding assay. We allowed tat1-72 to react with rhodamine isothiocyanate at a molar ratio of 1:1. The reaction product exhibited an $A_{555}/A_{280}$ ratio of 0.63, which indicated a substitution of approximately 0.75 mole of dye per mole of tat1-72. Upon separation of the unreacted dye from the tat1-72-rhodamine, by G-25 gel filtration (Pharmacia LKB, Piscataway, NJ), we recovered only 52 µg out of 150 µg of tat1-72 used in the reaction.

We saved an aliquot of the tat1-72-rhodamine for use (as a control) in cellular uptake experiments, and added the rest to 0.4 mg of antibody that had reacted with SMCC (20:1). The reaction mixture contained a tat1-72:Ab ratio of approximately 1:1, rather than the intended 5:1. (In a subsequent experiment, the 5:1 ratio turned out to be unsatisfactory, yielding a precipitate.) We allowed the cross-linking reaction to proceed overnight at 4°C. We then added a molar excess of cysteine to block the remaining maleimide groups and thus stop the cross-linking reaction. We centrifuged the reaction mixtures to remove any precipitate present.

We carried out electrophoresis using a 4-20% polyacrylamide gradient gel to analyze the supernatant under reducing and non-reducing conditions. We also analyzed the pellets by this procedure. In supernatants from antibody-tat1-72 (without rhodamine) conjugation experiments, we observed very little material on the 4-20% gel. However, in supernatants from the antibody-tat1-72-rhodamine conjugation experiments, we observed relatively heavy bands above the antibody, for the reduced sample. The antibody appeared to be conjugated to the tat1-72 in a ratio of approximately 1:1.

In cellular uptake experiments carried out with conjugate 2 (procedure as described above for conjugate 1), we obtained results similar to those obtained with conjugate 1. When visualizing the conjugate by rhodamine fluorescence or by fluorescein associated with a second antibody, we observed the conjugate in vesicles.

EXAMPLE 13

Additional Tat-E2 Conjugates

Chemically Cross-Linked Tat-E2 Conjugates

We chemically cross-linked transport polypeptide tat37-72 to four different repressor forms of E2. The four E2 repressor moieties used in these experiments were the carboxy-terminal 103 residues (i.e., 308-410) of BPV-1 ("E2.103"); the carboxy-terminal 249 residues (i.e., 162-410) of BPV-1 ("E2.249"); the carboxy-terminal 121 residues (i.e., 245-365) of HPV-16 ("HE2"); and the carboxy-terminal 121 residues of HPV-16, in which the cysteine residues at positions 300 and 309 were changed to serine, and the lysine residue at position 299 was changed to arginine ("HE2CCSS"). The recombinant production and purification of HE2 and HE2CCSS, followed by chemical cross-linking of HE2 and HE2CCSS to tat37-72, to form TxHE2 and TxHE2CCSS, repectively, are described in Example 9 (above). For the chemical cross-linking of E2.103 and E2.249 to tat37-72 (to yield the conjugates designated TxE2.103 and TxE2.249), we employed the same method used to make TxHE2 and TxHE2CCSS (Example 9, supra).

We expressed the protein E2.103 in E.coli from plasmid pET-E2.103. We obtained pET-E2.103 by a PCR cloning procedure analogous to that used to produce pET8c-123, described in Example 9 (above) and Figure 5. As in the construction of pET8c-123, we ligated a PCR-produced NcoI-bamHI E2 fragment into NcoI-BamHI-cleaved pET8c. Our PCR template for the E2 fragment was plasmid pCO-E2 (Hawley-Nelson et al., EMBO J., vol 7, pp. 525-31 (1988); United States patent 5,219,990). The oligonucleotide primers used to produce the E2 fragment from pCO-E2 were EA21 (SEQ ID NO:36) and EA22 (SEQ ID NO: 37). Primer EA21 introduced an NcoI site that added a methionine codon followed by an alanine codon 5' adjacent to the coding region for the carboxy-terminal 101 residues of BPV-1 E2.

We expressed the protein E2.249 in E.coli from plasmid pET8c-249. We constructed pET8c-249 by inserting the 1362 bp NcoI-BamHI fragment of plasmid pXB314 (Figure 9) into NcoI-BamHI-cleaved pET8c (Figure 5).

TATΔcys-BPV E2 Genetic Fusions

In addition to TATΔcys-249, we tested several other TATΔcys-BPV-1 E2 repressor fusions. Plasmid pTATΔcys-105 encoded tat residues 1-21 and 38-67, followed by the carboxy-terminal 105 residues of BPV-1. Plasmid pTATΔcys-161 encoded tat residues 1-21 and 38-62, followed by the carboxy-terminal 161 residues of BPV-1. We constructed plamids pTATΔcys-105 and pTATΔcys-161 from intermediate plasmids pFTE103 and pFTE403, respectively.

We produced pFTE103 and pFTE403 (as well as pFTE501) by ligating different inserts into BamHI-cleaved (complete digestion) vector pTAT72.

To obtain the insertion fragment for pFTE103, we isolated a 929 base pair PleI-BamHI fragment from pXB314 and ligated it to a double-stranded linker consisting of synthetic oligonucleotide FTE.3 (SEQ ID NO:23) and synthetic oligonucleotide FTE.4 (SEQ ID NO:24). The linker encoded tat residues 61-67 and had a BamHI overhang at the 5' end and a PleI overhang at the 3' end. We ligated the linker-bearing fragment from pXB3314 into BamHI-cleaved pTAT72, to obtain pFTE103. To obtain the insertion fragment for pFTE403, we digested pXB314 with NcoI and SpeI, generated blunt ends with Klenow treatment and ligated a BglII linker consisting of GAAGATCTTC (New England Biolabs, Beverly, MA, Cat. No. 1090) (SEQ ID NO:35) duplexed with itself. We purified the resulting 822-base pair fragment by eletrophoresis and then ligated it into BamHI-digested pTAT72 vector, to obtain pFTE403.

To delete tat residues 22-37, thereby obtaining plasmid pTATΔcys-105 from pFTE103 and pTATΔcys-161 from pFTE403, we employed the same method (described above) used to obtain plasmid pTATΔcys-249 from pFTE501.

TATΔCVS-HPV E2 Genetic Fusions

We constructed plasmids pTATΔcys-HE2.85 and pTATΔcys-HE2.121 to encode a fusion protein consisting of the tatΔcys transport moiety (tat residues 1-21, 38-72) followed by the carboxy-terminal 85 or 121 residues of HPV-16, respectively.

Our starting plasmids in the construction of pTATΔcys-HE2.85 and pTATΔcys-HE2.121 were, respectively, pET8c-85 and pET8c-123 (both described above). We digested pET8c-85 and pET8c-123 with BglII and NcoI, and isolated the large fragment in each case (4769 base pairs from pET8c-85 or 4880 base pairs from pET8c-123) for use as a vector. In both vectors, the E2 coding regions begin at the NcoI site. Into both vectors, we inserted the 220 bp BglII-AatII fragment from plasmid pTATΔcys, and a synthetic fragment. The 5' end of the BglII-AatII fragment is upstream of the T7 promoter and encodes the first 40 residues of tatΔcys (i.e., residues 1-21, 38-56). The synthetic fragment consisting of annealed oligonucleotides 374.67 (SEQ ID NO:25) and 374.68 (SEQ ID NO:26), encoded tat residues 57-72, with an AatII overhand at the 5' end and an NcoI overhand at the 3' end.

JB Series of Genetic Fusions

Plasmid pJB106 encodes a fusion protein (Figure 12) (SEQ ID NO:38) in which an amino-terminal methionine residue is followed by tat residues 47-58 and then HPV-16 E2 residues 245-365. To obtain pJB106, we carried out a three-way ligation, schematically depicted in Figure 11. We generated a 4602 base pair vector fragment by digesting plasmid pET8c with NcoI and BamHI. One insert was a 359 base pair MspI-BamHI fragment from pET8c-123, encoding HPV-16 E2 residues 248-365. The other insert was a synthetic fragment consisting of the annealed oligonucleotide pair, 374.185 (SEQ ID NO:27) and 374.186 (SEQ ID NO:28). The synthetic fragment encoded the amino-terminal methionine and tat residues 47-58, plus HPV16 residues 245-247 (i.e., ProAspThr). The synthetic fragment had an NcoI overhang at the 5' end and an MspI overhang at the 3' end.

We obtained plasmids pJB117 (SEQ ID NO:59), pJB118 (SEQ ID NO:60), pJB119 (SEQ ID NO:61), pJB120 (SEQ ID NO:62) and pJB122 (SEQ ID NO:63) by PCR deletion cloning in a manner similar to that used for pTATΔcys (described above and in Figure 8). We constructed plasmids pJB117 and pJB118 by deleting segments of pTATΔcys-HE2.121. We constructed plasmids pJB119 and pJB120 by deleting segments of pTATΔcys-161. In all four clonings, we used PCR primer 374.122 (SEQ ID NO:29) to cover the HindIII site downstream of the tat-E2 coding region. In each case, the other primer spanned the NdeI site at the start of the tatΔcys coding sequence, and deleted codons for residues at the beginning of tatΔcys (i.e., residues 2-21 and 38-46 for pJB117 and pJB119; and residues 2-21 for pJB118 and pJB120). For deletion of residues 2-21, we used primer 379.11 (SEQ ID NO:30). For deletion of residues 2-21 and 38-46, we used primer 379.12 (SEQ ID NO:31). Following the PCR reaction, we digested the PCR products with NdeI and HindIII. We then cloned the resulting restriction fragments into vector pTATΔcys-HE2.121, which had been previously digested with NdeI plus HindIII to yield a 4057 base pair receptor fragment. Thus, we constructed expression plasmids encoding fusion proteins consisting of amino acid residues as follows:

JB117 = Tat47-72-HPV16 E2 245-365;
JB118 = Tat38-72-HPV16 E2 245-365;
JB119 = Tat47-62-BPV1 E2 250-410; and
JB120 = Tat38-62-BPV1 E2 250-410.

We constructed pJB122, encoding tat residues 38-58 followed by HPV16 E2 residues 245-365 (i.e., the E2 carboxy-terminal 121 amino acids), by deleting from pJB118 codons for tat residues 59-72. We carried out this deletion by PCR, using primer 374.13 (SEQ ID NO:32), which covers the AatII site within the tat coding region, and primer 374.14 (SEQ ID NO:33), which covers the AatII site slightly downstream of the unique HindIII site downstream of the Tat-E2 gene. We digested the PCR product with AatII and isolated the resulting restriction fragment. In the final pJB122 construction step, we inserted the isolated AatII fragment into AatII-digested vector pJB118.

It should be noted that in all five of our pJB constructs described above, the tat coding sequence was preceded by a methionine codon for initiation of translation.

Purification of Tat-E2 Fusion Proteins

In all cases, we used E.coli to express our tat-E2 genetic fusions. Our general procedure for tat-E2 protein purification included the following initial steps: pelleting the cells; resuspending them in 8-10 volumes of lysis buffer (25 mM Tris (pH 7.5), 25 mM NaCl, 1 mM DTT, 0.5 mM EDTA) containing protease inhibitors -- generally, 1 mM PMSF, 4 µg/ml E64, 50 µg/ml aprotinin, 50 µg/ml pepstatin A, and 3 mM benzamidine); lysing the cells in a French press (2 passes at 12,000 psi); and centrifuging the lysates at 10,000-12,000 x g for 1 hour (except FTE proteins), at 4° C. Additional steps employed in purifying particular tat-E2 fusion proteins are described below.

E2.103 and E2.249 -- Following centrifugation of the lysate, we loaded the supernatant onto a Fast S Sepharose column and eluted the E2.103 or E2.249 protein with 1 M NaCl. We then further purified the E2.103 or E2.249 by chromatography on a P60 gel filtration column equilibrated with 100 mM HEPES (pH 7.5), 0.1 mM EDTA and 1 mM DTT.

FTE103 -- Following centrifugation of the lysate at 10,000 x g for 10 min. at 4° C, we recovered the FTE103 protein (which precipitated) by resuspending the pellet in 6 M urea and adding solid guanidine-HCl to a final concentration of 7 M. After centrifuging the suspension, we purified the FTE103 protein from the supernatant by chromatography on an A.5M gel filtration column in 6 M guanidine, 50 mM sodium phosphate (pH 5.4), 10 mM DTT. We collected the FTE103-containing fractions from the gel filtration column according to the appearance of a band having an apparent molecular weight of 19 kDa on Coomassie-stained SDS polyacrylamide electrophoresis gels.

FTE403 -- Our purification procedure for FTE403 was essentially the same as that for FTE103, except that FTE403 migrated on the gel filtration column with an apparent molecular weight of 25 kDa.

FTE501 -- Following centrifugation of the lysate at 10,000 x g, for 30 minutes, we resuspended the pellet in 6 M urea, added solid guanidine-HCl to a final concentration of 6 M, and DTT to a concentration of 10 mM. After 30 minutes at 37°C, we clarified the solution by centrifugation at 10,000 x g for 30 minutes. We then loaded the sample onto an A.5 agarose gel filtration column in 6 M guanidine-HCl, 50 mM sodium phosphate (pH 5.4), 10 mM DTT and collected the FTE501-containing fractions from the gel filtration column, according to the appearance of a band having an apparent molecular weight of 40 kDa on Coomassie-stained SDS polyacrylamide electrophoresis gels. We loaded the gel filtration-purified FTE501 onto a $C_{18}$ reverse phase HPLC column and eluted with a gradient of 0-75% acetonitrile in 0.1% trifluoroacetic acid. We collected the FTE501 protein in a single peak with an apparent molecular weight of 40 kDa.

TatΔcys-105 -- Following centrifugation of the lysate, we loaded the supernatant onto a Q-Sepharose column equilibrated with 25 mM Tris (pH 7.5), 0.5 mM EDTA. We loaded the Q-Sepharose column flow-through onto an S-Sepharose column equilibrated with 25 mM MES (pH 6.0), after adjusting the Q-Sepharose column flow-through to about pH 6.0 by adding MES (pH 6.0) to a final concentration of 30 mM. We recovered the tatΔcys-105 protein from the S-Sepharose column by application of sequential NaCl concentration steps in 25 mM MES (pH 6.0). TatΔcys-105 eluted in the pH 6.0 buffer at 800-1000 mM NaCl.

TatΔcys-161 -- Following centrifugation of the lysate, we loaded the supernatant onto an S-Sepharose column equilibrated with 25 mM Tris (pH 7.5), 0.5 mM EDTA. We recovered the tatΔcys-161 from the S-Sepharose column by application of a NaCl step gradient in 25 mM Tris (pH 7.5). TatΔcys-161 eluted in the pH 7.5 buffer at 500-700 mM NaCl.

TatΔcys-249 -- Following centrifugation of the lysate, we loaded the supernatant onto a Q-Sepharose column equilibrated with 25 mM Tris (pH 7.5), 0.5 mM EDTA. We recovered the tatΔcys-249 from the S-Sepharose column by application of a NaCl step gradient in 25 mM Tris (pH 7.5). TatΔcys-249 eluted in the 600-800 mM portion of the NaCl step gradient.

TatΔcys-HE2.85 and TatΔcys-HE2.121 -- Following centrifugation of the lysate, we loaded the supernatant onto a Q-Sepharose column. We loaded the flow-through onto an S-Sepharose column. We recovered the tatΔcys-HE2.85 or tatΔcys-HE2.121 from the S-Sepharose column by application of a NaCl step gradient. Both proteins eluted with 1 M NaCl.

HPV E2 and HPV E2CCSS -- See Example 9 (above).

JB106 -- Following centrifugation of the lysate, and collection of the supernatant, we added NaCl to 300 mM. We loaded the supernatant with added NaCl onto an S-Sepharose column equilibrated with 25 mM HEPES (pH 7.5). We treated the column with sequential salt concentration steps in 25 mM HEPES (pH 7.5), 1.5 mM EDTA, 1 mM DTT. We eluted the JB106 protein from the S-Sepharose column with 1 M NaCl.

JB117 -- Following centrifugation of the lysate, and collection of the supernatant, we added NaCl to 300 mM. Due to precipitation of JB117 at 300 mM NaCl, we diluted the JB117 supernatant to 100 mM NaCl and batch-loaded the protein onto the S-Sepharose column. We eluted JB117 from the S-Sepharose column with 1 M NaCl in 25 mM Tris (pH 7.5), 0.3 mM DTT.

JB118 -- Following centrifugation of the lysate, and collection of the supernatant, we added NaCl to 300 mM. We loaded the supernatant with added NaCl onto an S-Sepharose column equilibrated with 25 mM Tris (pH 7.5). We eluted the JB118 protein from the S-Sepharose column with 1 M NaCl in 25 mM Tris (pH 7.5), 0.3 mM DTT.

JB119, JB120, JB121 and JB122 -- Following centrifugation of the lysate, and collection of the supernatant, we added NaCl to 150 mM for JB119 and JB121, and 200 mM for JB120 and JB122. We loaded the supernatant with added NaCl onto an S-Sepharose column equilibrated with 25 mM Tris (pH 7.5). We eluted proteins JB119, JB120, JB121 and JB122 from the S-Sepharose column with 1 M NaCl in 25 mM Tris (pH 7.5), 0.3 mM DTT.

## EXAMPLE 14

### E2 Repression Assays - Additional Conjugates

We tested our tat-E2 fusion proteins for inhibition of transcriptional activation by the full-length papillomavirus E2 protein ("repression"). We measured E2 repression with a transient co-transfection assay in COS7 cells. The COS7 cells used in this assay were maintained in culture for only short periods of time. We thawed the COS7 cells at passage 13 and used them only through passage 25. Long periods of propagation led to low levels of E2 transcriptional activation and decreased repression and reproducibility. Our repression assay and method of computing repression activity are described in Example 9 (above). For the conjugates TxE2.103, TxE22.249, FTE103, FTE202, FTE403 and FTE501, we substituted the BPV-1 E2 transactivator, in equal amount, for the HPV-16 E2 transactivator. Accordingly, instead of transfecting with the HPV-16 E2 expression plasmid pAHE2, we transfected with the BPV-1 E2 expression plasmid pXB323, which is fully described in United States patent 5,219,990.

The genetic fusion protein JB106 has consistently been our most potent tat-E2 repressor conjugate. Data from a repression assay comparing JB106 and TxHE2CCSS are shown in Table III. Figure 13 graphically depicts the results presented in Table III.

In addition to JB106, several other tat-E2 repressor conjugates have yielded significant repression. As shown in Table IV, TxHE2, TxHE2CCSS, JB117, JB118, JB119, JB120 and JB122 displayed repression levels in the ++ range.

TABLE III

| Protein added (µg/ml) | | cpm-bkgd* | average of duplicates | average cpm-bkgd | % repression |
|---|---|---|---|---|---|
| 0 | | 3,872 | | | |
| 0 | | 3,694 | 3783 | -- | -- |
| 0 | | 17,896 | | | |
| 0 | | 18,891 | 18,393 | 14,610 | -- |
| 1 | JB106 | 16,384 | | | |
| 1 | JB106 | 17,249 | 16,816 | 13,033 | 10.8 |
| 3 | JB106 | 11,456 | | | |
| 3 | JB106 | 10,550 | 11,003 | 7,220 | 50.6 |
| 10 | JB106 | 6,170 | | | |
| 10 | JB106 | 7,006 | 6,588 | 2,805 | 81.0 |
| 30 | JB106 | 4,733 | | | |
| 30 | JB106 | 4,504 | 4,618 | 835 | 94.3 |
| 1 | TxHE2CCSS | 17,478 | | | |
| 1 | TxHE2CCSS | 18,047 | 17,762 | 13,979 | 4.3 |
| 3 | TxHE2CCSS | 14,687 | | | |
| 3 | TxHE2CCSS | 15,643 | 15,165 | 11,382 | 22.1 |
| 10 | TxHE2CCSS | 12,914 | | | |
| 10 | TxHE2CCSS | 12,669 | 12,791 | 9,008 | 38.3 |
| 30 | TxHE2CCSS | 7,956 | | | |
| 30 | TxHE2CCSS | 8,558 | 8,257 | 4,474 | 69.4 |
| 1 | HE2.123 | 18,290 | | | |
| 1 | HE2.123 | 18,744 | 18,517 | 14,734 | 0 |
| 3 | HE2.123 | 17,666 | | | |
| 3 | HE2.123 | 18,976 | 18,321 | 14,538 | 1.3 |
| 10 | HE2.123 | 18,413 | | | |
| 10 | HE2.123 | 17,862 | 18,137 | 14,354 | 2.6 |
| 30 | HE2.123 | 18,255 | | | |
| 30 | HE2.123 | 18,680 | 18,467 | 14,684 | 0.3 |

* Bkgd = 158 cpm.

Table IV summarizes our tat-E2 repressor assay results. Although we tested all of our tat-E2 repressor conjugates in similar assays, the conjugates were not all simultaneously tested in the same assay. Accordingly, we have expressed the level of repression activity, semi-quantitatively, as +++, ++, +, +/-, or -, with +++ being strong repression, and - being no detectable repression. Figure 13 illustrates the repression activity rating system used in Table IV. JB106 exemplifies the +++ activity level. TxHE2CCSS exemplifies the ++ activity level. The negative control, HE2.123, exemplifies the - activity level. The + activity level is intermediate between the activity observed with TxHE2CCSS and HE2.123. The two conjugates whose activity is shown as +/- had weak (but detectable) activity in some assays and no detectable activity in other assays.

TABLE IV

| Protein | Tat residues | E2 residues | Repression Level |
|---|---|---|---|
| TxE2.103 | 37-72 | BPV-1 308-410 | + |
| TxE2.249 | 37-72 | BPV-1 162-410 | - |
| TxHE2 | 37-72 | HPV-16 245-365 | ++ |
| TxHE2CCSS | 37-72 | HPV-16 245-365 | ++ |
| FTE103 | 1-67 | BPV-1 306-410 | - |
| FTE208 | 1-62 | BPV-1 311-410 | - |
| FTE403 | 1-62 | BPV-1 250-410 | - |
| FTE501 | 1-62 | BPV-1 162-410 | - |
| TatΔcys-105 | 1-21,38-67 | BPV-1 306-410 | - |
| TatΔcys-161 | 1-21,38-62 | BPV-1 250-410 | +/- |
| TatΔcys-249 | 1-21,38-62 | BPV-1 162-410 | +/- |
| TatΔcys-HE2.85 | 1-21,38-72 | HPV-16 281-365 | + |
| TatΔcys-HE2.121 | 1-21,38-72 | HPV-16 245-365 | + |
| JB106 | 47-58 | HPV-16 245-365 | +++ |
| JB117 | 47-72 | HPV-16 245-365 | ++ |
| JB118 | 38-72 | HPV-16 245-365 | ++ |
| JB119 | 47-62 | BPV-1 250-410 | ++ |
| JB120 | 38-62 | BPV-1 250-410 | ++ |
| JB122 | 38-58 | HPV-16 245-365 | ++ |

FTE103, FTE403, FTE208 and FTE501, the four conjugates having the tat amino-terminal region (i.e., residues 1-21) and the cysteine-rich region (i.e., residues 22-37) were completely defective for repression. Since we have shown, by indirect immunofluorescence, that FTE501 enters cells, we consider it likely that the E2 repressor activity has been lost in the FTE series as a result of the linkage to the tat transport polypeptide. Our data show that the absence of the cysteine-rich region of the tat moiety generally increased E2 repressor activity. In addition, the absence of the cysteine-rich region in tat-E2 conjugates appeared to increase protein production levels in E.coli, and increase protein solubility, without loss of transport into target cells. Deletion of the amino-terminal region of tat also increased E2 repressor activity. Fusion protein JB106, with only tat residues 47-58, was the most potent of our tat-E2 repressor conjugates. However, absence of the tat cysteine-rich region does not always result in preservation of E2 repressor activity in the conjugate. For example, the chemical conjugate TxE2.249 was insoluble and toxic to cells. Thus, linkage of even a cysteine-free portion of tat may lead to a non-functional E2 repressor conjugate.

EXAMPLE 15

Cleavable E2 Conjugates

Chemical conjugation of tat moieties to E2 protein resulted in at least a 20-fold reduction in binding of the E2 protein to E2 binding sites on DNA (data not shown). Therefore, we conducted experiments to evaluate cleavable cross-linking between the tat transport moiety and the E2 repressor moiety. We tested various cleavable cross-linking methods.

In one series of experiments, we activated the cysteine sulfhydryl groups of HPV E2-CCSS protein with aldrithiol in 100 mM HEPES (pH 7.5), 500 mM NaCl. We isolated the activated E2 repressor by gel filtration chromatography and treated it with tat37-72. We achieved low cross-linking efficiency because of rapid E2-CCSS dimer formation upon treatment with aldrithiol. To avoid this problem, we put the E2-CCSS into 8 M urea, at room temperature, and treated it with aldrithiol at 23°C for 60 minutes under denaturing conditions. We then refolded the E2CCSS-aldrithiol adduct, isolated it by gel filtration chromatography, and then allowed it to react with tat37-72. This procedure resulted in excellent cross-linking. We also cross-linked E2CSSS and E2CCSC to tat37-72, using a modification of the urea method, wherein we used S-Sepharose chromatography instead of gel filtration to isolate the E2-aldrithiol adducts. This modification increased recovery of the adducts and resulted in cross-linkage of approximately 90% of the E2 starting material used in the reaction.

The cleavable tat-E2 conjugates exhibited activity in the repression assay. However, the repression activity of the cleavable conjugates was slightly lower than that of similar conjugates cross-linked irreversibly. The slightly lower activity of the cleavable conjugates may be a reflection of protein half-life in the cells. Tat is relatively stable in cells.

E2 proteins generally have short half-lives in cells. Thus, irreversible cross-linkage between a tat moiety and an E2 moiety may stabilize the E2 moiety.

EXAMPLE 16

Herpes Simplex Virus Repressor Conjugate

Herpes simplex virus ("HSV") encodes a transcriptional activator, VP16, which induces expression of the immediate early HSV genes. Friedman et al. have produced an HSV VP16 repressor by deleting the carboxy-terminal transactivation domain of VP16 ("Expression of a Truncated Viral Trans-Activator Selectively Impedes Lytic Infection by Its Cognate Virus", Nature, 335, pp. 452-54 (1988)). We have produced an HSV-2 VP16 repressor in a similar manner.

To test cellular uptake and VP16 repressor activity of transport polypeptide-VP16 repressor conjugates, we simultaneously transfected a VP16-dependent reporter plasmid and a VP16 repressor plasmid into COS7 cells. Then we exposed the transfected cells to a transport polypeptide-VP16 repressor conjugate or to an appropriate control. The repression assay, described below, was analogous to the E2 repression assay described above, in Example 9.

VP16 Repression Assay Plasmids

Our reporter construct for the VP16 repression assay was plasmid pl75kCAT, obtained from G. Hayward (see, P. O'Hare and G.S. Hayward, "Expression of Recombinant Genes Containing Herpes Simplex Virus Delayed-Early and Immediate-Early Regulatory Regions and Trans Activation by Herpes Virus Infection", J. Virol., 52, pp. 522-31 (1984)). Plasmid p175kCAT contains the HSV-1 IE175 promoter driving a CAT reporter gene.

Our HSV-2 transactivator construct for the VP16 repression assay was plasmid pXB324, which contained the wild-type HSV-2 VP16 gene under the control of the chicken β-actin promoter. We constructed pXB324 by inserting into pXB100 (P. Han et al., "Transactivation of Heterologous Promoters by HIV-1 Tat", Nuc. Acids Res., 19, pp. 7225-29 (1991)), between the XhoI site and BamHI site, a 280 base pair fragment containing the chicken β-actin promoter and a 2318 base pair BamHI-EcoRI fragment from plasmid pCA5 (O'Hare and Hayward, supra) encoding the entire wild type HSV-2 VP16 protein.

Tat-VP16 Repressor Fusion Protein

We produced in bacteria fusion protein tat-VP16R.GF (SEQ ID NO:58), consisting of amino acids 47-58 of HIV tat protein followed by amino acids 43-412 of HSV VP16 protein. For bacterial production of a tat-VP16 repressor fusion protein, we constructed plasmid pET/tat-VP16R.GF, in a three-piece ligation. The first fragment was the vector pET-3d (described above under the alternate designatiion "pET-8c") digested with NcoI and BglII (approximately 4600 base pairs). The second fragment consisted of synthetic oligonucleotides 374.219 (SEQ ID NO:39) and 374.220 (SEQ ID NO:40), annealed to form a double-stranded DNA molecule. The 5' end of the synthetic fragment had an NcoI overhang containing an ATG translation start codon. Following the start codon were codons for tat residues 47-58. Immediately following the tat codons, in frame, were codons for VP16 residues 43-47. The 3' terminus of the synthetic fragment was a blunt end for ligation to the third fragment, an 1134 base pair PvuII-BglII fragment from pXB324R4, containing codons 48-412 of HSV-2 VP16. We derived pXB324R4 from pXB324 (described above). Plasmid pXB324R2 was an intermediate in the construction of pXB324R4.

We constructed pXB324R2 by inserting into pXB100 a 1342 base pair BamHI-AatII fragment, from pXB324, encoding the N-terminal 419 amino acids of HSV-2 VP16. To provide an in-frame stop codon, we used a 73 base pair AatII-EcoRI fragment from pSV2-CAT (C.M. Gorman et al., Molecular & Cellular Biology, 2, pp. 1044-51 (1982)). Thus, pXB324R2 encoded the first 419 amino acids of HSV-2 VP16 and an additional seven non-VP16 amino acids preceding the stop codon. To construct pXB324R4, we carried out a 3-piece ligation involving a 5145 base pair MluI-EcoRI fragment from pXB324R2, and two insert fragments. One insert was a 115 base pair MluI-NspI fragment from pXB324R2, encoding the first 198 residues of VP16. The second insert fragment was a double-stranded synthetic DNA molecule consisting of the synthetic oligonucleotides 374.32 (SEQ ID NO:41) and 374.33 (SEQ ID NO:42). When annealed, these oligonucleotides formed a 5' NspI sticky end and a 3' EcoRI sticky end. This synthetic fragment encoded VP16 residues 399-412, followed by a termination codon. Thus, plasmid pXB324R4 differed from pXB324R2 by lacking codons for VP16 amino acids 413-419 and the seven extraneous amino acids preceding the stop codon.

Purification of tat-VP16R.GF Fusion Protein

We expressed our genetic construct for tat-VP16R.GF in E.coli. We harvested the transformed E.coli by centrifugation; resuspended the cells in 8-10 volumes of lysis buffer (25 mM Tris (pH 7.5), 25 mM NaCl, 1mM DTT, 0.5 mM EDTA, 1 mM PMSF, 4 μg/ml E64, 50 μg/ml aprotinin, 50 μg/ml pepstatin A, and 3 mM benzamidine) ; lysed the cells in a French press (2 passes at 12,000 psi); and centrifuged the lysate at 10,000 to 12,000 x g for 1 hour, at 4°C. Following centrifugation of the lysate, we loaded the supernatant onto a Fast Q-Sepharose column equilibrated with 25 mM Tris (pH 7.5), 0.5 mM EDTA. We loaded the Q-Sepharose flow-through onto a Fast S-Sepharose column equilibrated in 25 mM MES (pH 6.0), 0.1 mM EDTA, 2 mM DTT. We recovered the tat-VP16 fusion protein from the S-Sepharose column with sequential NaCl concentration steps in 25 mM MES (pH 6.0), 0.1 mM EDTA, 2 mM DTT. The tat-VP16 fusion protein eluted in the 600-1000 mM NaCl fractions.

VP16 Repression Assay

We seeded HeLa cells in 24-well culture plates at $10^5$ cells/well. The following day, we transfected the cells, using the DEAE-dextran method, as described by B.R. Cullen, "Use of Eukaryotic Expressioon Technology in the Functional Analysis of Cloned Genes", Meth Enzymol., vol. 152, p. 684 (1987). We precipitated the DNA for the transfections and redissolved it, at a concentration of approximately 100 μg/ml, in 100 mM NaCl, 10 mM Tris (pH 7.5). For each transfection, the DNA-DEAE mix consisted of: 200 ng p175kCAT (+/- 1 ng pXB324) or 200 ng pSV-CAT (control), 1 mg/ml DEAE-dextran, and PBS, to a final volume of 100 μl. We exposed the cells to this mixture for 15-20 minutes, at 37°C, with occasional rocking of the culture plates. We then added to each well, 1 ml fresh DC medium (DMEM + 10% serum) with 80 μM chloroquine. After incubating the cells at 37°C for 2.5 hours, we aspirated the medium from each well and replaced it with fresh DC containing 10% DMSO. After 2.5 minutes at room temperature, we aspirated the DMSO-constaining medium and replaced it with fresh DC containing 0, 10 or 50 μg/ml purified tat-VP16.GF. The following day, we replaced the medium in each well with fresh medium of the same composition. Twenty-four hours later, we lysed the HeLa cells with 0.65% NP-40 (detergent) in 10 mM Tris (pH 8.0), 1 mM EDTA, 150 mM NaCl. We measured the protein concentration in each extract, for sample normalization in the assay.

At a tat-VP16.GF concentration of 50 μg/ml, cellular toxicity interfered with the assay. At a concentration of 10 μg/ml, the tat-VP16.GF fusion protein yielded almost complete repression of VP16-dependent CAT expression, with no visible cell death and approximately 30% repression of non-VP16-dependent CAT expression in controls. Thus, we observed specific repression of VP16-dependent transactivation in addition to a lesser amount non-specific repression.

EXAMPLE 17

Transport polypeptide - DNA Conjugates

Transcriptional activation by a DNA-binding transcription factor can be inhibited by introducting into cells DNA having the binding site for that transcription factor. The transcription factor becomes bound by the introduced DNA and is rendered unavailable to bind at the promoter site where it normally functions. This strategy has been employed to inhibit transcriptional activation of by NF-KB (Bielinska et al., "Regulation of Gene Expression with Double-Stranded Phosphorothioate Oligonucleotides", Science, vol. 250, pp. 997-1000 (1990)). Bielinska et al. observed dose-dependent inhibition when the double stranded DNA was put in the cell culture medium. We conjugated the transport polypeptide tat 37-72 to the double stranded DNA molecule to determine whether such conjugation would enhance the inhibition by increasing the cellular uptake of the DNA.

We purchased four custom-synthesized 39-mer phosphorothioate oligonucleotides designated NF1, NF2, NF3 and NF4, having nucleotide sequences (SEQ ID NO:43), (SEQ ID NO:44), (SEQ ID NO:45) and (SEQ ID NO:46), respectively. NF1 and NF2 form a duplex corresponding to the wild type NF-κB binding site. NF3 and NF4 form a duplex corresponding to a mutant NF-κB binding site.

We dissolved NF1 and NF3 in water, at a concentration of approximately 4 mg/ml. We then put 800 μg of NF1 and NF3 separately into 400 μl of 50 mM triethanolamine (pH 8.2), 50 mM NaCl, 10 mM Traut's reagent. We allowed the reaction to proceed for 50 minutes at room temperature. We stopped the reaction by gel filtration on a P6DG column (BioRad, Richmond, CA) equilibrated with 50 mM HEPES (pH 6.0), 50mM NaCl, to remove excess Traut's reagent. We monitored 260 nm absorbance to identify the oligonucleotide-containing fractions. Our recovery of the oligonucleotides was approximately 75%. We then annealed Traut-modified NF1 with NF2 (0.55 mg/ml final concentration) and annealed Traut-modified NF3 with NF4 0.50 mg/ml final concentration). Finally, we allowed 0.4 mg of each Traut-modified DNA to react with 0.6 mg of tat37-72-BMH (prepared as described in Example 9, above), in 1 ml of 100 mM HEPES (pH 7.5), for 60 minutes at room temperature. We monitored the extent of the cross-linking reaction by polyacrylamide gel electrophoresis followed by ethidium bromide staining of the gel. In general, we observed that about 50% of the DNA was modified under these conditions.

These double-stranded DNA molecules were tested, essentially according to the methods of Bielinska et al. (supra), with and without tat linkage, for inhibition of NF-κB transcriptional activation. Tat linkage significantly enhanced the transactivation by NF-κB.

Recombinant DNA sequences prepared by the processes described herein are exemplified by a culture deposited in the American Type Culture Collection, Rockville, Maryland. The Escherichia coli culture identified as pJB106 was deposited on July 28, 1993 and assigned ATCC accession number 69368.

While we have described a number of embodiments of this invention, it is apparent that our basic constructions can be altered to provide other embodiments that utilize the processes and products of this invention. Therefore, it will be appreciated that the scope of this invention is to be defined by the appended claims rather than by the specific embodiments that have been presented by way of example.

## Claims

1. A fusion protein consisting of a carboxy-terminal cargo moiety and an amino-terminal transport moiety, wherein

   (a) the transport moiety is characterized by:

      (i) the presence of amino acids 49-57 of HIV tat protein;
      (ii) the absence of amino acids 22-36 of HIV tat protein; and
      (iii) the absence of amino acids 73-86 of HIV tat protein; and

   (b) the cargo moiety retains biological activity following transport moiety-dependent intracellular delivery.

2. The fusion protein according to claim 1, wherein the cargo moiety is selected from the group consisting of therapeutic molecules, prophylactic molecules and diagnostic molecules.

3. The fusion protein according to claim 1 or 2, wherein the cargo moiety consists of human papillomavirus E2 repressor and the transport moiety is selected from the group consisting of:

   (a) amino acids 47-58 of HIV tat protein (SEQ ID NO: 47);
   (b) amino acids 47-72 of HIV tat protein (SEQ ID NO: 48);
   (c) amino acids 38-72 of HIV tat protein (SEQ ID NO: 49); and
   (d) amino acids 38-58 of HIV tat protein (SEQ ID NO: 50).

4. The fusion protein according to any one of claims 1 to 3, wherein the cargo moiety consists of amino acids 245-365 of the human papillomavirus E2 protein (SEQ ID NO: 51).

5. The fusion protein according to claim 4 selected from the group consisting of JB106 having SEQ ID NO: 38, JB117 having SEQ ID NO: 59, JB118 having SEQ ID NO: 60, JB122 having SEQ ID NO: 63.

6. The fusion protein according to claim 1 or 2, wherein the cargo moiety consists of a bovine papillomavirus E2 repressor and the transport moiety is selected from the group consisting of:

(a) amino acids 47-62 of HIV tat protein (SEQ ID NO: 52); and
(b) amino acids 38-62 of HIV tat protein (SEQ ID NO: 53).

**7.** The fusion protein according to any one of claims 1, 2 or 6, wherein the cargo moiety is an E2 repressor consisting of amino acids 250-410 of the bovine papillomavirus E2 protein (SEQ ID NO: 56).

**8.** The fusion protein according to claim 7 which is JB119 having SEQ ID NO: 61 or JB120 having SEQ ID NO: 62.

**9.** The fusion protein of claim 1 or 2, wherein the cargo moiety consists of amino acids 43-412 of HSV VP16 protein and the transport moiety consists of amino acids 47-58 of HIV tat protein.

**10.** The fusion protein according to any one of claims 1 to 9, wherein the transport moiety is preceded by an amino-terminal methionine.

**11.** A DNA molecule comprising a nucleotide sequence encoding a fusion protein according to claim 5 or 8.

**12.** A DNA molecule comprising a nucleotide sequence encoding fusion protein tat-VP16R.GF having SEQ ID NO: 58.

**13.** The DNA molecule according to claim 11 or 12, wherein the nucleotide sequence encoding the fusion protein is operatively linked to expression control sequences.

**14.** A unicellular host transformed with a DNA molecule according to claim 13.

**15.** A method for producing a fusion protein according to any one of claims 5, 8 or 9 comprising the steps of:

(a) culturing a transformed unicellular host according to claim 14; and
(b) recovering the fusion protein from said culture.

**16.** A covalently linked chemical conjugate consisting of a transport polypeptide moiety and a cargo moiety, wherein:

(a) the transport polypeptide moiety of the conjugate is characterized by:

(i) the presence of amino acids 49-57 of HIV tat protein;
(ii) the absence of amino acids 22-36 of HIV tat protein; and
(iii) the absence of amino acids 73-86 of HIV tat protein; and

(b) the cargo moiety of the conjugate retains biological activity following transport moiety-dependent intracellular delivery.

**17.** The covalently linked chemical conjugate according to claim 16, wherein the transport polypeptide moiety consists of amino acids 37-72 of HIV tat protein (SEQ ID NO: 2).

**18.** The covalently linked chemical conjugate according to claim 17, wherein the cargo moiety is selected from the group consisting of:

(a) amino acids 245-365 of human papillomavirus E2 protein (SEQ ID NO: 51); and
(b) amino acids 245-365 of human papillomavirus E2 protein, wherein amino acids 300 and 309 have been changed to cysteine (SEQ ID NO: 55).

**19.** The covalently linked chemical conjugate according to claim 17, wherein the cargo moiety is a double-stranded DNA selected from the group consisting of

(a) oligonucleotide NF1 having SEQ ID NO: 43 annealed to oligonucleotide NF2 having SEQ ID NO: 44 and
(b) oligonucleotide NF3 having SEQ ID NO: 45 annealed to oligonucleotide NF4 having SEQ ID NO: 46.

**20.** A pharmaceutical composition comprising a pharmaceutically effective amount of a fusion protein according to any one of claims 1 to 10 or a covalently linked chemical conjugate according to any one of claims 16 to 19.

**21.** Use of a fusion protein according to any one of claims 1 to 10 or a covalently linked chemical conjugate according to any one of claims 16 to 19 for the preparation of a pharmaceutical composition for the intracellular delivery of cargo.

**22.** A method for producing a fusion protein consisting of a carboxy-terminal cargo moiety and an amino-terminal transport moiety, characterized by the step of genetically fusing

(a) a transport moiety that is characterized by:

(i) the presence of amino acids 49-57 of HIV tat protein;
(ii) the absence of amino acids 22-36 of HIV tat protein; and
(iii) the absence of amino acids 73-86 of HIV tat protein; and

(b) a cargo moiety that retains biological activity following transport moiety-dependent intracellular delivery.

**23.** The method according to claim 22, wherein the cargo moiety is selected from the group consisting of therapeutic molecules, prophylactic molecules and diagnostic molecules.

**24.** The method according to claim 22 or 23, wherein the cargo moiety consists of human papillomavirus E2 repressor and the transport moiety is selected from the group consisting of:

(a) amino acids 47-58 of HIV tat protein (SEQ ID NO: 47);
(b) amino acids 47-72 of HIV tat protein (SEQ ID NO: 48);
(c) amino acids 38-72 of HIV tat protein (SEQ ID NO: 49); and
(d) amino acids 38-58 of HIV tat protein (SEQ ID NO: 50).

**25.** The method according to any one of claims 22 to 24, wherein the cargo moiety consists of amino acids 245-365 of the human papillomavirus E2 protein (SEQ ID NO: 51).

**26.** The method according to claim 25, wherein said fusion protein is selected from the group consisting of JB106 having SEQ ID NO: 38, JB117 having SEQ ID NO: 59, JB118 having SEQ ID NO: 60, JB122 having SEQ ID NO: 63.

**27.** The method according to claim 22 or 23, wherein the cargo moiety consists of a bovine papillomavirus E2 repressor and the transport moiety is selected from the group consisting of:

(a) amino acids 47-62 of HIV tat protein (SEQ ID NO: 52); and
(b) amino acids 38-62 of HIV tat protein (SEQ ID NO: 53).

**28.** The method according to any one of claims 22, 23 or 27, wherein the cargo moiety is an E2 repressor consisting of amino acids 250-410 of the bovine papillomavirus E2 protein (SEQ ID NO: 56).

**29.** The method according to claim 28, wherein said fusion protein is JB119 having SEQ ID NO: 61 or JB120 having SEQ ID NO: 62.

**30.** The method of claim 22 or 23, wherein the cargo moiety consists of amino acids 43-412 of HSV VP16 protein and the transport moiety consists of amino acids 47-58 of HIV tat protein.

**31.** The method according to any one of claims 22 to 30, wherein the transport moiety is preceded by an amino-terminal methionine.

**32.** A method for producing a DNA molecule comprising a nucleotide sequence encoding a fusion protein consisting of a carboxy-terminal cargo moiety and an amino-terminal transport moiety comprising the step of introducing into a plasmid a nucleotide sequence encoding a fusion protein produced by the method according to claim 26 or 29.

**33.** A method for producing a DNA molecule comprising a nucleotide sequence encoding a fusion protein consisting of a carboxy-terminal cargo moiety and an amino-terminal transport moiety comprising the step of introducing into a plasmid a nucleotide sequence encoding fusion protein tat-VP16R.GF having SEQ ID NO: 58.

**34.** The method according to claim 32 or 33, wherein the nucleotide sequence encoding the fusion protein is operatively linked to expression control sequences.

**35.** A method for transforming a unicellular host comprising the step of introducing into said host a DNA molecule produced by the method according to claim 34.

**36.** A method for producing a fusion protein according to any one of claims 26, 29 or 30 comprising the steps of:

(a) culturing a transformed unicellular host produced by the method according to claim 35; and
(b) recovering the fusion protein from said culture.

**37.** A method for producing a covalently linked chemical conjugate consisting of a transport polypeptide moiety and a cargo moiety, comprising the step of linking of:

(a) a transport polypeptide moiety that is characterized by:

(i) the presence of amino acids 49-57 of HIV tat protein;
(ii) the absence of amino acids 22-36 of HIV tat protein; and
(iii) the absence of amino acids 73-86 of HIV tat protein; and

(b) a cargo moiety that retains biological activity following transport moiety-dependent intracellular delivery.

**38.** The method according to claim 37, wherein the transport polypeptide moiety consists of amino acids 37-72 of HIV tat protein (SEQ ID NO: 2).

**39.** The method according to claim 38, wherein the cargo moiety is selected from the group consisting of:

(a) amino acids 245-365 of human papillomavirus E2 protein (SEQ ID NO: 51); and
(b) amino acids 245-365 of human papillomavirus E2 protein, wherein amino acids 300 and 309 have been changed to cysteine (SEQ ID NO: 55).

**40.** The method according to claim 38, wherein the cargo moiety is a double-stranded DNA selected from the group consisting of

(a) oligonucleotide NF1 having SEQ ID NO: 43 annealed to oligonucleotide NF2 having SEQ ID NO: 44 and
(b) oligonucleotide NF3 having SEQ ID NO: 45 annealed to oligonucleotide NF4 having SEQ ID NO: 46.

**41.** A method for the preparation of a pharmaceutical composition comprising a pharmaceutically effective amount of a fusion protein produced by the method according to any one of claims 22 to 31 or a covalently linked chemical conjugate produced by the method according to any one of claims 37 to 40, wherein said fusion protein or said covalently linked chemical conjugate is formulated with a pharmaceutically acceptable carrier.

**Patentansprüche**

**1.** Fusionsprotein, bestehend aus einer carboxyterminalen Frachteinheit und einer aminoterminalen Transporteinheit, wobei

(a) die Transporteinheit gekennzeichnet ist durch:

(i) das Vorhandensein der Aminosäuren 49 bis 57 des HIV-tat-Proteins;
(ii) das Fehlen der Aminosäuren 22 bis 36 des HIV-tat-Proteins; und
(iii) das Fehlen der Aminosäuren 73 bis 86 des HIV-tat-Proteins; und

(b) die Frachteinheit die biologische Aktivität nach der Transporteinheit-abhängigen intrazellulären Ablieferung behält.

**2.** Fusionsprotein nach Anspruch 1, wobei die Frachteinheit ein therapeutisches, prophylaktisches oder diagnostisches Molekül ist.

3. Fusionsprotein nach Anspruch 1 oder 2, wobei die Frachteinheit aus dem menschlichen Papillomavirus E2-Repressor besteht und die Transporteinheit:

   (a) die Aminosäuren 47 bis 58 des HIV-tat-Proteins (SEQ ID NR: 47);
   (b) die Aminosäuren 47 bis 72 des HIV-tat-Proteins (SEQ ID NR: 48);
   (c) die Aminosäuren 38 bis 72 des HIV-tat-Proteins (SEQ ID NR: 49); oder
   (d) die Aminosäuren 38 bis 58 des HIV-tat-Proteins (SEQ ID NR: 50)

   aufweist.

4. Fusionsprotein nach einem der Ansprüche 1 bis 3, wobei die Frachteinheit aus den Aminosäuren 245 bis 365 des menschlichen Papillomavirus E2-Proteins (SEQ ID NR: 51) besteht.

5. Fusionsprotein nach Anspruch 4, ausgewählt aus JB106, das die Sequenz der SEQ ID NR: 38 aufweist, JB117, das die Sequenz der SEQ ID NR: 59 aufweist, JB118, das die Sequenz der SEQ ID NR: 60 aufweist, oder JB122, das die Sequenz der SEQ ID NR: 63 aufweist.

6. Fusionsprotein nach Anspruch 1 oder 2, wobei die Frachteinheit aus dem Rinderpapillomavirus E2-Repressor besteht und die Transporteinheit:

   (a) die Aminosäuren 47 bis 62 des HIV-tat-Proteins (SEQ ID NR: 52); oder
   (b) die Aminosäuren 38 bis 62 des HIV-tat-Proteins (SEQ ID NR: 53)

   aufweist.

7. Fusionsprotein nach einem der Ansprüche 1, 2 oder 6, wobei die Frachteinheit ein E2-Repressor bestehend aus den Aminosäuren 250 bis 410 des Rinderpapillomavirus E2-Proteins (SEQ ID NR: 56) ist.

8. Fusionsprotein nach Anspruch 7, das JB119, das die Sequenz der SEQ ID NR: 61 aufweist, oder JB120, das die Sequenz der SEQ ID NR: 62 aufweist, ist.

9. Fusionsprotein nach Anspruch 1 oder 2, wobei die Frachteinheit aus den Aminosäuren 43 bis 412 des HSV-VP16-Proteins und die Transporteinheit aus den Aminosäuren 47 bis 58 des HIV-tat-Proteins besteht.

10. Fusionsprotein nach einem der Ansprüche 1 bis 9, wobei der Transporteinheit ein aminoterminales Methionin vorausgeht.

11. DNA-Molekül, umfassend eine Nucleotidsequenz, die ein Fusionsprotein nach Anspruch 5 oder 8 codiert.

12. DNA-Molekül, umfassend eine Nucleotidsequenz, die ein Fusionsprotein tat-VP16R.GF codiert, das die Sequenz der SEQ ID NR: 58 aufweist.

13. DNA-Molekül nach Anspruch 11 oder 12, wobei die Nucleotidsequenz, die das Fusionsprotein codiert, funktionell mit Expressionskontrollsequenzen verbunden ist.

14. Einzelliger Wirt, der mit einem DNA-Molekül nach Anspruch 13 transformiert ist.

15. Verfahren zur Herstellung eines Fusionsproteins nach einem der Ansprüche 5, 8 oder 9, umfassend die Schritte:

   (a) Züchtung eines transformierten einzelligen Wirts nach Anspruch 13; und
   (b) Gewinnung des Fusionsproteins aus der Kultur.

16. Kovalent verknüpftes chemisches Konjugat, bestehend aus einer Transportpolypeptideinheit und einer Frachteinheit, wobei:

   (a) die Transportpolypeptideinheit des Konjugats gekennzeichnet ist durch:

(i) das Vorhandensein der Aminosäuren 49 bis 57 des HIV-tat-Proteins;
(ii) das Fehlen der Aminosäuren 22 bis 36 des HIV-tat-Proteins; und
(iii) das Fehlen der Aminosäuren 73 bis 86 des HIV-tat-Proteins; und

(b) die Frachteinheit des Konjugats die biologische Aktivität nach der Transporteinheit-abhängigen intrazellulären Ablieferung behält.

**17.** Kovalent verknüpftes chemisches Konjugat nach Anspruch 16, wobei die Transportpolypeptideinheit aus den Aminosäuren 37 bis 72 des HIV-tat-Proteins (SEQ ID NR: 2) besteht.

**18.** Kovalent verknüpftes chemisches Konjugat nach Anspruch 17, wobei die Frachteinheit:

(a) die Aminosäuren 245 bis 365 des menschlichen Papillomavirus E2-Proteins (SEQ ID NR: 51); oder
(b) die Aminosäuren 245 bis 365 des menschlichen Papillomavirus E2-Proteins aufweist, wobei die Aminosäuren 300 und 309 durch Cystein ersetzt wurden (SEQ ID NR: 55).

**19.** Kovalent verknüpftes chemisches Konjugat nach Anspruch 17, wobei die Frachteinheit eine doppelsträngige DNA ist, ausgewählt aus:

(a) Oligonucleotid NF1, das die Sequenz der SEQ ID NR: 43 aufweist, aneliert an Oligonucleotid NF2, das die Sequenz der SEQ ID NR: 44 aufweist; und
(b) Oligonucleotid NF3, das die Sequenz der SEQ ID NR: 45 aufweist, aneliert an Oligonucleotid NF4, das die Sequenz der SEQ ID NR: 46 aufweist.

**20.** Arzneimittel, umfassend eine pharmazeutisch wirksame Menge eines Fusionsproteins nach einem der Ansprüche 1 bis 10 oder eines kovalent verknüpften chemischen Konjugats nach einem der Ansprüche 16 bis 19.

**21.** Verwendung eines Fusionsproteins nach einem der Ansprüche 1 bis 10 oder eines kovalent verknüpften chemischen Konjugats nach einem der Ansprüche 16 bis 19 zur Herstellung eines Arzneimittels zur intrazellulären Ablieferung eines Frachtmoleküls.

**22.** Verfahren zur Herstellung eines Fusionsproteins bestehend aus einer carboxyterminalen Frachteinheit und einer aminoterminalen Transporteinheit, gekennzeichnet durch den Schritt einer genetischen Fusion von:

(a) einer Transporteinheit, die gekennzeichnet ist durch:

(i) das Vorhandensein der Aminosäuren 49 bis 57 des HIV-tat-Proteins;
(ii) das Fehlen der Aminosäuren 22 bis 36 des HIV-tat-Proteins; und
(iii) das Fehlen der Aminosäuren 73 bis 86 des HIV-tat-Proteins; und

(b) einer Frachteinheit, die die biologische Aktivität nach der Transporteinheit-abhängigen intrazellulären Ablieferung behält.

**23.** Verfahren nach Anspruch 22, wobei die Frachteinheit ein therapeutisches, prophylaktisches oder diagnostisches Molekül ist.

**24.** Verfahren nach Anspruch 22 oder 23, wobei die Frachteinheit aus dem menschlichen Papillomavirus E2-Repressor besteht und die Transporteinheit:

(a) die Aminosäuren 47 bis 58 des HIV-tat-Proteins (SEQ ID NR: 47);
(b) die Aminosäuren 47 bis 72 des HIV-tat-Proteins (SEQ ID NR: 48);
(c) die Aminosäuren 38 bis 72 des HIV-tat-Proteins (SEQ ID NR: 49); oder
(d) die Aminosäuren 38 bis 58 des HIV-tat-Proteins (SEQ ID NR: 50)

aufweist.

**25.** Verfahren nach einem der Ansprüche 22 bis 24, wobei die Frachteinheit aus den Aminosäuren 245 bis 365 des menschlichen Papillomavirus E2-Proteins (SEQ ID NR: 51) besteht.

**26.** Verfahren nach Anspruch 25, wobei das Fusionsprotein ausgewählt ist aus JB106, das die Sequenz der SEQ ID NR: 38 aufweist, JB117, das die Sequenz der SEQ ID NR: 59 aufweist, JB118, das die Sequenz der SEQ ID NR: 60 aufweist, und JB122, das die Sequenz der SEQ ID NR: 63 aufweist.

**27.** Verfahren nach Anspruch 22 oder 23, wobei die Frachteinheit aus dem Rinderpapillomavirus E2-Repressor besteht und die Transporteinheit:

(a) die Aminosäuren 47 bis 62 des HIV-tat-Proteins (SEQ ID NR: 52); oder
(b) die Aminosäuren 38 bis 62 des HIV-tat-Proteins (SEQ ID NR: 53)

aufweist.

**28.** Verfahren nach einem der Ansprüche 22, 23 oder 27, wobei die Frachteinheit ein E2-Repressor bestehend aus den Aminosäuren 250 bis 410 des Rinderpapillomavirus E2-Proteins (SEQ ID NR: 56) ist.

**29.** Verfahren nach Anspruch 28, wobei das Fusionsprotein JB119, das die Sequenz der SEQ ID NR: 61 aufweist, oder JB120 ist, das die Sequenz der SEQ ID NR: 62 aufweist.

**30.** Verfahren nach Anspruch 22 oder 23, wobei die Frachteinheit aus den Aminosäuren 43 bis 412 des HSV-VP16-Proteins und die Transporteinheit aus den Aminosäuren 47 bis 58 des HIV-tat-Proteins besteht.

**31.** Verfahren nach einem der Ansprüche 22 bis 30, wobei der Transporteinheit ein aminoterminales Methionin vorausgeht.

**32.** Verfahren zur Herstellung eines DNA-Moleküls, umfassend eine Nucleotidsequenz, die ein Fusionsprotein codiert, das aus einer carboxyterminalen Frachteinheit und einer aminoterminalen Transporteinheit besteht, umfassend den Schritt der Einbringung einer Nucleotidsequenz, die ein Fusionsprotein codiert, das mittels der Methode nach Anspruch 26 oder 29 hergestellt ist, in ein Plasmid.

**33.** Verfahren zur Herstellung eines DNA-Moleküls, umfassend eine Nucleotidsequenz, die ein Fusionsprotein codiert, das aus einer carboxyterminalen Frachteinheit und einer aminoterminalen Transporteinheit besteht, umfassend den Schritt der Einbringung einer Nucleotidsequenz, die das Fusionsprotein tat-VP16R.GF codiert, das die Sequenz der SEQ ID NR: 58 aufweist, in ein Plasmid.

**34.** Verfahren nach Anspruch 32 oder 33, wobei die Nucleotidsequenz, die das Fusionsprotein codiert, funktionell mit Expressionskontrollsequenzen verbunden ist.

**35.** Verfahren zur Transformation eines einzelligen Wirts, umfassend den Schritt der Einbringung eines DNA-Moleküls, das mittels der Methode nach Anspruch 34 hergestellt ist, in den Wirt.

**36.** Verfahren zur Herstellung eines Fusionsproteins nach einem der Ansprüche 26, 29 oder 30, umfassend die Schritte:

(a) Züchtung eines transformierten einzelligen Wirts, der mittels der Methode nach Anspruch 35 hergestellt ist; und
(b) Gewinnung des Fusionsproteins aus der Kultur.

**37.** Verfahren zur Herstellung eines kovalent verknüpften chemischen Konjugats, das aus einer Transportpolypeptideinheit und einer Frachteinheit besteht, umfassend den Schritt der Verknüpfung von:

(a) einer Transportpolypeptideinheit, die gekennzeichnet ist durch:

(i) das Vorhandensein der Aminosäuren 49 bis 57 des HIV-tat-Proteins;
(ii) das Fehlen der Aminosäuren 22 bis 36 des HIV-tat-Proteins; und
(iii) das Fehlen der Aminosäuren 73 bis 86 des HIV-tat-Proteins; und

(b) einer Frachteinheit, die die biologische Aktivität nach der Transporteinheit-abhängigen intrazellulären Ablieferung behält.

**38.** Verfahren nach Anspruch 37, wobei die Transportpolypeptideinheit aus den Aminosäuren 37 bis 72 des HIV-tat-Proteins (SEQ ID NR: 2) besteht.

**39.** Verfahren nach Anspruch 38, wobei die Frachteinheit:

(a) die Aminosäuren 245 bis 365 des menschlichen Papillomavirus E2-Proteins (SEQ ID NR: 51); oder
(b) die Aminosäuren 245 bis 365 des menschlichen Papillomavirus E2-Proteins aufweist, wobei die Aminosäuren 300 und 309 durch Cystein ersetzt wurden (SEQ ID NR: 55).

**40.** Verfahren nach Anspruch 38, wobei die Frachteinheit eine doppelsträngige DNA ist, ausgewählt aus:

(a) Oligonucleotid NF1, das die Sequenz der SEQ ID NR: 43 aufweist, aneliert an Oligonucleotid NF2, das die Sequenz der SEQ ID NR: 44 aufweist; und
(b) Oligonucleotid NF3, das die Sequenz der SEQ ID NR: 45 aufweist, aneliert an Oligonucleotid NF4, das die Sequenz der SEQ ID NR: 46 aufweist.

**41.** Verfahren zur Herstellung eines Arzneimittels, umfassend eine pharmazeutisch wirksame Menge eines Fusionsproteins, das mittels des Verfahrens nach einem der Ansprüche 22 bis 31 hergestellt ist, oder eines kovalent verknüpften chemischen Konjugats, das mittels des Verfahrens nach einem der Ansprüche 37 bis 40 hergestellt ist, wobei das Fusionsprotein oder das kovalent verknüpfte chemische Konjugat mit einem pharmazeutisch verträglichen Träger formuliert ist.

## Revendications

**1.** Protéine de fusion consistant en un fragment de chargement à terminaison carboxy et un fragment de transport à terminaison amino, dans laquelle

(a) le fragment de transport est caractérisé par :

(i) la présence des acides aminés 49-57 de la protéine tat du VIH;
(ii) l'absence des acides aminés 22-36 de la protéine tat du VIH ; et
(iii) l'absence des acides aminés 73-86 de la protéine tat du VIH ; et

(b) le fragment de chargement conserve l'activité biologique à la suite de la distribution intracellulaire dépendant du fragment de transport.

**2.** Protéine de fusion selon la revendication 1, dans laquelle le fragment de chargement est choisi parmi des molécules thérapeutiques, des molécules prophylactiques et des molécules de diagnostic.

**3.** Protéine de fusion selon la revendication 1 ou 2, dans laquelle le fragment de chargement consiste en le répresseur E2 du papillomavirus humain et le fragment de transport est choisi parmi :

(a) les acides aminés 47-58 de la protéine tat du VIH (SEQ ID NO: 47)
(b) les acides aminés 47-72 de la protéine tat du VIH (SEQ ID NO: 48)
(c) les acides aminés 38-72 de la protéine tat du VIH (SEQ ID NO: 49) ; et
(d) les acides aminés 38-58 de la protéine tat du VIH (SEQ ID NO:50).

**4.** Protéine de fusion selon l'une quelconque des revendications 1 à 3, dans laquelle le fragment de chargement consiste en les acides aminés 245-365 de la protéine E2 de papillomavirus humain (SEQ ID NO: 51).

**5.** Protéine de fusion selon la revendication 4 choisie parmi JB106 ayant la SEQ ID NO: 38, JB117 ayant la SEQ ID NO: 59, JB118 ayant la SEQ ID NO: 60 et JB122 ayant la SEQ ID NO: 63.

**6.** Protéine de fusion selon la revendication 1 ou 2, dans laquelle le fragment de chargement consiste en un répresseur E2 de papillomavirus bovin et le fragment de transport est choisi parmi :

(a) les acides aminés 47-62 de la protéine tat du VIH (SEQ ID NO: 52) ; et

(b) les acides aminés 38-62 de la protéine tat du VIH (SEQ ID NO: 53).

7. Protéine de fusion selon l'une quelconque des revendications 1, 2 ou 6, dans laquelle le fragment de chargement est un répresseur E2 consistant en les acides aminés 250-410 de la protéine E2 du papillomavirus bovin (SEQ ID NO: 56).

8. Protéine de fusion selon la revendication 7, qui est JB119 ayant la SEQ ID NO: 61 ou JB120 ayant la SEQ ID NO:62.

9. Protéine de fusion selon la revendication 1 ou 2, dans laquelle le fragment de chargement consiste en les acides aminés 43-412 de la protéine VP16 de HSV et le fragment de transport consiste en les acides aminés 47-58 de la protéine tat du VIH.

10. Protéine de fusion selon l'une quelconque des revendications 1 à 9, dans laquelle le fragment de transport est précédé par une méthionine à terminaison amino.

11. Molécule d'ADN comprenant une séquence de nucléotides encodant une protéine de fusion selon la revendication 5 ou 8.

12. Molécule d'ADN comprenant une séquence de nucléotides encodant la protéine de fusion tat-VP16R.GF ayant la SEQ ID NO: 58.

13. Molécule d'ADN selon la revendication 11 ou 12, dans laquelle la séquence de nucléotides encodant la protéine de fusion est liée opérationnellement aux séquences de contrôle d'expression.

14. Hôte monocellulaire transformé avec une molécule d'ADN selon la revendication 13.

15. Procédé de production d'une protéine de fusion selon l'une quelconque des revendications 5, 8 ou 9 comprenant les étapes de :

   (a) culture d'un hôte monocellulaire transformé selon la revendication 13 ; et
   (b) récupération de la protéine de fusion à partir de ladite culture.

16. Conjugué chimique lié de façon covalente consistant en un fragment de polypeptide de transport et en un fragment de chargement, dans lequel :

   (a) le fragment de polypeptide de transport du conjugué est caractérisé par :

      (i) la présence des acides aminés 49-57 de la protéine tat du VIH;
      (ii) l'absence des acides aminés 22-36 de la protéine tat du VIH ; et
      (iii) l'absence des acides aminés 73-86 de la protéine tat du VIH ; et

   (b) le fragment de chargement du conjugué conserve l'activité biologique à la suite de la distribution intracellulaire dépendant du fragment de transport.

17. Conjugué chimique lié de façon covalente selon la revendication 16, dans lequel le fragment de polypeptide de transport consiste en les acides aminés 37-72 de la protéine tat du VIH (SEQ ID NO: 2).

18. Conjugué chimique lié de façon covalente selon la revendication 17, dans lequel le fragment de chargement est choisi parmi :

   (a) les acides aminés 245-365 de la protéine E2 du papillomavirus humain (SEQ ID NO: 51) et
   (b) les acides aminés 245-365 de la protéine E2 du papillomavirus humain, dans lesquels les acides aminés 300 et 309 ont été changés en cystéine (SEQ ID NO: 55).

19. Conjugué chimique lié de façon covalente selon la revendication 17, dans lequel le fragment de chargement est un ADN à double brin choisi parmi

   (a) l'oligonucléotide NF1 ayant la SEQ ID NO: 43 accolé à l'oligonucléotide NF2 ayant la SEQ ID NO: 44 et

(b) l'oligonucléotide NF3 ayant la SEQ ID NO: 45 accolé à l'oligonucléotide NF4 ayant la SEQ ID NO: 46.

**20.** Composition pharmaceutique comprenant une quantité pharmaceutiquement efficace d'une protéine de fusion selon l'une quelconque des revendications 1 à 10 ou d'un conjugué chimique lié de façon covalente selon l'une quelconque des revendications 16 à 19.

**21.** Utilisation d'une protéine de fusion selon l'une quelconque des revendications 1 à 10 ou d'un conjugué chimique lié de façon covalente selon l'une quelconque des revendications 16 à 19 pour la préparation d'une composition pharmaceutique pour la distribution intracellulaire d'un chargement.

**22.** Procédé de production d'une protéine de fusion consistant en un fragment de chargement à terminaison carboxy et en un fragment de transport à terminaison amino, caractérisé par l'étape de fusion génétiquement de

(a) un fragment de transport qui est caractérisé par :

(i) la présence des acides aminés 49-57 de la protéine tat du VIH;
(ii) l'absence des acides aminés 22-36 de la protéine tat du VIH ; et
(iii) l'absence des acides aminés 73-86 de la protéine tat du VIH ; et

(b) un fragment de chargement qui conserve l'activité biologique à la suite de la distribution intracellulaire dépendant du fragment de transport.

**23.** Procédé selon la revendication 22, dans lequel le fragment de chargement est choisi parmi des molécules thérapeutiques, des molécules prophylactiques et des molécules de diagnostic.

**24.** Procédé selon la revendication 22 ou 23, dans lequel le fragment de chargement consiste en le répresseur E2 du papillomavirus humain et le fragment de transport est choisi parmi:

(a) les acides aminés 47-58 de la protéine tat du VIH (SEQ ID NO: 47)
(b) les acides aminés 47-72 de la protéine tat du VIH (SEQ ID NO: 48)
(c) les acides aminés 38-72 de la protéine tat du VIH (SEQ ID NO: 49) ; et
(d) les acides aminés 38-58 de la protéine tat du VIH (SEQ ID NO:50).

**25.** Procédé selon l'une quelconque des revendications 22 à 24, dans lequel le fragment de chargement consiste en les acides aminés 245-365 de la protéine E2 du papillomavirus humain (SEQ ID NO: 51).

**26.** Procédé selon la revendication 25, dans lequel ladite protéine de fusion est choisie parmi JB106 ayant la SEQ ID NO: 38, JB117 ayant la SEQ ID NO: 59, JB118 ayant la SEQ ID NO: 60 et JB122 ayant la SEQ ID NO: 63.

**27.** Procédé selon la revendication 22 ou 23, dans lequel le fragment de chargement consiste en le répresseur E2 du papillomavirus bovin et le fragment de transport est choisi parmi :

(a) les acides aminés 47-62 de la protéine tat du VIH (SEQ ID NO: 52) ; et
(b) les acides aminés 38-62 de la protéine tat du VIH (SEQ ID NO: 53).

**28.** Procédé selon l'une quelconque des revendications 22, 23 ou 27, dans lequel le fragment de chargement est un répresseur E2 consistant en les acides aminés 250-410 de la protéine E2 du papillomavirus bovin (SEQ ID NO: 56).

**29.** Procédé selon la revendication 28, dans lequel ladite protéine de fusion est JB119 ayant la SEQ ID NO: 61 ou JB120 ayant la SEQ ID NO: 62.

**30.** Procédé selon la revendication 22 ou 23, dans lequel le fragment de chargement consiste en les acides aminés 43-412 de la protéine VP16 de HSV et le fragment de transport consiste en les acides aminés 47-58 de la protéine tat du VIH.

**31.** Procédé selon l'une quelconque des revendications 22 à 30, dans lequel le fragment de transport est précédé par une méthionine à terminaison amino.

**32.** Procédé de production d'une molécule d'ADN comprenant une séquence de nucléotides encodant une protéine de fusion consistant en un fragment de chargement à terminaison carboxy et un fragment de transport à terminaison amino comprenant l'étape d'introduire dans un plasmide une séquence de nucléotides encodant une protéine de fusion produite par le procédé selon la revendication 26 ou 29.

**33.** Procédé de production d'une molécule d'ADN comprenant une séquence de nucléotides encodant une protéine de fusion consistant en un fragment de chargement à terminaison carboxy et un fragment de transport à terminaison amino comprenant l'étape d'introduire dans un plasmide une séquence de nucléotides encodant la protéine de fusion tat-VP16R.GF ayant la SEQ ID NO: 58.

**34.** Procédé selon la revendication 32 ou 33, dans lequel la séquence de nucléotides encodant la protéine de fusion est liée opérationnellement aux séquences de contrôle d'expression.

**35.** Procédé de transformation d'un hôte unicellulaire comprenant l'étape d'introduire dans ledit hôte une molécule d'ADN produite par le procédé selon la revendication 34.

**36.** Procédé de production d'une protéine de fusion selon l'une quelconque des revendications 26, 29 ou 30 comprenant les étapes de :

(a) culture d'un hôte monocellulaire transformé produit par le procédé selon la revendication 35 ; et
(b) récupération de la protéine de fusion à partir de ladite culture.

**37.** Procédé de production d'un conjugué chimique lié de façon covalente consistant en un fragment de polypeptide de transport et un fragment de chargement, comprenant l'étape de liaison de :

(a) un fragment de polypeptide de transport qui est caractérisé par :

(i) la présence des acides aminés 49-57 de la protéine tat du VIH;
(ii) l'absence des acides aminés 22-36 de la protéine tat du VIH ; et
(iii) l'absence des acides aminés 73-86 de la protéine tat du VIH ; et

(b) un fragment de chargement qui conserve l'activité biologique à la suite de la distribution intracellulaire dépendant du fragment de transport.

**38.** Procédé selon la revendication 37, dans lequel le fragment de polypeptide de transport consiste en les acides aminés 37-72 de la protéine tat du VIH (SEQ ID NO: 2).

**39.** Procédé selon la revendication 38, dans lequel le fragment de chargement est choisi parmi :

(a) les acides aminés 245-365 de la protéine E2 du papillomavirus humain (SEQ ID NO: 51) et
(b) les acides aminés 245-365 de la protéine E2 du papillomavirus humain, dans lesquels les acides aminés 300 et 309 ont été changés en cystéine (SEQ ID NO: 55).

**40.** Procédé selon la revendication 38, dans lequel le fragment de chargement est un ADN à double brin choisi parmi :

(a) l'oligonucléotide NF1 ayant la SEQ ID NO: 43 accolé à l'oligonucléotide NF2 ayant la SEQ ID NO: 44 et
(b) l'oligonucléotide NF3 ayant la SEQ ID NO: 45 accolé à l'oligonucléotide NF4 ayant la SEQ ID NO: 46.

**41.** Procédé de préparation d'une composition pharmaceutique comprenant une quantité pharmaceutiquement efficace d'une protéine de fusion produite par le procédé selon l'une quelconque des revendications 22 à 31 ou d'un conjugué chimique lié de façon covalente produit par le procédé selon l'une quelconque des revendications 37 à 40, dans lequel ladite protéine de fusion ou ledit conjugué chimique lié de façon covalente est formulé avec un véhicule pharmaceutiquement acceptable.

# FIG. 1

| | | | | | | | | | | | | | | |
|---|---|---|---|---|---|---|---|---|---|---|---|---|---|---|
| Met | Glu | Pro | Val | Asp | Pro | Arg | Leu | Glu | Pro | Trp | Lys | His | Pro | Gly |
| 1 | | | | 5 | | | | | 10 | | | | | 15 |
| Ser | Gln | Pro | Lys | Thr | Ala | Cys | Thr | Asn | Cys | Tyr | Cys | Lys | Lys | Cys |
| | | | | 20 | | | | | 25 | | | | | 30 |
| Cys | Phe | His | Cys | Gln | Val | Cys | Phe | Ile | Thr | Lys | Ala | Leu | Gly | Ile |
| | | | | 35 | | | | | 40 | | | | | 45 |
| Ser | Tyr | Gly | Arg | Lys | Lys | Arg | Arg | Gln | Arg | Arg | Arg | Pro | Pro | Gln |
| | | | | 50 | | | | | 55 | | | | | 60 |
| Gly | Ser | Gln | Thr | His | Gln | Val | Ser | Leu | Ser | Lys | Gln | Pro | Thr | Ser |
| | | | | 65 | | | | | 70 | | | | | 75 |
| Gln | Ser | Arg | Gly | Asp | Pro | Thr | Gly | Pro | Lys | Glu | | | | |
| | | | | 80 | | | | | 85 | | | | | |

FIG. 2

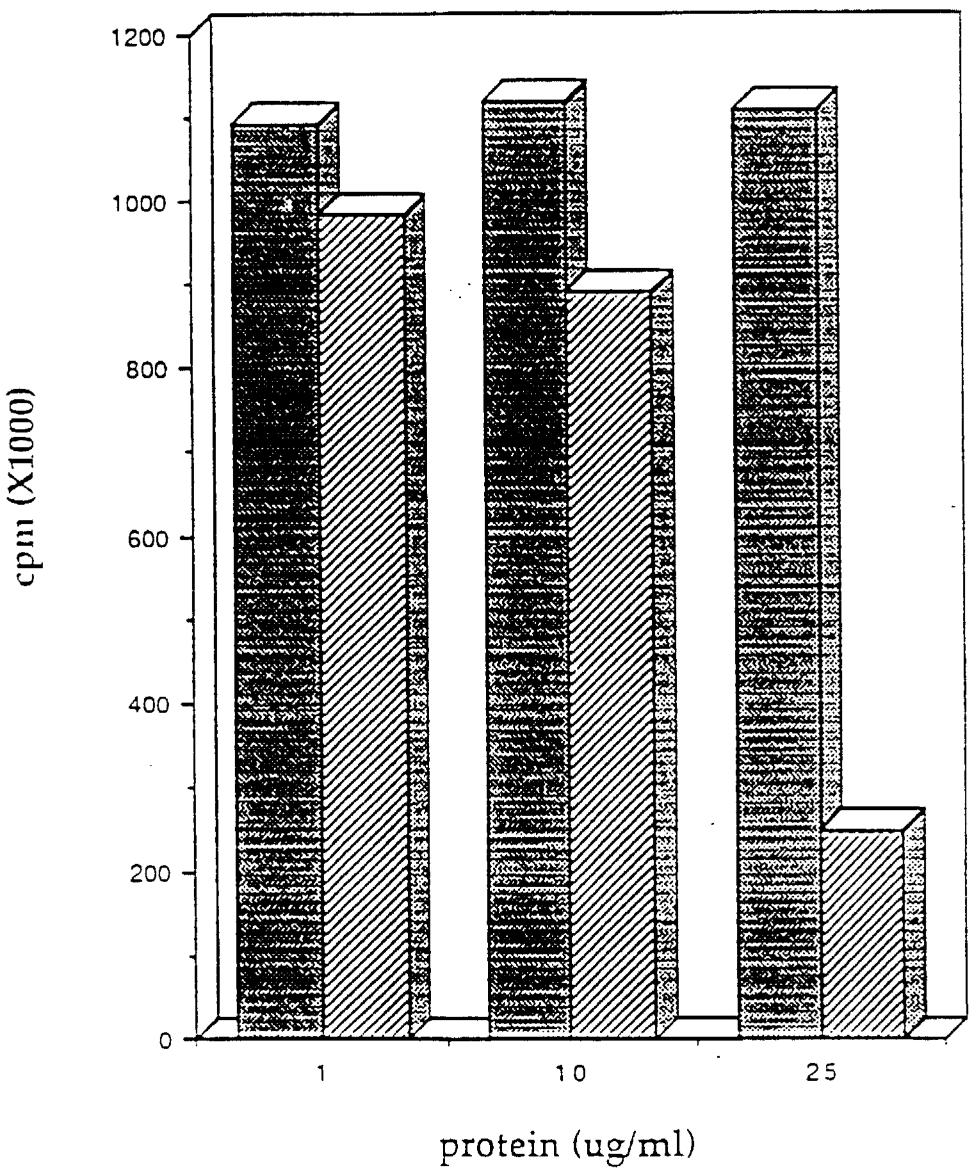

FIG. 3

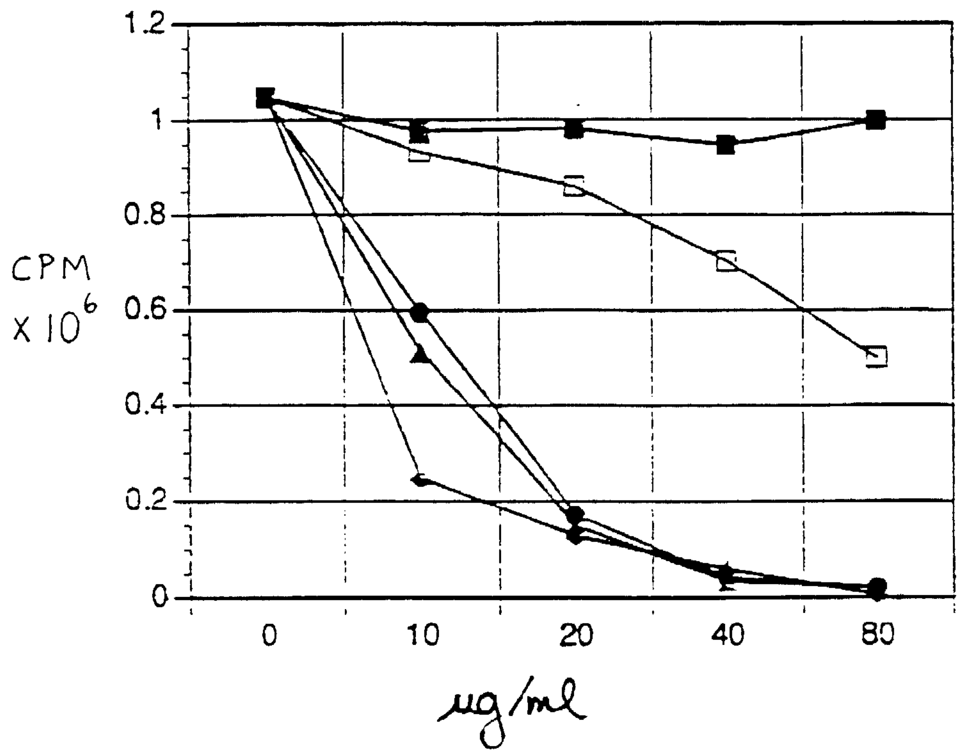

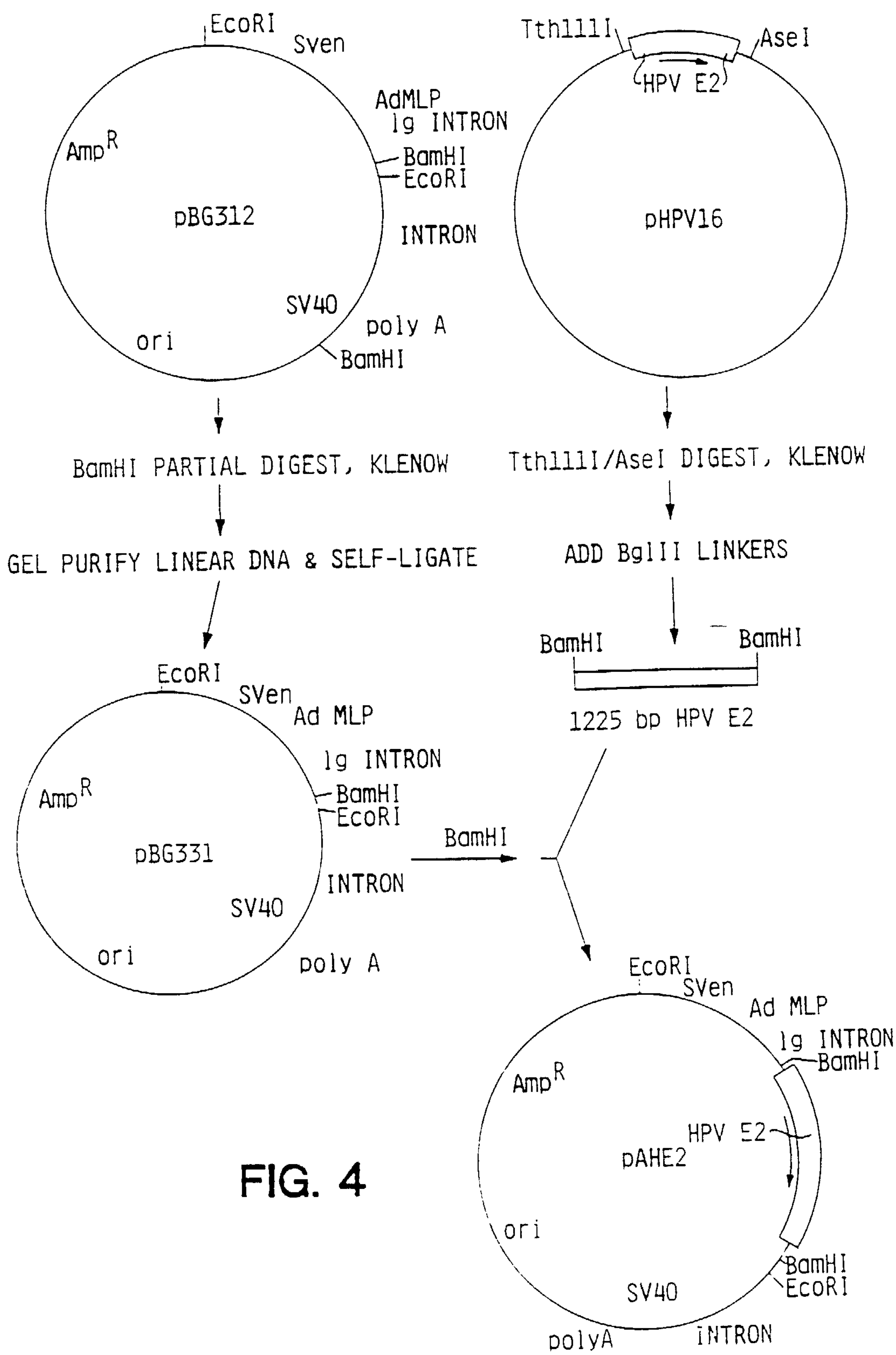
EcoRI
Sven
AdMLP
Ig INTRON
AmpR
BamHI
EcoRI
pBG312
INTRON
SV40
poly A
ori
BamHI
Tth111I
AseI
HPV E2
pHPV16
BamHI PARTIAL DIGEST, KLENOW
Tth111I/AseI DIGEST, KLENOW
GEL PURIFY LINEAR DNA & SELF-LIGATE
ADD BglII LINKERS
BamHI
BamHI
1225 bp HPV E2
EcoRI
SVen
Ad MLP
Ig INTRON
AmpR
BamHI
EcoRI
pBG331
BamHI
INTRON
SV40
ori
poly A
EcoRI
SVen
Ad MLP
Ig INTRON
BamHI
AmpR
HPV E2
pAHE2
ori
BamHI
EcoRI
SV40
polyA
INTRON

FIG. 4

FIG. 5

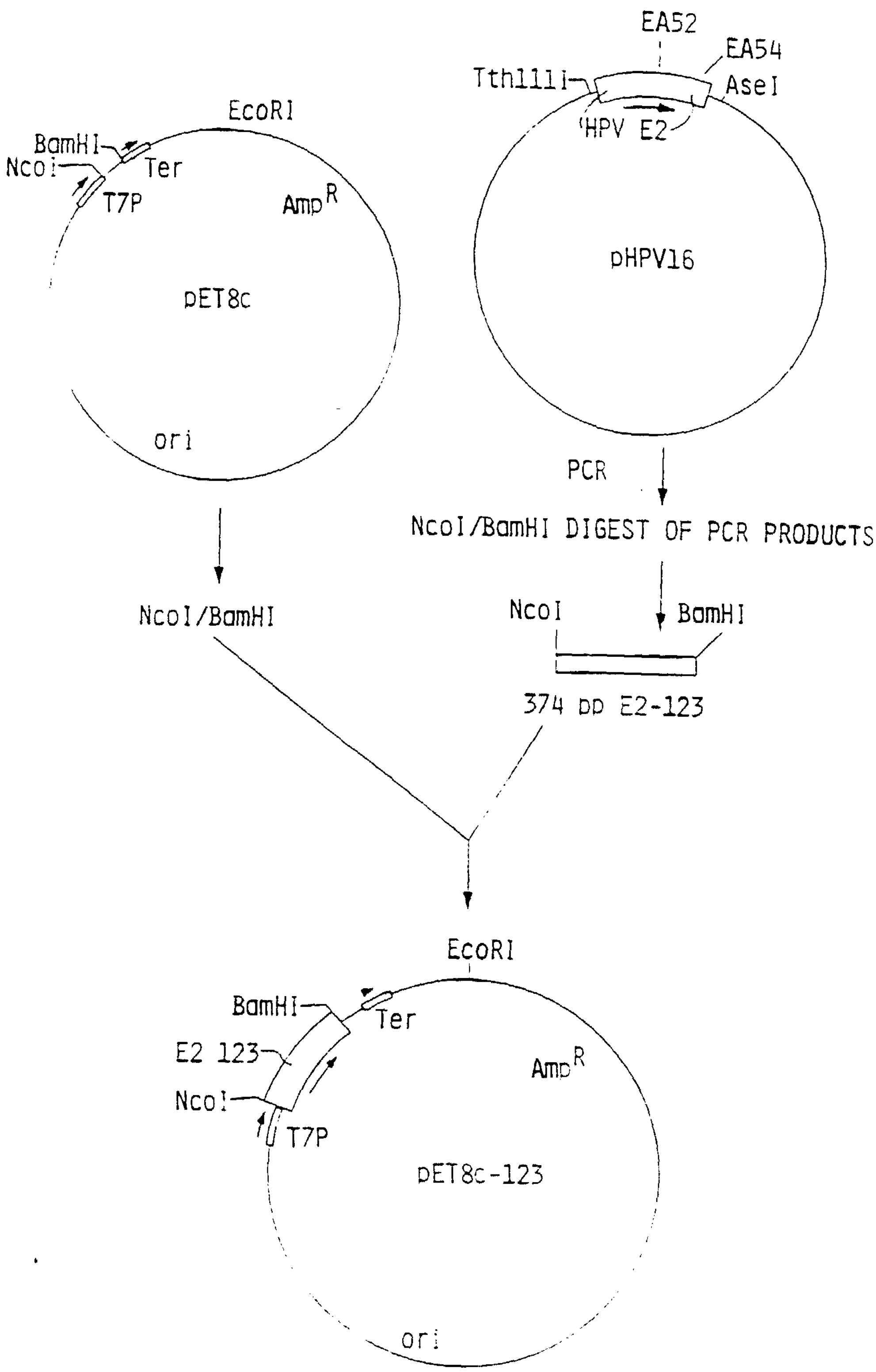
EA52
EA54
Tth111I
AseI
HPV E2
pHPV16
EcoRI
BamHI
NcoI
Ter
T7P
AmpR
pET8c
ori
PCR
NcoI/BamHI DIGEST OF PCR PRODUCTS
NcoI/BamHI
NcoI
BamHI
374 bp E2-123
EcoRI
BamHI
E2 123
NcoI
Ter
AmpR
T7P
pET8c-123
ori

FIG. 6

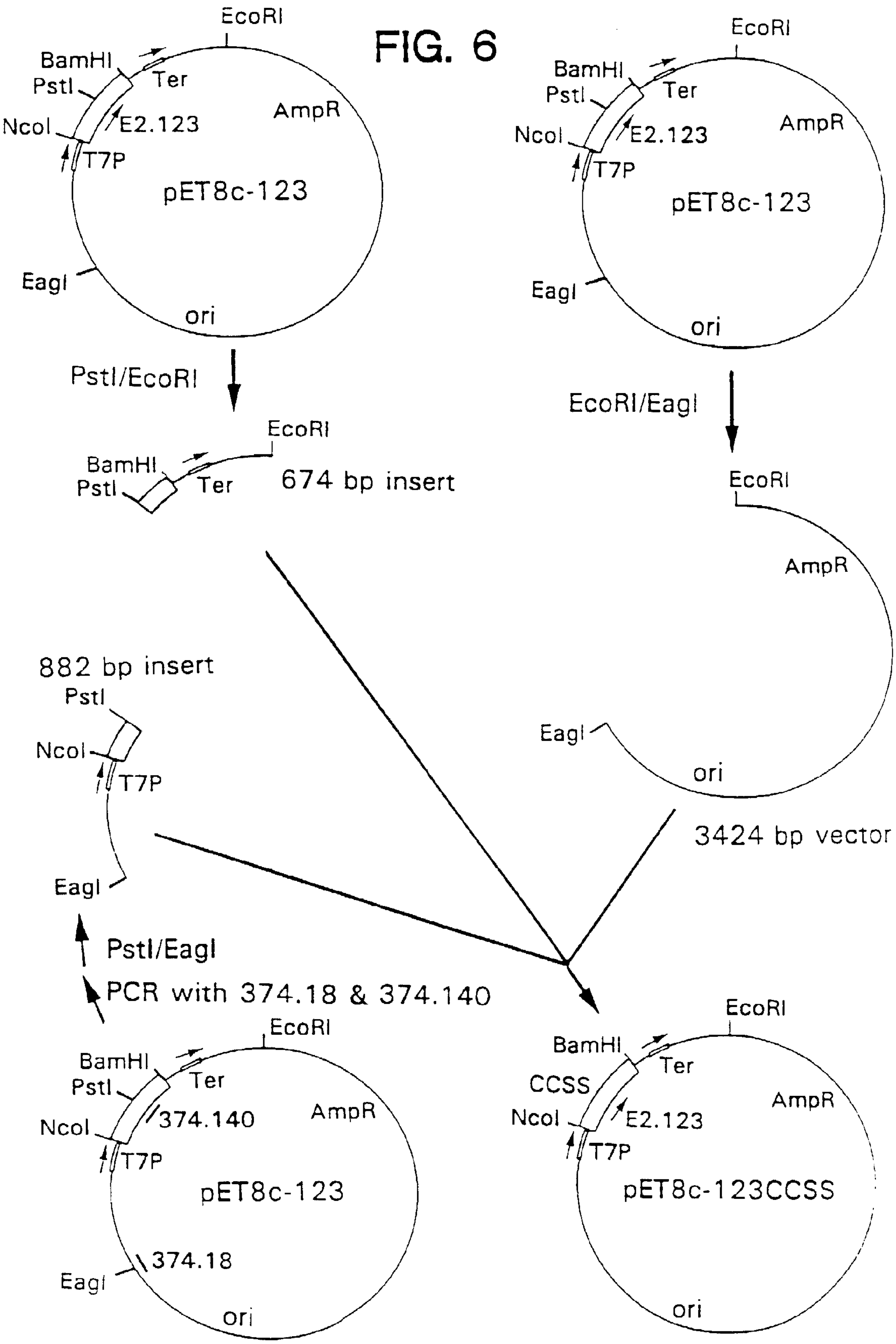
EcoRI
BamHI
PstI
Ter
NcoI
E2.123
AmpR
T7P
pET8c-123
EagI
ori
PstI/EcoRI
EcoRI
BamHI
PstI
Ter
674 bp insert
EcoRI
BamHI
PstI
Ter
NcoI
E2.123
AmpR
T7P
pET8c-123
EagI
ori
EcoRI/EagI
EcoRI
AmpR
EagI
ori
3424 bp vector
882 bp insert
PstI
NcoI
T7P
EagI
PstI/EagI
PCR with 374.18 & 374.140
EcoRI
BamHI
PstI
Ter
NcoI
374.140
AmpR
T7P
pET8c-123
EagI
374.18
ori
EcoRI
BamHI
CCSS
Ter
NcoI
E2.123
AmpR
T7P
pET8c-123CCSS
ori

FIG. 7

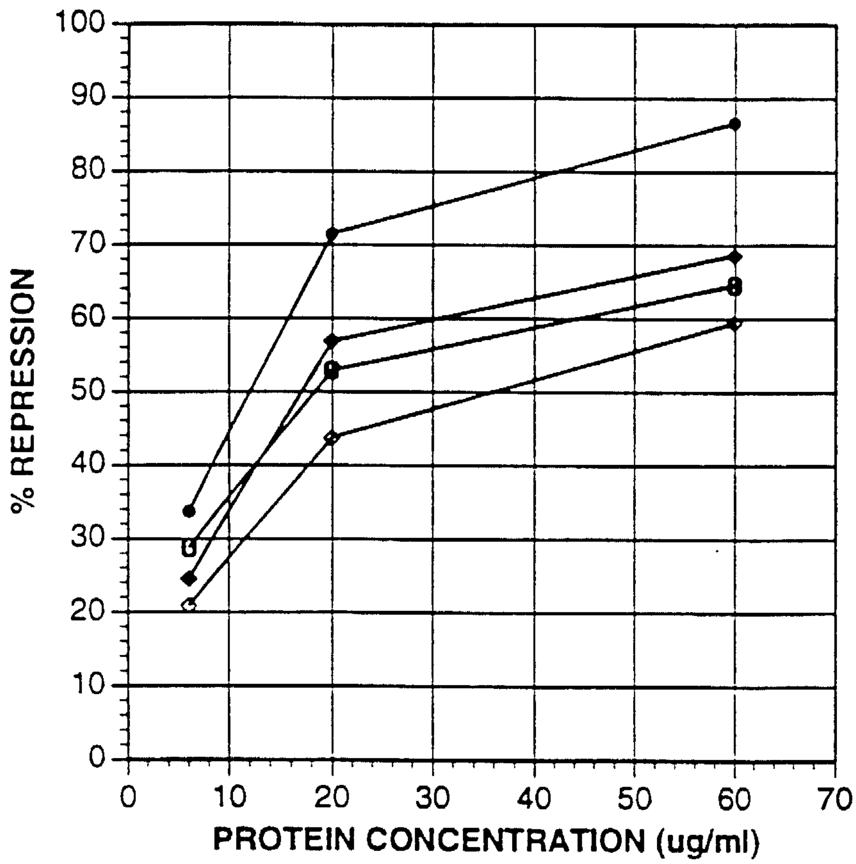
100
90
80
70
60
50
40
30
20
10
0
% REPRESSION
0
10
20
30
40
50
60
70
PROTEIN CONCENTRATION (ug/ml)

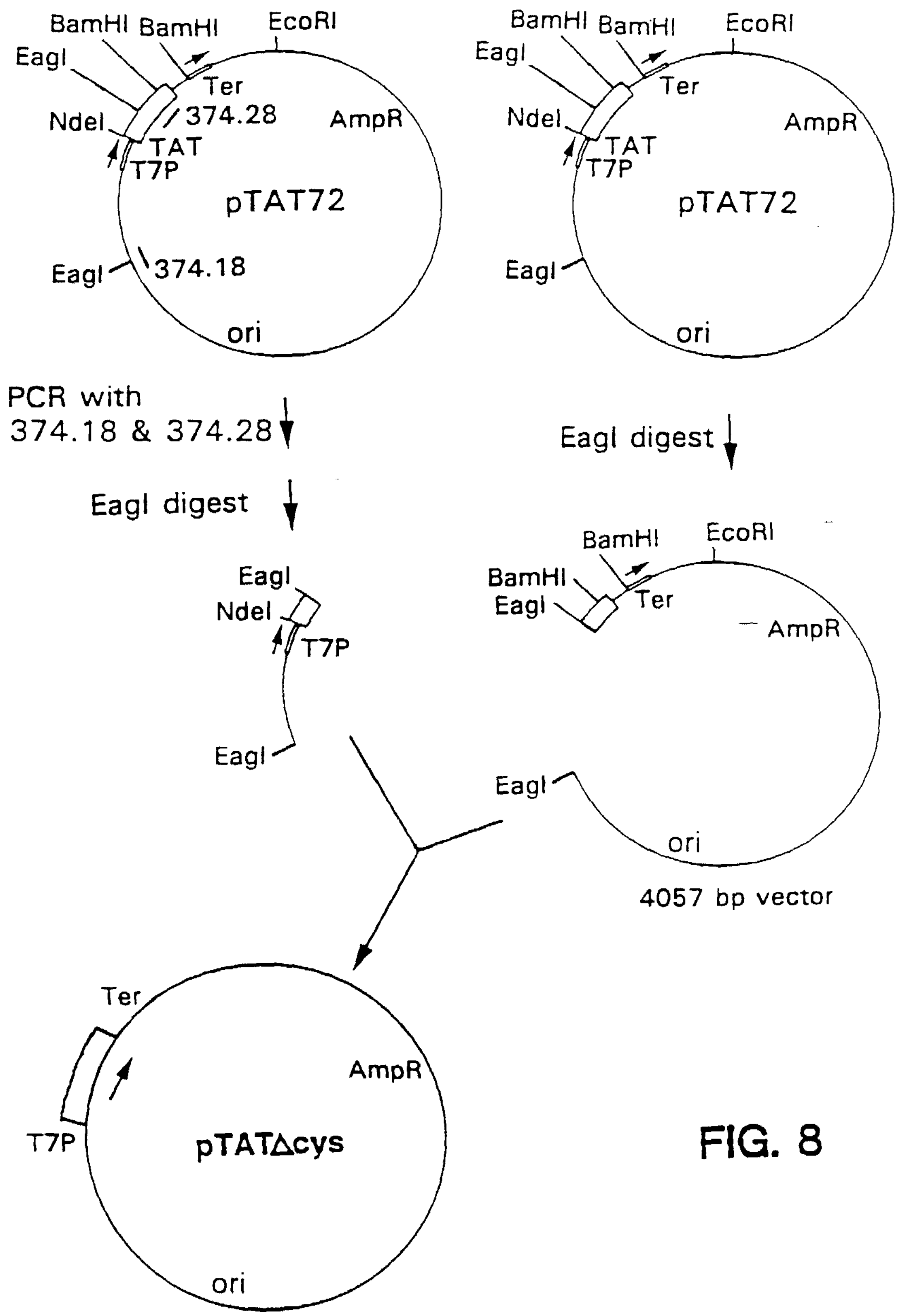
BamHI
BamHI
EcoRI
EagI
Ter
374.28
NdeI
AmpR
TAT
T7P
pTAT72
EagI
374.18
ori
BamHI
BamHI
EcoRI
EagI
Ter
NdeI
AmpR
TAT
T7P
pTAT72
EagI
ori
PCR with
374.18 & 374.28
EagI digest
EagI digest
EagI
NdeI
T7P
EagI
BamHI
EcoRI
BamHI
EagI
Ter
AmpR
EagI
ori
4057 bp vector
Ter
AmpR
T7P
pTATΔcys
ori

FIG. 8

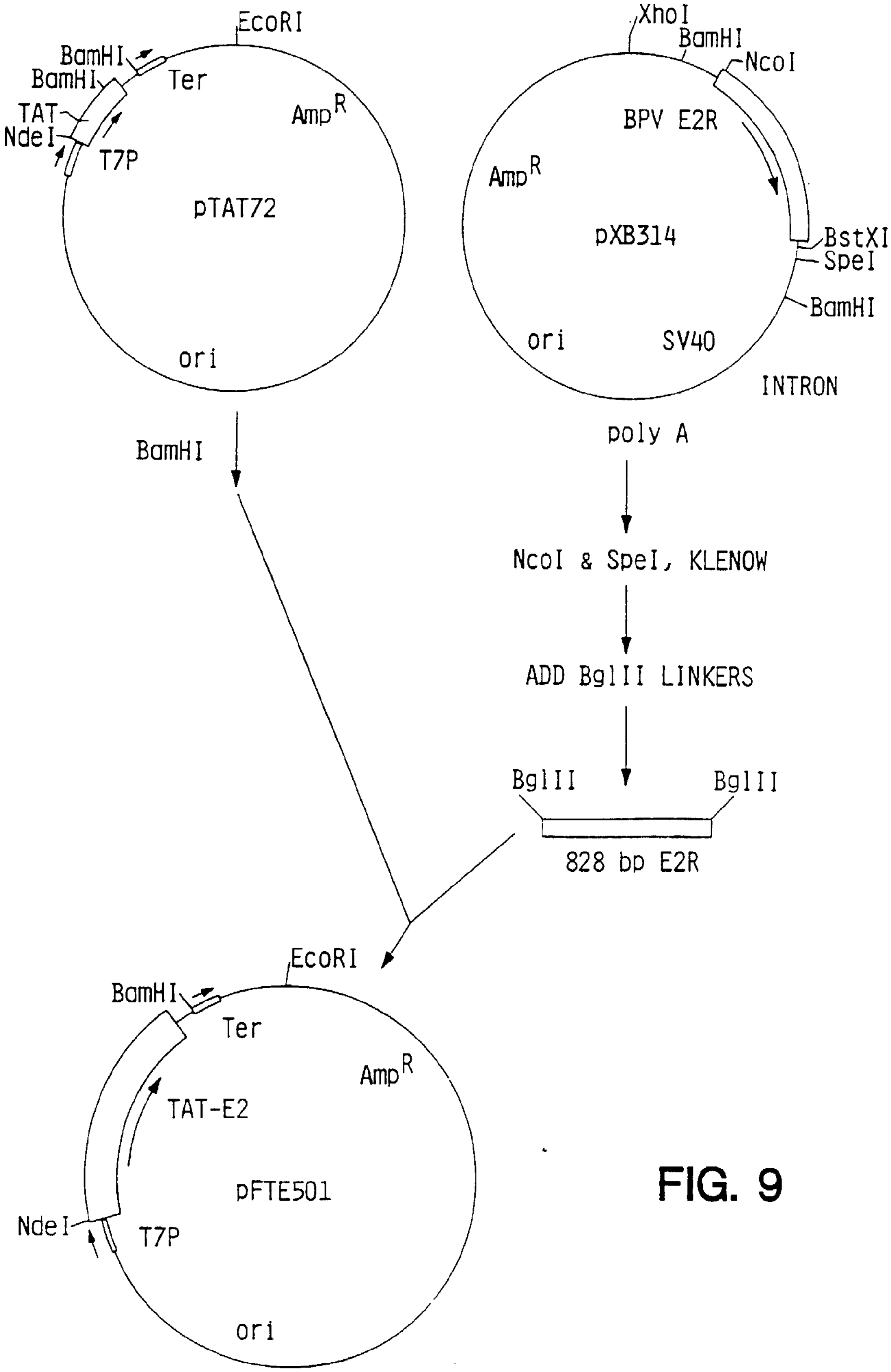
EcoRI
BamHI
BamHI
TAT
NdeI
Ter
T7P
AmpR
pTAT72
ori
BamHI
XhoI
BamHI
NcoI
BPV E2R
AmpR
pXB314
BstXI
SpeI
BamHI
ori
SV40
INTRON
poly A
NcoI & SpeI, KLENOW
ADD BglII LINKERS
BglII
BglII
828 bp E2R
EcoRI
BamHI
Ter
AmpR
TAT-E2
pFTE501
NdeI
T7P
ori

FIG. 9

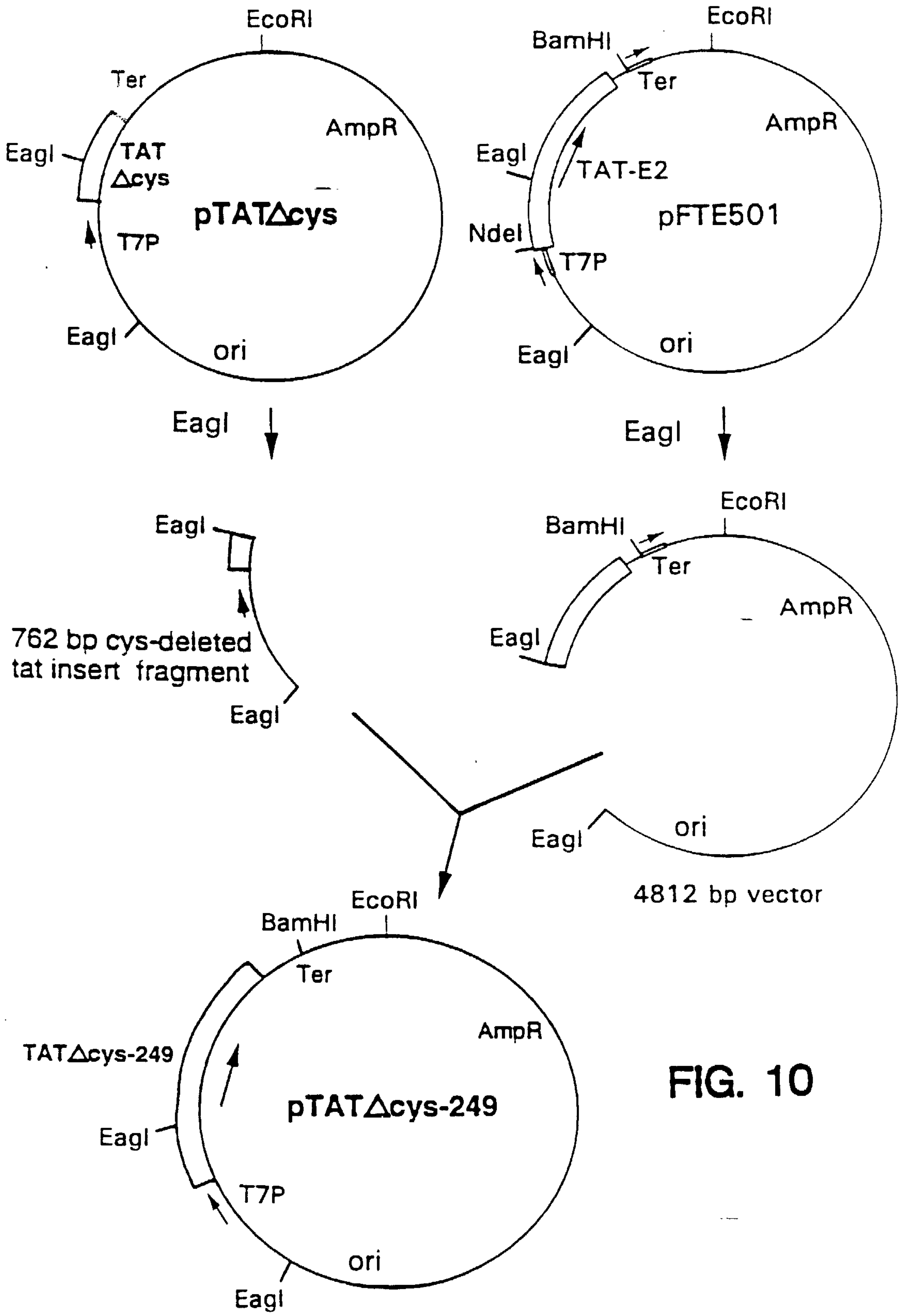
EcoRI
Ter
AmpR
EagI
TAT
Δcys
pTATΔcys
T7P
EagI
ori
BamHI
EcoRI
Ter
AmpR
EagI
TAT-E2
pFTE501
NdeI
T7P
EagI
ori
EagI
EagI
EagI
762 bp cys-deleted
tat insert fragment
EagI
BamHI
EcoRI
Ter
AmpR
EagI
EagI
ori
4812 bp vector
BamHI
EcoRI
Ter
AmpR
TATΔcys-249
pTATΔcys-249
EagI
T7P
ori
EagI

FIG. 10

FIG. 11

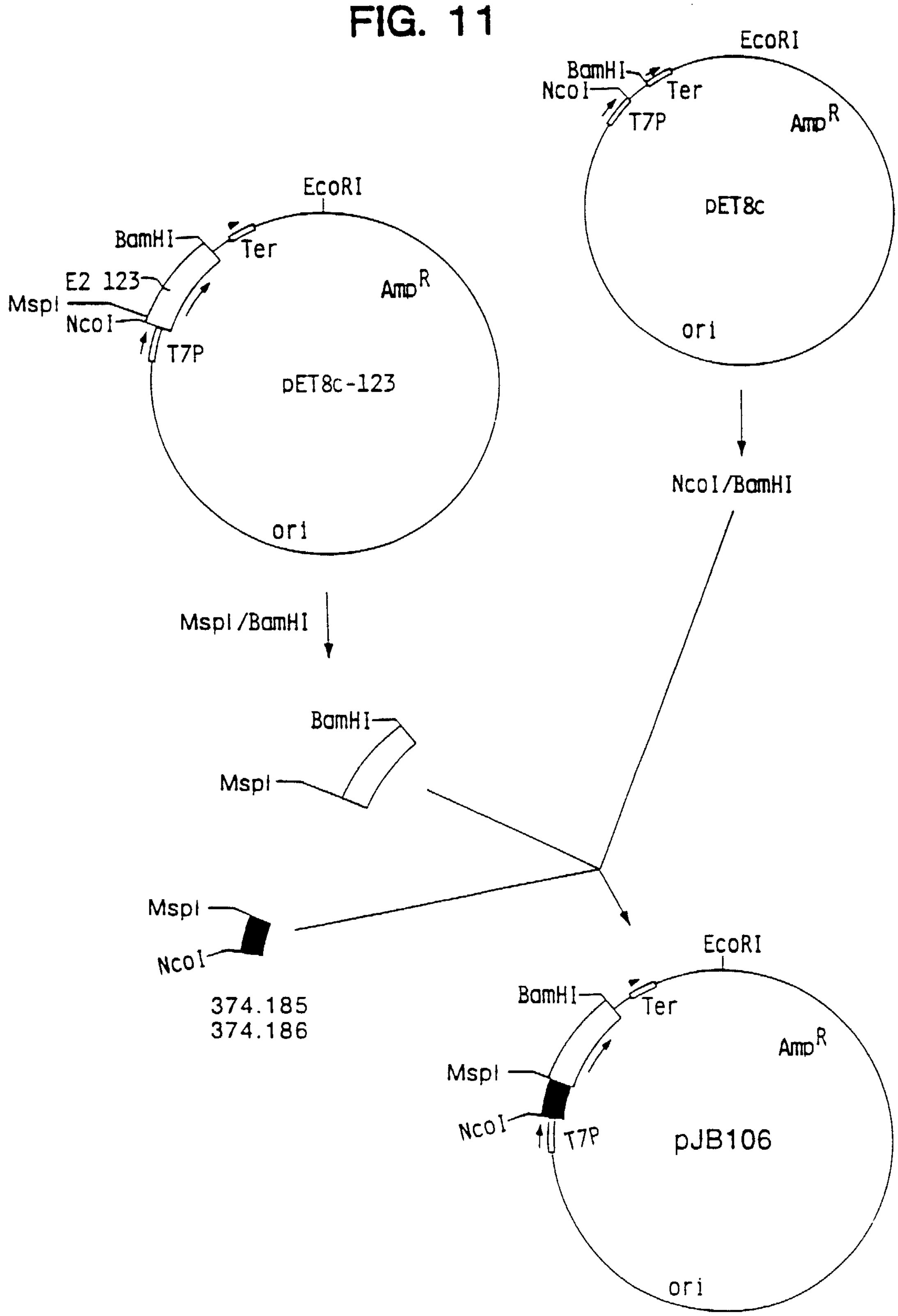
EcoRI
BamHI
Ncol
Ter
T7P
AmpR
pET8c
ori
NcoI/BamHI
EcoRI
BamHI
Ter
E2 123
Mspl
NcoI
AmpR
T7P
pET8c-123
ori
Mspl/BamHI
BamHI
Mspl
Mspl
NcoI
374.185
374.186
EcoRI
BamHI
Ter
AmpR
Mspl
NcoI
T7P
pJB106
ori

# FIG. 12

*MET* **TYR GLY ARG LYS LYS ARG ARG GLN ARG ARG**
**47**

**ARG PRO** PRO ASP THR GLY ASN PRO CYS HIS THR THR
**58** 245

LYS LEU LEU HIS ARG ASP SER VAL ASP SER ALA PRO
255

ILE LEU THR ALA PHE ASN SER SER HIS LYS GLY ARG
267

ILE ASN CYS ASN SER ASN THR THR PRO ILE VAL HIS
279

LEU LYS GLY ASP ALA ASN THR LEU LYS CYS LEU ARG
291

TYR ARG PHE LYS LYS HIS CYS THR LEU TYR THR ALA
303

VAL SER SER THR TRP HIS TRP THR GLY HIS ASN VAL
315

LYS HIS LYS SER ALA ILE VAL THR LEU THR TYR ASP
327

SER GLU TRP GLN ARG ASP GLN PHE LEU SER GLN VAL
339

LYS ILE PRO LYS THR ILE THR VAL SER THR GLY PHE
351

365
MET SER ILE

FIG. 13

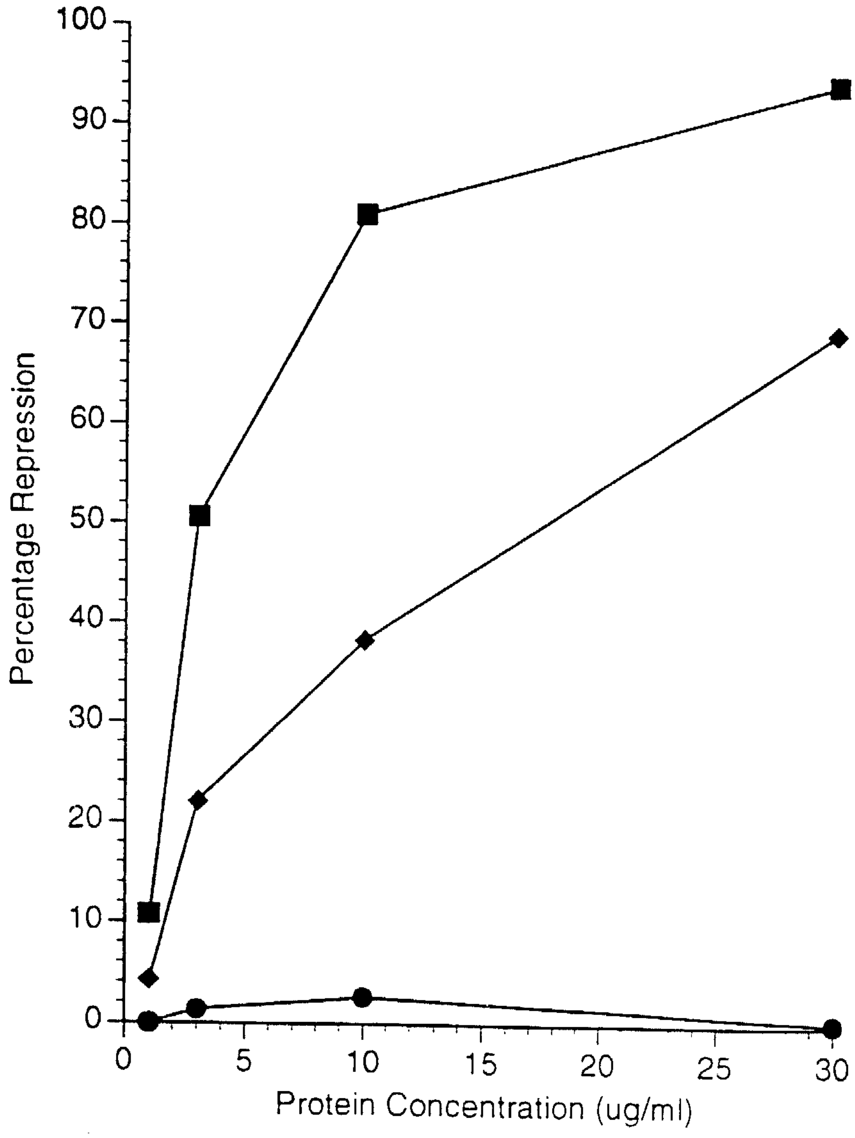
100
90
80
70
60
50
40
30
20
10
0
Percentage Repression
0
5
10
15
20
25
30
Protein Concentration (ug/ml)

SEQUENCE LISTING

(1) GENERAL INFORMATION:

(i) APPLICANT:

BIOGEN, INC.
BARSOUM, James G. (US only)
FAWELL, Stephen E. (US only)
PEPINSKY, R. B. (US only)

(ii) TITLE OF INVENTION: TAT-DERIVED TRANSPORT POLYPEPTIDES

(iii) NUMBER OF SEQUENCES: 63

(iv) CORRESPONDENCE ADDRESS:

(A) ADDRESSEE: FISH & NEAVE
(B) STREET: 1251 Avenue of the Americas
(C) CITY: New York
(D) STATE: New York
(E) COUNTRY: USA
(F) ZIP: 10020

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:

(A) APPLICATION NUMBER:
(B) FILING DATE:
(C) CLASSIFICATION:

(vii) PRIOR APPLICATION DATA:

(A) APPLICATION NUMBER: US 07/934,375
(B) FILING DATE: 21-AUG-1992

(viii) ATTORNEY/AGENT INFORMATION:

(A) NAME: Haley Jr., James F.
(B) REGISTRATION NUMBER: 27,794
(C) REFERENCE/DOCKET NUMBER: B170CIP

(ix) TELECOMMUNICATION INFORMATION:

(A) TELEPHONE: (212) 596-9000
(B) TELEFAX: (212) 596-9090
(C) TELEX: 14-8367

(2) INFORMATION FOR SEQ ID NO:1:

(i) SEQUENCE CHARACTERISTICS:

(A) LENGTH: 86 amino acids
(B) TYPE: amino acid
(D) TOPOLOGY: linear

(ii) MOLECULE TYPE: protein

(vi) ORIGINAL SOURCE:

(A) ORGANISM: human immunodeficiency virus
(B) STRAIN: type 1

(xi) SEQUENCE DESCRIPTION: SEQ ID NO:1:

```
Met Glu Pro Val Asp Pro Arg Leu Glu Pro Trp Lys His Pro Gly Ser
1               5                   10                  15

Gln Pro Lys Thr Ala Cys Thr Asn Cys Tyr Cys Lys Lys Cys Cys Phe
            20                  25                  30

His Cys Gln Val Cys Phe Ile Thr Lys Ala Leu Gly Ile Ser Tyr Gly
        35                  40                  45

Arg Lys Lys Arg Arg Gln Arg Arg Arg Pro Pro Gln Gly Ser Gln Thr
    50                  55                  60

His Gln Val Ser Leu Ser Lys Gln Pro Thr Ser Gln Ser Arg Gly Asp
65                  70                  75                  80

Pro Thr Gly Pro Lys Glu
                85
```

(2) INFORMATION FOR SEQ ID NO:2:

(i) SEQUENCE CHARACTERISTICS:

(A) LENGTH: 36 amino acids
(B) TYPE: amino acid
(D) TOPOLOGY: linear

(ii) MOLECULE TYPE: peptide

(xi) SEQUENCE DESCRIPTION: SEQ ID NO:2:

```
Cys Phe Ile Thr Lys Ala Leu Gly Ile Ser Tyr Gly Arg Lys Lys Arg
1               5                   10                  15

Arg Gln Arg Arg Arg Pro Pro Gln Gly Ser Gln Thr His Gln Val Ser
            20                  25                  30

Leu Ser Lys Gln
        35
```

(2) INFORMATION FOR SEQ ID NO:3:

(i) SEQUENCE CHARACTERISTICS:

(A) LENGTH: 22 amino acids
(B) TYPE: amino acid
(D) TOPOLOGY: linear

(ii) MOLECULE TYPE: peptide

(xi) SEQUENCE DESCRIPTION: SEQ ID NO:3:

```
Cys Phe Ile Thr Lys Ala Leu Gly Ile Ser Tyr Gly Arg Lys Lys Arg
1               5                   10                  15

Arg Gln Arg Arg Arg Pro
            20
```

(2) INFORMATION FOR SEQ ID NO:4:

(i) SEQUENCE CHARACTERISTICS:

(A) LENGTH: 24 amino acids
(B) TYPE: amino acid
(D) TOPOLOGY: linear

(ii) MOLECULE TYPE: peptide

(xi) SEQUENCE DESCRIPTION: SEQ ID NO:4:

```
Phe Ile Thr Lys Ala Leu Gly Ile Ser Tyr Gly Arg Lys Lys Arg Arg
1               5                   10                  15

Gln Arg Arg Arg Pro Gly Gly Cys
            20
```

(2) INFORMATION FOR SEQ ID NO:5:

(i) SEQUENCE CHARACTERISTICS:

(A) LENGTH: 15 amino acids
(B) TYPE: amino acid
(D) TOPOLOGY: linear

(ii) MOLECULE TYPE: peptide

(xi) SEQUENCE DESCRIPTION: SEQ ID NO:5:

```
Cys Gly Gly Tyr Gly Arg Lys Lys Arg Arg Gln Arg Arg Arg Pro
1               5                   10                  15
```

(2) INFORMATION FOR SEQ ID NO:6:

(i) SEQUENCE CHARACTERISTICS:

(A) LENGTH: 15 amino acids
(B) TYPE: amino acid
(D) TOPOLOGY: linear

(ii) MOLECULE TYPE: peptide

(xi) SEQUENCE DESCRIPTION: SEQ ID NO:6:

```
Tyr Gly Arg Lys Lys Arg Arg Gln Arg Arg Arg Pro Gly Gly Cys
1               5                   10                  15
```

(2) INFORMATION FOR SEQ ID NO:7:

(i) SEQUENCE CHARACTERISTICS:

(A) LENGTH: 56 amino acids
(B) TYPE: amino acid
(D) TOPOLOGY: linear

(ii) MOLECULE TYPE: peptide

(xi) SEQUENCE DESCRIPTION: SEQ ID NO:7:

```
Met Glu Pro Val Asp Pro Arg Leu Glu Pro Trp Lys His Pro Gly Ser
1               5                   10                  15

Gln Pro Lys Thr Ala Phe Ile Thr Lys Ala Leu Gly Ile Ser Tyr Gly
            20                  25                  30

Arg Lys Lys Arg Arg Gln Arg Arg Arg Pro Pro Gln Gly Ser Gln Thr
        35                  40                  45

His Gln Val Ser Leu Ser Lys Gln
    50                  55
```

(2) INFORMATION FOR SEQ ID NO:8:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:8:

```
GATCCCAGAC CCACCAGGTT TCTCTGTCGG GCCCTTAAG                              39
```

(2) INFORMATION FOR SEQ ID NO:9:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:9:

```
AATTCTTAAG GGCCCGACAG AGAAACCTGG TGGGTCTGG                              39
```

(2) INFORMATION FOR SEQ ID NO:10:

(i) SEQUENCE CHARACTERISTICS:

(A) LENGTH: 5098 base pairs
(B) TYPE: nucleic acid
(C) STRANDEDNESS: double
(D) TOPOLOGY: circular

(ii) MOLECULE TYPE: DNA (genomic)

(xi) SEQUENCE DESCRIPTION: SEQ ID NO:10:

TTGAAGACGA AAGGGCCTCG TGATACGCCT ATTTTTATAG GTTAATGTCA TGATAATAAT 60

GGTTTCTTAG ACGTCAGGTG GCACTTTTCG GGGAAATGTG CGCGGAACCC CTATTTGTTT 120

ATTTTTCTAA ATACATTCAA ATATGTATCC GCTCATGAGA CAATAACCCT GATAAATGCT 180

TCAATAATAT TGAAAAAGGA AGAGTATGAG TATTCAACAT TTCCGTGTCG CCCTTATTCC 240

CTTTTTTGCG GCATTTTGCC TTCCTGTTTT TGCTCACCCA GAAACGCTGG TGAAAGTAAA 300

AGATGCTGAA GATCAGTTGG GTGCACGAGT GGGTTACATC GAACTGGATC TCAACAGCGG 360

TAAGATCCTT GAGAGTTTTC GCCCCGAAGA ACGTTTTCCA ATGATGAGCA CTTTTAAAGT 420

TCTGCTATGT GGCGCGGTAT TATCCCGTGT TGACGCCGGG CAAGAGCAAC TCGGTCGCCG 480

CATACACTAT TCTCAGAATG ACTTGGTTGA GTACTCACCA GTCACAGAAA AGCATCTTAC 540

GGATGGCATG ACAGTAAGAG AATTATGCAG TGCTGCCATA ACCATGAGTG ATAACACTGC 600

GGCCAACTTA CTTCTGACAA CGATCGGAGG ACCGAAGGAG CTAACCGCTT TTTTGCACAA 660

CATGGGGGAT CATGTAACTC GCCTTGATCG TTGGGAACCG GAGCTGAATG AAGCCATACC 720

AAACGACGAG CGTGACACCA CGATGCCTGC AGCAATGGCA ACAACGTTGC GCAAACTATT 780

AACTGGCGAA CTACTTACTC TAGCTTCCCG GCAACAATTA ATAGACTGGA TGGAGGCGGA 840

TAAAGTTGCA GGACCACTTC TGCGCTCGGC CCTTCCGGCT GGCTGGTTTA TTGCTGATAA 900

ATCTGGAGCC GGTGAGCGTG GGTCTCGCGG TATCATTGCA GCACTGGGGC CAGATGGTAA 960

GCCCTCCCGT ATCGTAGTTA TCTACACGAC GGGGAGTCAG GCAACTATGG ATGAACGAAA 1020

TAGACAGATC GCTGAGATAG GTGCCTCACT GATTAAGCAT TGGTAACTGT CAGACCAAGT 1080

TTACTCATAT ATACTTTAGA TTGATTTAAA ACTTCATTTT TAATTTAAAA GGATCTAGGT 1140

GAAGATCCTT TTTGATAATC TCATGACCAA AATCCCTTAA CGTGAGTTTT CGTTCCACTG 1200

AGCGTCAGAC CCCGTAGAAA AGATCAAAGG ATCTTCTTGA GATCCTTTTT TTCTGCGCGT 1260

AATCTGCTGC TTGCAAACAA AAAAACCACC GCTACCAGCG GTGGTTTGTT TGCCGGATCA 1320

AGAGCTACCA ACTCTTTTTC CGAAGGTAAC TGGCTTCAGC AGAGCGCAGA TACCAAATAC 1380

TGTCCTTCTA GTGTAGCCGT AGTTAGGCCA CCACTTCAAG AACTCTGTAG CACCGCCTAC 1440

ATACCTCGCT CTGCTAATCC TGTTACCAGT GGCTGCTGCC AGTGGCGATA AGTCGTGTCT 1500

TACCGGGTTG GACTCAAGAC GATAGTTACC GGATAAGGCG CAGCGGTCGG GCTGAACGGG 1560

GGGTTCGTGC ACACAGCCCA GCTTGGAGCG AACGACCTAC ACCGAACTGA GATACCTACA 1620

GCGTGAGCAT TGAGAAAGCG CCACGCTTCC CGAAGGGAGA AAGGCGGACA GGTATCCGGT 1680

AAGCGGCAGG GTCGGAACAG GAGAGCGCAC GAGGGAGCTT CCAGGGGGAA ACGCCTGGTA 1740

TCTTTATAGT CCTGTCGGGT TTCGCCACCT CTGACTTGAG CGTCGATTTT TGTGATGCTC 1800

GTCAGGGGGG CGGAGCCTAT GGAAAAACGC CAGCAACGCG GCCTTTTTAC GGTTCCTGGC 1860

CTTTTGCTGG CCTTTTGCTC ACATGTTCTT TCCTGCGTTA TCCCCTGATT CTGTGGATAA 1920

CCGTATTACC GCCTTTGAGT GAGCTGATAC CGCTCGCCGC AGCCGAACGA CCGAGCGCAG 1980

CGAGTCAGTG AGCGAGGAAG CGGAAGAGCG CCTGATGCGG TATTTTCTCC TTACGCATCT 2040

GTGCGGTATT TCACACCGCA TATATGGTGC ACTCTCAGTA CAATCTGCTC TGATGCCGCA 2100

TAGTTAAGCC AGTATACACT CCGCTATCGC TACGTGACTG GGTCATGGCT GCGCCCCGAC 2160

ACCCGCCAAC ACCCGCTGAC GCGCCCTGAC GGGCTTGTCT GCTCCCGGCA TCCGCTTACA 2220

GACAAGCTGT GACCGTCTCC GGGAGCTGCA TGTGTCAGAG GTTTTCACCG TCATCACCGA 2280

AACGCGCGAG GCAGCTGCGG TAAAGCTCAT CAGCGTGGTC GTGAAGCGAT TCACAGATGT 2340

CTGCCTGTTC ATCCGCGTCC AGCTCGTTGA GTTTCTCCAG AAGCGTTAAT GTCTGGCTTC 2400

TGATAAAGCG GGCCATGTTA AGGGCGGTTT TTTCCTGTTT GGTCACTTGA TGCCTCCGTG 2460
TAAGGGGGAA TTTCTGTTCA TGGGGGTAAT GATACCGATG AAACGAGAGA GGATGCTCAC 2520
GATACGGGTT ACTGATGATG AACATGCCCG GTTACTGGAA CGTTGTGAGG GTAAACAACT 2580
GGCGGTATGG ATGCGGCGGG ACCAGAGAAA AATCACTCAG GGTCAATGCC AGCGCTTCGT 2640
TAATACAGAT GTAGGTGTTC CACAGGGTAG CCAGCAGCAT CCTGCGATGC AGATCCGGAA 2700
CATAATGGTG CAGGGCGCTG ACTTCCGCGT TTCCAGACTT TACGAAACAC GGAAACCGAA 2760
GACCATTCAT GTTGTTGCTC AGGTCGCAGA CGTTTTGCAG CAGCAGTCGC TTCACGTTCG 2820
CTCGCGTATC GGTGATTCAT TCTGCTAACC AGTAAGGCAA CCCCGCCAGC CTAGCCGGGT 2880
CCTCAACGAC AGGAGCACGA TCATGCGCAC CCGTGGCCAG GACCCAACGC TGCCCGAGAT 2940
GCGCCGCGTG CGGCTGCTGG AGATGGCGGA CGCGATGGAT ATGTTCTGCC AAGGGTTGGT 3000
TTGCGCATTC ACAGTTCTCC GCAAGAATTG ATTGGCTCCA ATTCTTGGAG TGGTGAATCC 3060
GTTAGCGAGG TGCCGCCGGC TTCCATTCAG GTCGAGGTGG CCCGGCTCCA TGCACCGCGA 3120
CGCAACGCGG GGAGGCAGAC AAGGTATAGG GCGGCGCCTA CAATCCATGC CAACCCGTTC 3180
CATGTGCTCG CCGAGGCGGC ATAAATCGCC GTGACGATCA GCGGTCCAGT GATCGAAGTT 3240
AGGCTGGTAA GAGCCGCGAG CGATCCTTGA AGCTGTCCCT GATGGTCGTC ATCTACCTGC 3300
CTGGACAGCA TGGCCTGCAA CGCGGGCATC CCGATGCCGC CGGAAGCGAG AAGAATCATA 3360
ATGGGGAAGG CCATCCAGCC TCGCGTCGCG AACGCCAGCA AGACGTAGCC CAGCGCGTCG 3420
GCCGCCATGC CGGCGATAAT GGCCTGCTTC TCGCCGAAAC GTTTGGTGGC GGGACCAGTG 3480
ACGAAGGCTT GAGCGAGGGC GTGCAAGATT CCGAATACCG CAAGCGACAG GCCGATCATC 3540
GTCGCGCTCC AGCGAAAGCG GTCCTCGCCG AAAATGACCC AGAGCGCTGC CGGCACCTGT 3600
CCTACGAGTT GCATGATAAA GAAGACAGTC ATAAGTGCGG CGACGATAGT CATGCCCCGC 3660
GCCCACCGGA AGGAGCTGAC TGGGTTGAAG GCTCTCAAGG GCATCGGTCG ACGCTCTCCC 3720
TTATGCGACT CCTGCATTAG GAAGCAGCCC AGTAGTAGGT TGAGGCCGTT GAGCACCGCC 3780
GCCGCAAGGA ATGGTGCATG CAAGGAGATG GCGCCCAACA GTCCCCCGGC CACGGGGCCT 3840
GCCACCATAC CCACGCCGAA ACAAGCGCTC ATGAGCCCGA AGTGGCGAGC CCGATCTTCC 3900
CCATCGGTGA TGTCGGCGAT ATAGGCGCCA GCAACCGCAC CTGTGGCGCC GGTGATGCCG 3960
GCCACGATGC GTCCGGCGTA GAGGATCGAG ATCTCGATCC CGCGAAATTA ATACGACTCA 4020
CTATAGGGAG ACCACAACGG TTTCCCTCTA GAAATAATTT TGTTTAACTT TAAGAAGGAG 4080

```
ATATACATAT GGAACCGGTC GACCCGCGTC TGGAACCATG GAAACACCCC GGGTCCCAGC    4140
CGAAAACCGC GTGCACCAAC TGCTACTGCA AAAAATGCTG CTTCCACTGC CAGGTTTGCT    4200
TCATCACCAA AGCCCTAGGT ATCTCTTACG GCCGTAAAAA ACGTCGTCAG CGACGTCGTC    4260
CGCCGCAGGG ATCCCAGACC CACCAGGTTT CTCTGTCGGG CCCGGCGGAC AGCGGCGACG    4320
CCCTGCTGGA GCGCAACTAT CCCACTGGCG CGGAGTTCCT CGGCGACGGC GGCGACGTCA    4380
GCTTCAGCAC CCGCGGCACG CAGAACTGGA CGGTGGAGCG GCTGCTCCAG GCGCACCGCC    4440
AACTGGAGGA GCGCGGCTAT GTGTTCGTCG GCTACCACGG CACCTTCCTC GAAGCGGCGC    4500
AAAGCATCGT CTTCGGCGGG GTGCGCGCGC GCAGCCAGGA CCTCGACGCG ATCTGGCGCG    4560
GTTTCTATAT CGCCGGCGAT CCGGCGCTGG CCTACGGCTA CGCCCAGGAC CAGGAACCCG    4620
ACGCACGCGG CCGGATCCGC AACGGTGCCC TGCTGCGGGT CTATGTGCCG CGCTCGAGCC    4680
TGCCGGGCTT CTACCGCACC AGCCTGACCC TGGCCGCGCC GGAGGCGGCG GGCGAGGTCG    4740
AACGGCTGAT CGGCCATCCG CTGCCGCTGC GCCTGGACGC CATCACCGGC CCCGAGGAGG    4800
AAGGCGGGCG CCTGGAGACC ATTCTCGGCT GGCCGCTGGC CGAGCGCACC GTGGTGATTC    4860
CCTCGGCGAT CCCCACCGAC CCGCGCAACG TCGGCGGCGA CCTCGACCCG TCCAGCATCC    4920
CCGACAAGGA ACAGGCGATC AGCGCCCTGC CGGACTACGC CAGCCAGCCC GGCAAACCGC    4980
CGCGCGAGGA CCTGAAGTAA CTGCCGCGAC CGGCCGGCTC CCTTCGCAGG AGCCGGCCTT    5040
CTCGGGGCCT GGCCATACAT CAGGTTTTCC TGATGCCAGC CCAATCGAAT ATGAATTC      5098
```

(2) INFORMATION FOR SEQ ID NO:11:

(i) SEQUENCE CHARACTERISTICS:

(A) LENGTH: 4910 base pairs
(B) TYPE: nucleic acid
(C) STRANDEDNESS: double
(D) TOPOLOGY: circular

(ii) MOLECULE TYPE: DNA (genomic)

(xi) SEQUENCE DESCRIPTION: SEQ ID NO:11:

```
TTGAAGACGA AAGGGCCTCG TGATACGCCT ATTTTTATAG GTTAATGTCA TGATAATAAT      60
GGTTTCTTAG ACGTCAGGTG GCACTTTTCG GGGAAATGTG CGCGGAACCC CTATTTGTTT     120
ATTTTTCTAA ATACATTCAA ATATGTATCC GCTCATGAGA CAATAACCCT GATAAATGCT     180
TCAATAATAT TGAAAAAGGA AGAGTATGAG TATTCAACAT TTCCGTGTCG CCCTTATTCC     240
```

CTTTTTTGCG GCATTTTGCC TTCCTGTTTT TGCTCACCCA GAAACGCTGG TGAAAGTAAA 300

AGATGCTGAA GATCAGTTGG GTGCACGAGT GGGTTACATC GAACTGGATC TCAACAGCGG 360

TAAGATCCTT GAGAGTTTTC GCCCCGAAGA ACGTTTTCCA ATGATGAGCA CTTTTAAAGT 420

TCTGCTATGT GGCGCGGTAT TATCCCGTGT TGACGCCGGG CAAGAGCAAC TCGGTCGCCG 480

CATACACTAT TCTCAGAATG ACTTGGTTGA GTACTCACCA GTCACAGAAA AGCATCTTAC 540

GGATGGCATG ACAGTAAGAG AATTATGCAG TGCTGCCATA ACCATGAGTG ATAACACTGC 600

GGCCAACTTA CTTCTGACAA CGATCGGAGG ACCGAAGGAG CTAACCGCTT TTTTGCACAA 660

CATGGGGGAT CATGTAACTC GCCTTGATCG TTGGGAACCG GAGCTGAATG AAGCCATACC 720

AAACGACGAG CGTGACACCA CGATGCCTGC AGCAATGGCA ACAACGTTGC GCAAACTATT 780

AACTGGCGAA CTACTTACTC TAGCTTCCCG GCAACAATTA ATAGACTGGA TGGAGGCGGA 840

TAAAGTTGCA GGACCACTTC TGCGCTCGGC CCTTCCGGCT GGCTGGTTTA TTGCTGATAA 900

ATCTGGAGCC GGTGAGCGTG GGTCTCGCGG TATCATTGCA GCACTGGGGC CAGATGGTAA 960

GCCCTCCCGT ATCGTAGTTA TCTACACGAC GGGGAGTCAG GCAACTATGG ATGAACGAAA 1020

TAGACAGATC GCTGAGATAG GTGCCTCACT GATTAAGCAT TGGTAACTGT CAGACCAAGT 1080

TTACTCATAT ATACTTTAGA TTGATTTAAA ACTTCATTTT TAATTTAAAA GGATCTAGGT 1140

GAAGATCCTT TTTGATAATC TCATGACCAA AATCCCTTAA CGTGAGTTTT CGTTCCACTG 1200

AGCGTCAGAC CCCGTAGAAA AGATCAAAGG ATCTTCTTGA GATCCTTTTT TTCTGCGCGT 1260

AATCTGCTGC TTGCAAACAA AAAAACCACC GCTACCAGCG GTGGTTTGTT TGCCGGATCA 1320

AGAGCTACCA ACTCTTTTTC CGAAGGTAAC TGGCTTCAGC AGAGCGCAGA TACCAAATAC 1380

TGTCCTTCTA GTGTAGCCGT AGTTAGGCCA CCACTTCAAG AACTCTGTAG CACCGCCTAC 1440

ATACCTCGCT CTGCTAATCC TGTTACCAGT GGCTGCTGCC AGTGGCGATA AGTCGTGTCT 1500

TACCGGGTTG GACTCAAGAC GATAGTTACC GGATAAGGCG CAGCGGTCGG GCTGAACGGG 1560

GGGTTCGTGC ACACAGCCCA GCTTGGAGCG AACGACCTAC ACCGAACTGA GATACCTACA 1620

GCGTGAGCAT TGAGAAAGCG CCACGCTTCC CGAAGGGAGA AAGGCGGACA GGTATCCGGT 1680

AAGCGGCAGG GTCGGAACAG GAGAGCGCAC GAGGGAGCTT CCAGGGGGAA ACGCCTGGTA 1740

TCTTTATAGT CCTGTCGGGT TTCGCCACCT CTGACTTGAG CGTCGATTTT TGTGATGCTC 1800

GTCAGGGGGG CGGAGCCTAT GGAAAAACGC CAGCAACGCG GCCTTTTTAC GGTTCCTGGC 1860

CTTTTGCTGG CCTTTTGCTC ACATGTTCTT TCCTGCGTTA TCCCCTGATT CTGTGGATAA 1920

CCGTATTACC GCCTTTGAGT GAGCTGATAC CGCTCGCCGC AGCCGAACGA CCGAGCGCAG 1980

CGAGTCAGTG AGCGAGGAAG CGGAAGAGCG CCTGATGCGG TATTTTCTCC TTACGCATCT 2040

GTGCGGTATT TCACACCGCA TATATGGTGC ACTCTCAGTA CAATCTGCTC TGATGCCGCA 2100

TAGTTAAGCC AGTATACACT CCGCTATCGC TACGTGACTG GGTCATGGCT GCGCCCCGAC 2160

ACCCGCCAAC ACCCGCTGAC GCGCCCTGAC GGGCTTGTCT GCTCCCGGCA TCCGCTTACA 2220

GACAAGCTGT GACCGTCTCC GGGAGCTGCA TGTGTCAGAG GTTTTCACCG TCATCACCGA 2280

AACGCGCGAG GCAGCTGCGG TAAAGCTCAT CAGCGTGGTC GTGAAGCGAT TCACAGATGT 2340

CTGCCTGTTC ATCCGCGTCC AGCTCGTTGA GTTTCTCCAG AAGCGTTAAT GTCTGGCTTC 2400

TGATAAAGCG GGCCATGTTA AGGGCGGTTT TTTCCTGTTT GGTCACTTGA TGCCTCCGTG 2460

TAAGGGGGAA TTTCTGTTCA TGGGGGTAAT GATACCGATG AAACGAGAGA GGATGCTCAC 2520

GATACGGGTT ACTGATGATG AACATGCCCG GTTACTGGAA CGTTGTGAGG GTAAACAACT 2580

GGCGGTATGG ATGCGGCGGG ACCAGAGAAA AATCACTCAG GGTCAATGCC AGCGCTTCGT 2640

TAATACAGAT GTAGGTGTTC CACAGGGTAG CCAGCAGCAT CCTGCGATGC AGATCCGGAA 2700

CATAATGGTG CAGGGCGCTG ACTTCCGCGT TTCCAGACTT TACGAAACAC GGAAACCGAA 2760

GACCATTCAT GTTGTTGCTC AGGTCGCAGA CGTTTTGCAG CAGCAGTCGC TTCACGTTCG 2820

CTCGCGTATC GGTGATTCAT TCTGCTAACC AGTAAGGCAA CCCCGCCAGC CTAGCCGGGT 2880

CCTCAACGAC AGGAGCACGA TCATGCGCAC CCGTGGCCAG GACCCAACGC TGCCCGAGAT 2940

GCGCCGCGTG CGGCTGCTGG AGATGGCGGA CGCGATGGAT ATGTTCTGCC AAGGGTTGGT 3000

TTGCGCATTC ACAGTTCTCC GCAAGAATTG ATTGGCTCCA ATTCTTGGAG TGGTGAATCC 3060

GTTAGCGAGG TGCCGCCGGC TTCCATTCAG GTCGAGGTGG CCCGGCTCCA TGCACCGCGA 3120

CGCAACGCGG GGAGGCAGAC AAGGTATAGG GCGGCGCCTA CAATCCATGC CAACCCGTTC 3180

CATGTGCTCG CCGAGGCGGC ATAAATCGCC GTGACGATCA GCGGTCCAGT GATCGAAGTT 3240

AGGCTGGTAA GAGCCGCGAG CGATCCTTGA AGCTGTCCCT GATGGTCGTC ATCTACCTGC 3300

CTGGACAGCA TGGCCTGCAA CGCGGGCATC CCGATGCCGC CGGAAGCGAG AAGAATCATA 3360

ATGGGGAAGG CCATCCAGCC TCGCGTCGCG AACGCCAGCA AGACGTAGCC CAGCGCGTCG 3420

GCCGCCATGC CGGCGATAAT GGCCTGCTTC TCGCCGAAAC GTTTGGTGGC GGGACCAGTG 3480

ACGAAGGCTT GAGCGAGGGC GTGCAAGATT CCGAATACCG CAAGCGACAG GCCGATCATC 3540

GTCGCGCTCC AGCGAAAGCG GTCCTCGCCG AAAATGACCC AGAGCGCTGC CGGCACCTGT 3600

```
CCTACGAGTT GCATGATAAA GAAGACAGTC ATAAGTGCGG CGACGATAGT CATGCCCCGC 3660
GCCCACCGGA AGGAGCTGAC TGGGTTGAAG GCTCTCAAGG GCATCGGTCG ACGCTCTCCC 3720
TTATGCGACT CCTGCATTAG GAAGCAGCCC AGTAGTAGGT TGAGGCCGTT GAGCACCGCC 3780
GCCGCAAGGA ATGGTGCATG CAAGGAGATG GCGCCCAACA GTCCCCCGGC CACGGGGCCT 3840
GCCACCATAC CCACGCCGAA ACAAGCGCTC ATGAGCCCGA AGTGGCGAGC CCGATCTTCC 3900
CCATCGGTGA TGTCGGCGAT ATAGGCGCCA GCAACCGCAC CTGTGGCGCC GGTGATGCCG 3960
GCCACGATGC GTCCGGCGTA GAGGATCGAG ATCTCGATCC CGCGAAATTA ATACGACTCA 4020
CTATAGGGAG ACCACAACGG TTTCCCTCTA GAAATAATTT TGTTTAACTT TAAGAAGGAG 4080
ATATATATGG AACCGGTCGT TTCTCTGTCG GGCCCGGCGG ACAGCGGCGA CGCCCTGCTG 4140
GAGCGCAACT ATCCCACTGG CGCGGAGTTC CTCGGCGACG GCGGCGACGT CAGCTTCAGC 4200
ACCCGCGGCA CGCAGAACTG GACGGTGGAG CGGCTGCTCC AGGCGCACCG CCAACTGGAG 4260
GAGCGCGGCT ATGTGTTCGT CGGCTACCAC GGCACCTTCC TCGAAGCGGC GCAAAGCATC 4320
GTCTTCGGCG GGGTGCGCGC GCGCAGCCAG GACCTCGACG CGATCTGGCG CGGTTTCTAT 4380
ATCGCCGGCG ATCCGGCGCT GGCCTACGGC TACGCCCAGG ACCAGGAACC CGACGCACGC 4440
GGCCGGATCC GCAACGGTGC CCTGCTGCGG GTCTATGTGC CGCGCTCGAG CCTGCCGGGC 4500
TTCTACCGCA CCAGCCTGAC CCTGGCCGCG CCGGAGGCGG CGGGCGAGGT CGAACGGCTG 4560
ATCGGCCATC CGCTGCCGCT GCGCCTGGAC GCCATCACCG GCCCCGAGGA GGAAGGCGGG 4620
CGCCTGGAGA CCATTCTCGG CTGGCCGCTG GCCGAGCGCA CCGTGGTGAT TCCCTCGGCG 4680
ATCCCCACCG ACCCGCGCAA CGTCGGCGGC GACCTCGACC CGTCCAGCAT CCCCGACAAG 4740
GAACAGGCGA TCAGCGCCCT GCCGGACTAC GCCAGCCAGC CCGGCAAACC GCCGCGCGAG 4800
GACCTGAAGT AACTGCCGCG ACCGGCCGGC TCCCTTCGCA GGAGCCGGCC TTCTCGGGGC 4860
CTGGCCATAC ATCAGGTTTT CCTGATGCCA GCCCAATCGA ATATGAATTC 4910
```

(2) INFORMATION FOR SEQ ID NO:12:

(i) SEQUENCE CHARACTERISTICS:

(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:12:

TATGGAACCG GTCGTTTCTC TGTCGGGCC 29

(2) INFORMATION FOR SEQ ID NO:13:

(i) SEQUENCE CHARACTERISTICS:

(A) LENGTH: 23 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:13:

CGACAGAGAA ACGACCGGTT CCA 23

(2) INFORMATION FOR SEQ ID NO:14:

(i) SEQUENCE CHARACTERISTICS:

(A) LENGTH: 4977 base pairs
(B) TYPE: nucleic acid
(C) STRANDEDNESS: double
(D) TOPOLOGY: circular

(ii) MOLECULE TYPE: DNA (genomic)

(xi) SEQUENCE DESCRIPTION: SEQ ID NO:14:

TTCTTGAAGA CGAAAGGGCC TCGTGATACG CCTATTTTTA TAGGTTAATG TCATGATAAT 60

AATGGTTTCT TAGACGTCAG GTGGCACTTT TCGGGGAAAT GTGCGCGGAA CCCCTATTTG 120

TTTATTTTTC TAAATACATT CAAATATGTA TCCGCTCATG AGACAATAAC CCTGATAAAT 180

GCTTCAATAA TATTGAAAAA GGAAGAGTAT GAGTATTCAA CATTTCCGTG TCGCCCTTAT 240

TCCCTTTTTT GCGGCATTTT GCCTTCCTGT TTTTGCTCAC CCAGAAACGC TGGTGAAAGT 300

AAAAGATGCT GAAGATCAGT TGGGTGCACG AGTGGGTTAC ATCGAACTGG ATCTCAACAG 360

CGGTAAGATC CTTGAGAGTT TTCGCCCCGA AGAACGTTTT CCAATGATGA GCACTTTTAA 420

AGTTCTGCTA TGTGGCGCGG TATTATCCCG TGTTGACGCC GGGCAAGAGC AACTCGGTCG 480

CCGCATACAC TATTCTCAGA ATGACTTGGT TGAGTACTCA CCAGTCACAG AAAAGCATCT 540

TACGGATGGC ATGACAGTAA GAGAATTATG CAGTGCTGCC ATAACCATGA GTGATAACAC 600

TGCGGCCAAC TTACTTCTGA CAACGATCGG AGGACCGAAG GAGCTAACCG CTTTTTTGCA 660

CAACATGGGG GATCATGTAA CTCGCCTTGA TCGTTGGGAA CCGGAGCTGA ATGAAGCCAT 720

ACCAAACGAC GAGCGTGACA CCACGATGCC TGCAGCAATG GCAACAACGT TGCGCAAACT 780

ATTAACTGGC GAACTACTTA CTCTAGCTTC CCGGCAACAA TTAATAGACT GGATGGAGGC 840

GGATAAAGTT GCAGGACCAC TTCTGCGCTC GGCCCTTCCG GCTGGCTGGT TTATTGCTGA 900

TAAATCTGGA GCCGGTGAGC GTGGGTCTCG CGGTATCATT GCAGCACTGG GGCCAGATGG 960

TAAGCCCTCC CGTATCGTAG TTATCTACAC GACGGGGAGT CAGGCAACTA TGGATGAACG 1020

AAATAGACAG ATCGCTGAGA TAGGTGCCTC ACTGATTAAG CATTGGTAAC TGTCAGACCA 1080

AGTTTACTCA TATATACTTT AGATTGATTT AAAACTTCAT TTTTAATTTA AAAGGATCTA 1140

GGTGAAGATC CTTTTTGATA ATCTCATGAC CAAAATCCCT TAACGTGAGT TTTCGTTCCA 1200

CTGAGCGTCA GACCCCGTAG AAAAGATCAA AGGATCTTCT TGAGATCCTT TTTTTCTGCG 1260

CGTAATCTGC TGCTTGCAAA CAAAAAAACC ACCGCTACCA GCGGTGGTTT GTTTGCCGGA 1320

TCAAGAGCTA CCAACTCTTT TTCCGAAGGT AACTGGCTTC AGCAGAGCGC AGATACCAAA 1380

TACTGTCCTT CTAGTGTAGC CGTAGTTAGG CCACCACTTC AAGAACTCTG TAGCACCGCC 1440

TACATACCTC GCTCTGCTAA TCCTGTTACC AGTGGCTGCT GCCAGTGGCG ATAAGTCGTG 1500

TCTTACCGGG TTGGACTCAA GACGATAGTT ACCGGATAAG GCGCAGCGGT CGGGCTGAAC 1560

GGGGGGTTCG TGCACACAGC CCAGCTTGGA GCGAACGACC TACACCGAAC TGAGATACCT 1620

ACAGCGTGAG CATTGAGAAA GCGCCACGCT TCCCGAAGGG AGAAAGGCGG ACAGGTATCC 1680

GGTAAGCGGC AGGGTCGGAA CAGGAGAGCG CACGAGGGAG CTTCCAGGGG GAAACGCCTG 1740

GTATCTTTAT AGTCCTGTCG GGTTTCGCCA CCTCTGACTT GAGCGTCGAT TTTTGTGATG 1800

CTCGTCAGGG GGGCGGAGCC TATGGAAAAA CGCCAGCAAC GCGGCCTTTT TACGGTTCCT 1860

GGCCTTTTGC TGGCCTTTTG CTCACATGTT CTTTCCTGCG TTATCCCCTG ATTCTGTGGA 1920

TAACCGTATT ACCGCCTTTG AGTGAGCTGA TACCGCTCGC CGCAGCCGAA CGACCGAGCG 1980

CAGCGAGTCA GTGAGCGAGG AAGCGGAAGA GCGCCTGATG CGGTATTTTC TCCTTACGCA 2040

TCTGTGCGGT ATTTCACACC GCATATATGG TGCACTCTCA GTACAATCTG CTCTGATGCC 2100

GCATAGTTAA GCCAGTATAC ACTCCGCTAT CGCTACGTGA CTGGGTCATG GCTGCGCCCC 2160

GACACCCGCC AACACCCGCT GACGCGCCCT GACGGGCTTG TCTGCTCCCG GCATCCGCTT 2220

ACAGACAAGC TGTGACCGTC TCCGGGAGCT GCATGTGTCA GAGGTTTTCA CCGTCATCAC 2280

CGAAACGCGC GAGGCAGCTG CGGTAAAGCT CATCAGCGTG GTCGTGAAGC GATTCACAGA 2340

TGTCTGCCTG TTCATCCGCG TCCAGCTCGT TGAGTTTCTC CAGAAGCGTT AATGTCTGGC 2400

TTCTGATAAA GCGGGCCATG TTAAGGGCGG TTTTTTCCTG TTTGGTCACT TGATGCCTCC 2460

GTGTAAGGGG GAATTTCTGT TCATGGGGGT AATGATACCG ATGAAACGAG AGAGGATGCT 2520

CACGATACGG GTTACTGATG ATGAACATGC CCGGTTACTG GAACGTTGTG AGGGTAAACA 2580

ACTGGCGGTA TGGATGCGGC GGGACCAGAG AAAAATCACT CAGGGTCAAT GCCAGCGCTT 2640

CGTTAATACA GATGTAGGTG TTCCACAGGG TAGCCAGCAG CATCCTGCGA TGCAGATCCG 2700

GAACATAATG GTGCAGGGCG CTGACTTCCG CGTTTCCAGA CTTTACGAAA CACGGAAACC 2760

GAAGACCATT CATGTTGTTG CTCAGGTCGC AGACGTTTTG CAGCAGCAGT CGCTTCACGT 2820

TCGCTCGCGT ATCGGTGATT CATTCTGCTA ACCAGTAAGG CAACCCCGCC AGCCTAGCCG 2880

GGTCCTCAAC GACAGGAGCA CGATCATGCG CACCCGTGGC CAGGACCCAA CGCTGCCCGA 2940

GATGCGCCGC GTGCGGCTGC TGGAGATGGC GGACGCGATG GATATGTTCT GCCAAGGGTT 3000

GGTTTGCGCA TTCACAGTTC TCCGCAAGAA TTGATTGGCT CCAATTCTTG GAGTGGTGAA 3060

TCCGTTAGCG AGGTGCCGCC GGCTTCCATT CAGGTCGAGG TGGCCCGGCT CCATGCACCG 3120

CGACGCAACG CGGGGAGGCA GACAAGGTAT AGGGCGGCGC CTACAATCCA TGCCAACCCG 3180

TTCCATGTGC TCGCCGAGGC GGCATAAATC GCCGTGACGA TCAGCGGTCC AGTGATCGAA 3240

GTTAGGCTGG TAAGAGCCGC GAGCGATCCT TGAAGCTGTC CCTGATGGTC GTCATCTACC 3300

TGCCTGGACA GCATGGCCTG CAACGCGGGC ATCCCGATGC CGCCGGAAGC GAGAAGAATC 3360

ATAATGGGGA AGGCCATCCA GCCTCGCGTC GCGAACGCCA GCAAGACGTA GCCCAGCGCG 3420

TCGGCCGCCA TGCCGGCGAT AATGGCCTGC TTCTCGCCGA AACGTTTGGT GGCGGGACCA 3480

GTGACGAAGG CTTGAGCGAG GGCGTGCAAG ATTCCGAATA CCGCAAGCGA CAGGCCGATC 3540

ATCGTCGCGC TCCAGCGAAA GCGGTCCTCG CCGAAAATGA CCCAGAGCGC TGCCGGCACC 3600

TGTCCTACGA GTTGCATGAT AAAGAAGACA GTCATAAGTG CGGCGACGAT AGTCATGCCC 3660

CGCGCCCACC GGAAGGAGCT GACTGGGTTG AAGGCTCTCA AGGGCATCGG TCGACGCTCT 3720

CCCTTATGCG ACTCCTGCAT TAGGAAGCAG CCCAGTAGTA GGTTGAGGCC GTTGAGCACC 3780

GCCGCCGCAA GGAATGGTGC ATGCAAGGAG ATGGCGCCCA ACAGTCCCCC GGCCACGGGG 3840

CCTGCCACCA TACCCACGCC GAAACAAGCG CTCATGAGCC CGAAGTGGCG AGCCCGATCT 3900

TCCCCATCGG TGATGTCGGC GATATAGGCG CCAGCAACCG CACCTGTGGC GCCGGTGATG 3960

CCGGCCACGA TGCGTCCGGC GTAGAGGATC GAGATCTCCA TCCCGCGAAA TTAATACGAC 4020

```
TCACTATAGG GAGACCACAA CGGTTTCCCT CTAGAAATAA TTTTGTTTAA CTTTAAGAAG    4080
GAGATATACC ATGGTACCAG ACACCGGAAA CCCCTGCCAC ACCACTAAGT TGTTGCACAG    4140
AGACTCAGTG GACAGTGCTC CAATCCTCAC TGCATTTAAC AGCTCACACA AAGGACGGAT    4200
TAACTGTAAT AGTAACACTA CACCCATAGT ACATTTAAAA GGTGATGCTA ATACTTTAAA    4260
ATGTTTAAGA TATAGATTTA AAAAGCATTG TACATTGTAT ACTGCAGTGT CGTCTACATG    4320
GCATTGGACA GGACATAATG TAAAACATAA AAGTGCAATT GTTACACTTA CATATGATAG    4380
TGAATGGCAA CGTGACCAAT TTTTGTCTCA AGTTAAAATA CCAAAAACTA TTACAGTGTC    4440
TACTGGATTT ATGTCTATAT GAGGATCCGG CTGCTAACAA AGCCCGAAAG GAAGCTGAGT    4500
TGGCTGCTGC CACCGCTGAG CAATAACTAG CATAACCCCT TGGGGCCTCT AAACGGGTCT    4560
TGAGGGGTTT TTTGCTGAAA GGAGGAACTA TATCCGGATA TCCACAGGAC GGGTGTGGTC    4620
GCCATGATCG CGTAGTCGAT AGTGGCTCCA AGTAGCGAAG CGAGCAGGAC TGGGCGGCGG    4680
CCAAAGCGGT CGGACAGTGC TCCGAGAACG GGTGCGCATA GAAATTGCAT CAACGCATAT    4740
AGCGCTAGCA GCACGCCATA GTGACTGGCG ATGCTGTCGG AATGGACGAT ATCCCGCAAG    4800
AGGCCCGGCA GTACCGGCAT AACCAAGCCT ATGCCTACAG CATCCAGGGT GACGGTGCCG    4860
AGGATGACGA TGAGCGCATT GTTAGATTTC ATACACGGTG CCTGACTGCG TTAGCAATTT    4920
AACTGTGATA AACTACCGCA TTAAAGCTTA TCGATGATAA GCTGTCAAAC ATGAGAA       4977
```

(2) INFORMATION FOR SEQ ID NO:15:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:15:

```
CTCCCATGGT ACCAGACACC GGAAACC    27
```

(2) INFORMATION FOR SEQ ID NO:16:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:16:

GGGGGATCCT CATATAGACA TAAATCC 27

(2) INFORMATION FOR SEQ ID NO:17:

(i) SEQUENCE CHARACTERISTICS:

(A) LENGTH: 4977 base pairs
(B) TYPE: nucleic acid
(C) STRANDEDNESS: double
(D) TOPOLOGY: circular

(ii) MOLECULE TYPE: DNA (genomic)

(xi) SEQUENCE DESCRIPTION: SEQ ID NO:17:

TTCTTGAAGA CGAAAGGGCC TCGTGATACG CCTATTTTTA TAGGTTAATG TCATGATAAT 60

AATGGTTTCT TAGACGTCAG GTGGCACTTT TCGGGGAAAT GTGCGCGGAA CCCCTATTTG 120

TTTATTTTTC TAAATACATT CAAATATGTA TCCGCTCATG AGACAATAAC CCTGATAAAT 180

GCTTCAATAA TATTGAAAAA GGAAGAGTAT GAGTATTCAA CATTTCCGTG TCGCCCTTAT 240

TCCCTTTTTT GCGGCATTTT GCCTTCCTGT TTTTGCTCAC CCAGAAACGC TGGTGAAAGT 300

AAAAGATGCT GAAGATCAGT TGGGTGCACG AGTGGGTTAC ATCGAACTGG ATCTCAACAG 360

CGGTAAGATC CTTGAGAGTT TTCGCCCCGA AGAACGTTTT CCAATGATGA GCACTTTTAA 420

AGTTCTGCTA TGTGGCGCGG TATTATCCCG TGTTGACGCC GGGCAAGAGC AACTCGGTCG 480

CCGCATACAC TATTCTCAGA ATGACTTGGT TGAGTACTCA CCAGTCACAG AAAAGCATCT 540

TACGGATGGC ATGACAGTAA GAGAATTATG CAGTGCTGCC ATAACCATGA GTGATAACAC 600

TGCGGCCAAC TTACTTCTGA CAACGATCGG AGGACCGAAG GAGCTAACCG CTTTTTTGCA 660

CAACATGGGG GATCATGTAA CTCGCCTTGA TCGTTGGGAA CCGGAGCTGA ATGAAGCCAT 720

ACCAAACGAC GAGCGTGACA CCACGATGCC TGCAGCAATG GCAACAACGT TGCGCAAACT 780

ATTAACTGGC GAACTACTTA CTCTAGCTTC CCGGCAACAA TTAATAGACT GGATGGAGGC 840

GGATAAAGTT GCAGGACCAC TTCTGCGCTC GGCCCTTCCG GCTGGCTGGT TTATTGCTGA 900

TAAATCTGGA GCCGGTGAGC GTGGGTCTCG CGGTATCATT GCAGCACTGG GGCCAGATGG 960

TAAGCCCTCC CGTATCGTAG TTATCTACAC GACGGGGAGT CAGGCAACTA TGGATGAACG 1020

AAATAGACAG ATCGCTGAGA TAGGTGCCTC ACTGATTAAG CATTGGTAAC TGTCAGACCA 1080

AGTTTACTCA TATATACTTT AGATTGATTT AAAACTTCAT TTTTAATTTA AAAGGATCTA 1140

GGTGAAGATC CTTTTTGATA ATCTCATGAC CAAAATCCCT TAACGTGAGT TTTCGTTCCA 1200

CTGAGCGTCA GACCCCGTAG AAAAGATCAA AGGATCTTCT TGAGATCCTT TTTTTCTGCG 1260

CGTAATCTGC TGCTTGCAAA CAAAAAAACC ACCGCTACCA GCGGTGGTTT GTTTGCCGGA 1320

TCAAGAGCTA CCAACTCTTT TTCCGAAGGT AACTGGCTTC AGCAGAGCGC AGATACCAAA 1380

TACTGTCCTT CTAGTGTAGC CGTAGTTAGG CCACCACTTC AAGAACTCTG TAGCACCGCC 1440

TACATACCTC GCTCTGCTAA TCCTGTTACC AGTGGCTGCT GCCAGTGGCG ATAAGTCGTG 1500

TCTTACCGGG TTGGACTCAA GACGATAGTT ACCGGATAAG GCGCAGCGGT CGGGCTGAAC 1560

GGGGGGTTCG TGCACACAGC CCAGCTTGGA GCGAACGACC TACACCGAAC TGAGATACCT 1620

ACAGCGTGAG CATTGAGAAA GCGCCACGCT TCCCGAAGGG AGAAAGGCGG ACAGGTATCC 1680

GGTAAGCGGC AGGGTCGGAA CAGGAGAGCG CACGAGGGAG CTTCCAGGGG GAAACGCCTG 1740

GTATCTTTAT AGTCCTGTCG GGTTTCGCCA CCTCTGACTT GAGCGTCGAT TTTTGTGATG 1800

CTCGTCAGGG GGGCGGAGCC TATGGAAAAA CGCCAGCAAC GCGGCCTTTT TACGGTTCCT 1860

GGCCTTTTGC TGGCCTTTTG CTCACATGTT CTTTCCTGCG TTATCCCCTG ATTCTGTGGA 1920

TAACCGTATT ACCGCCTTTG AGTGAGCTGA TACCGCTCGC CGCAGCCGAA CGACCGAGCG 1980

CAGCGAGTCA GTGAGCGAGG AAGCGGAAGA GCGCCTGATG CGGTATTTTC TCCTTACGCA 2040

TCTGTGCGGT ATTTCACACC GCATATATGG TGCACTCTCA GTACAATCTG CTCTGATGCC 2100

GCATAGTTAA GCCAGTATAC ACTCCGCTAT CGCTACGTGA CTGGGTCATG GCTGCGCCCC 2160

GACACCCGCC AACACCCGCT GACGCGCCCT GACGGGCTTG TCTGCTCCCG GCATCCGCTT 2220

ACAGACAAGC TGTGACCGTC TCCGGGAGCT GCATGTGTCA GAGGTTTTCA CCGTCATCAC 2280

CGAAACGCGC GAGGCAGCTG CGGTAAAGCT CATCAGCGTG GTCGTGAAGC GATTCACAGA 2340

TGTCTGCCTG TTCATCCGCG TCCAGCTCGT TGAGTTTCTC CAGAAGCGTT AATGTCTGGC 2400

TTCTGATAAA GCGGGCCATG TTAAGGGCGG TTTTTTCCTG TTTGGTCACT TGATGCCTCC 2460

GTGTAAGGGG GAATTTCTGT TCATGGGGGT AATGATACCG ATGAAACGAG AGAGGATGCT 2520

CACGATACGG GTTACTGATG ATGAACATGC CCGGTTACTG GAACGTTGTG AGGGTAAACA 2580

ACTGGCGGTA TGGATGCGGC GGGACCAGAG AAAAATCACT CAGGGTCAAT GCCAGCGCTT 2640

CGTTAATACA GATGTAGGTG TTCCACAGGG TAGCCAGCAG CATCCTGCGA TGCAGATCCG 2700

GAACATAATG GTGCAGGGCG CTGACTTCCG CGTTTCCAGA CTTTACGAAA CACGGAAACC 2760
GAAGACCATT CATGTTGTTG CTCAGGTCGC AGACGTTTTG CAGCAGCAGT CGCTTCACGT 2820
TCGCTCGCGT ATCGGTGATT CATTCTGCTA ACCAGTAAGG CAACCCCGCC AGCCTAGCCG 2880
GGTCCTCAAC GACAGGAGCA CGATCATGCG CACCCGTGGC CAGGACCCAA CGCTGCCCGA 2940
GATGCGCCGC GTGCGGCTGC TGGAGATGGC GGACGCGATG GATATGTTCT GCCAAGGGTT 3000
GGTTTGCGCA TTCACAGTTC TCCGCAAGAA TTGATTGGCT CCAATTCTTG GAGTGGTGAA 3060
TCCGTTAGCG AGGTGCCGCC GGCTTCCATT CAGGTCGAGG TGGCCCGGCT CCATGCACCG 3120
CGACGCAACG CGGGGAGGCA GACAAGGTAT AGGGCGGCGC CTACAATCCA TGCCAACCCG 3180
TTCCATGTGC TCGCCGAGGC GGCATAAATC GCCGTGACGA TCAGCGGTCC AGTGATCGAA 3240
GTTAGGCTGG TAAGAGCCGC GAGCGATCCT TGAAGCTGTC CCTGATGGTC GTCATCTACC 3300
TGCCTGGACA GCATGGCCTG CAACGCGGGC ATCCCGATGC CGCCGGAAGC GAGAAGAATC 3360
ATAATGGGGA AGGCCATCCA GCCTCGCGTC GCGAACGCCA GCAAGACGTA GCCCAGCGCG 3420
TCGGCCGCCA TGCCGGCGAT AATGGCCTGC TTCTCGCCGA AACGTTTGGT GGCGGGACCA 3480
GTGACGAAGG CTTGAGCGAG GGCGTGCAAG ATTCCGAATA CCGCAAGCGA CAGGCCGATC 3540
ATCGTCGCGC TCCAGCGAAA GCGGTCCTCG CCGAAAATGA CCCAGAGCGC TGCCGGCACC 3600
TGTCCTACGA GTTGCATGAT AAAGAAGACA GTCATAAGTG CGGCGACGAT AGTCATGCCC 3660
CGCGCCCACC GGAAGGAGCT GACTGGGTTG AAGGCTCTCA AGGGCATCGG TCGACGCTCT 3720
CCCTTATGCG ACTCCTGCAT TAGGAAGCAG CCCAGTAGTA GGTTGAGGCC GTTGAGCACC 3780
GCCGCCGCAA GGAATGGTGC ATGCAAGGAG ATGGCGCCCA ACAGTCCCCC GGCCACGGGG 3840
CCTGCCACCA TACCCACGCC GAAACAAGCG CTCATGAGCC CGAAGTGGCG AGCCCGATCT 3900
TCCCCATCGG TGATGTCGGC GATATAGGCG CCAGCAACCG CACCTGTGGC GCCGGTGATG 3960
CCGGCCACGA TGCGTCCGGC GTAGAGGATC GAGATCTCGA TCCCGCGAAA TTAATACGAC 4020
TCACTATAGG GAGACCACAA CGGTTTCCCT CTAGAAATAA TTTTGTTTAA CTTTAAGAAG 4080
GAGATATACC ATGGTACCAG ACACCGGAAA CCCCTGCCAC ACCACTAAGT TGTTGCACAG 4140
AGACTCAGTG GACAGTGCTC CAATCCTCAC TGCATTTAAC AGCTCACACA AAGGACGGAT 4200
TAACTGTAAT AGTAACACTA CACCCATAGT ACATTTAAAA GGTGATGCTA ATACTTTAAG 4260
ATCTTTAAGA TATAGATTTA AAAAGCATTC TACATTGTAT ACTGCAGTGT CGTCTACATG 4320
GCATTGGACA GGACATAATG TAAAACATAA AAGTGCAATT GTTACACTTA CATATGATAG 4380

```
TGAATGGCAA CGTGACCAAT TTTTGTCTCA AGTTAAAATA CCAAAAACTA TTACAGTGTC    4440
TACTGGATTT ATGTCTATAT GAGGATCCGG CTGCTAACAA AGCCCGAAAG GAAGCTGAGT    4500
TGGCTGCTGC CACCGCTGAG CAATAACTAG CATAACCCCT TGGGGCCTCT AAACGGGTCT    4560
TGAGGGGTTT TTTGCTGAAA GGAGGAACTA TATCCGGATA TCCACAGGAC GGGTGTGGTC    4620
GCCATGATCG CGTAGTCGAT AGTGGCTCCA AGTAGCGAAG CGAGCAGGAC TGGGCGGCGG    4680
CCAAAGCGGT CGGACAGTGC TCCGAGAACG GGTGCGCATA GAAATTGCAT CAACGCATAT    4740
AGCGCTAGCA GCACGCCATA GTGACTGGCG ATGCTGTCGG AATGGACGAT ATCCCGCAAG    4800
AGGCCCGGCA GTACCGGCAT AACCAAGCCT ATGCCTACAG CATCCAGGGT GACGGTGCCG    4860
AGGATGACGA TGAGCGCATT GTTAGATTTC ATACACGGTG CCTGACTGCG TTAGCAATTT    4920
AACTGTGATA AACTACCGCA TTAAAGCTTA TCGATGATAA GCTGTCAAAC ATGAGAA       4977
```

(2) INFORMATION FOR SEQ ID NO:18:

(i) SEQUENCE CHARACTERISTICS:

(A) LENGTH: 59 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:18:

```
CGACACTGCA GTATACAATG TAGAATGCTT TTTAAATCTA TATCTTAAAG ATCTTAAAG    59
```

(2) INFORMATION FOR SEQ ID NO:19:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:19:

```
GCGTCGGCCG CCATGCCGGC GATAAT    26
```

(2) INFORMATION FOR SEQ ID NO:20:

(i) SEQUENCE CHARACTERISTICS:

(A) LENGTH: 4819 base pairs
(B) TYPE: nucleic acid
(C) STRANDEDNESS: double
(D) TOPOLOGY: circular

(ii) MOLECULE TYPE: DNA (genomic)

(xi) SEQUENCE DESCRIPTION: SEQ ID NO:20:

```
TTCTTGAAGA CGAAAGGGCC TCGTGATACG CCTATTTTTA TAGGTTAATG TCATGATAAT     60
AATGGTTTCT TAGACGTCAG GTGGCACTTT TCGGGGAAAT GTGCGCGGAA CCCCTATTTG    120
TTTATTTTTC TAAATACATT CAAATATGTA TCCGCTCATG AGACAATAAC CCTGATAAAT    180
GCTTCAATAA TATTGAAAAA GGAAGAGTAT GAGTATTCAA CATTTCCGTG TCGCCCTTAT    240
TCCCTTTTTT GCGGCATTTT GCCTTCCTGT TTTTGCTCAC CCAGAAACGC TGGTGAAAGT    300
AAAAGATGCT GAAGATCAGT TGGGTGCACG AGTGGGTTAC ATCGAACTGG ATCTCAACAG    360
CGGTAAGATC CTTGAGAGTT TTCGCCCCGA AGAACGTTTT CCAATGATGA GCACTTTTAA    420
AGTTCTGCTA TGTGGCGCGG TATTATCCCG TGTTGACGCC GGGCAAGAGC AACTCGGTCG    480
CCGCATACAC TATTCTCAGA ATGACTTGGT TGAGTACTCA CCAGTCACAG AAAAGCATCT    540
TACGGATGGC ATGACAGTAA GAGAATTATG CAGTGCTGCC ATAACCATGA GTGATAACAC    600
TGCGGCCAAC TTACTTCTGA CAACGATCGG AGGACCGAAG GAGCTAACCG CTTTTTTGCA    660
CAACATGGGG GATCATGTAA CTCGCCTTGA TCGTTGGGAA CCGGAGCTGA ATGAAGCCAT    720
ACCAAACGAC GAGCGTGACA CCACGATGCC TGCAGCAATG GCAACAACGT TGCGCAAACT    780
ATTAACTGGC GAACTACTTA CTCTAGCTTC CCGGCAACAA TTAATAGACT GGATGGAGGC    840
GGATAAAGTT GCAGGACCAC TTCTGCGCTC GGCCCTTCCG GCTGGCTGGT TTATTGCTGA    900
TAAATCTGGA GCCGGTGAGC GTGGGTCTCG CGGTATCATT GCAGCACTGG GGCCAGATGG    960
TAAGCCCTCC CGTATCGTAG TTATCTACAC GACGGGGAGT CAGGCAACTA TGGATGAACG   1020
AAATAGACAG ATCGCTGAGA TAGGTGCCTC ACTGATTAAG CATTGGTAAC TGTCAGACCA   1080
AGTTTACTCA TATATACTTT AGATTGATTT AAAACTTCAT TTTTAATTTA AAAGGATCTA   1140
GGTGAAGATC CTTTTTGATA ATCTCATGAC CAAAATCCCT TAACGTGAGT TTTCGTTCCA   1200
CTGAGCGTCA GACCCCGTAG AAAAGATCAA AGGATCTTCT TGAGATCCTT TTTTTCTGCG   1260
CGTAATCTGC TGCTTGCAAA CAAAAAAACC ACCGCTACCA GCGGTGGTTT GTTTGCCGGA   1320
TCAAGAGCTA CCAACTCTTT TTCCGAAGGT AACTGGCTTC AGCAGAGCGC AGATACCAAA   1380
```

TACTGTCCTT CTAGTGTAGC CGTAGTTAGG CCACCACTTC AAGAACTCTG TAGCACCGCC 1440

TACATACCTC GCTCTGCTAA TCCTGTTACC AGTGGCTGCT GCCAGTGGCG ATAAGTCGTG 1500

TCTTACCGGG TTGGACTCAA GACGATAGTT ACCGGATAAG GCGCAGCGGT CGGGCTGAAC 1560

GGGGGGTTCG TGCACACAGC CCAGCTTGGA GCGAACGACC TACACCGAAC TGAGATACCT 1620

ACAGCGTGAG CATTGAGAAA GCGCCACGCT TCCCGAAGGG AGAAAGGCGG ACAGGTATCC 1680

GGTAAGCGGC AGGGTCGGAA CAGGAGAGCG CACGAGGGAG CTTCCAGGGG GAAACGCCTG 1740

GTATCTTTAT AGTCCTGTCG GGTTTCGCCA CCTCTGACTT GAGCGTCGAT TTTTGTGATG 1800

CTCGTCAGGG GGGCGGAGCC TATGGAAAAA CGCCAGCAAC GCGGCCTTTT TACGGTTCCT 1860

GGCCTTTTGC TGGCCTTTTG CTCACATGTT CTTTCCTGCG TTATCCCCTG ATTCTGTGGA 1920

TAACCGTATT ACCGCCTTTG AGTGAGCTGA TACCGCTCGC CGCAGCCGAA CGACCGAGCG 1980

CAGCGAGTCA GTGAGCGAGG AAGCGGAAGA GCGCCTGATG CGGTATTTTC TCCTTACGCA 2040

TCTGTGCGGT ATTTCACACC GCATATATGG TGCACTCTCA GTACAATCTG CTCTGATGCC 2100

GCATAGTTAA GCCAGTATAC ACTCCGCTAT CGCTACGTGA CTGGGTCATG GCTGCGCCCC 2160

GACACCCGCC AACACCCGCT GACGCGCCCT GACGGGCTTG TCTGCTCCCG GCATCCGCTT 2220

ACAGACAAGC TGTGACCGTC TCCGGGAGCT GCATGTGTCA GAGGTTTTCA CCGTCATCAC 2280

CGAAACGCGC GAGGCAGCTG CGGTAAAGCT CATCAGCGTG GTCGTGAAGC GATTCACAGA 2340

TGTCTGCCTG TTCATCCGCG TCCAGCTCGT TGAGTTTCTC CAGAAGCGTT AATGTCTGGC 2400

TTCTGATAAA GCGGGCCATG TTAAGGGCGG TTTTTTCCTG TTTGGTCACT TGATGCCTCC 2460

GTGTAAGGGG GAATTTCTGT TCATGGGGGT AATGATACCG ATGAAACGAG AGAGGATGCT 2520

CACGATACGG GTTACTGATG ATGAACATGC CCGGTTACTG GAACGTTGTG AGGGTAAACA 2580

ACTGGCGGTA TGGATGCGGC GGGACCAGAG AAAAATCACT CAGGGTCAAT GCCAGCGCTT 2640

CGTTAATACA GATGTAGGTG TTCCACAGGG TAGCCAGCAG CATCCTGCGA TGCAGATCCG 2700

GAACATAATG GTGCAGGGCG CTGACTTCCG CGTTTCCAGA CTTTACGAAA CACGGAAACC 2760

GAAGACCATT CATGTTGTTG CTCAGGTCGC AGACGTTTTG CAGCAGCAGT CGCTTCACGT 2820

TCGCTCGCGT ATCGGTGATT CATTCTGCTA ACCAGTAAGG CAACCCCGCC AGCCTAGCCG 2880

GGTCCTCAAC GACAGGAGCA CGATCATGCG CACCCGTGGC CAGGACCCAA CGCTGCCCGA 2940

GATGCGCCGC GTGCGGCTGC TGGAGATGGC GGACGCGATG GATATGTTCT GCCAAGGGTT 3000

GGTTTGCGCA TTCACAGTTC TCCGCAAGAA TTGATTGGCT CCAATTCTTG GAGTGGTGAA 3060

TCCGTTAGCG AGGTGCCGCC GGCTTCCATT CAGGTCGAGG TGGCCCGGCT CCATGCACCG 3120
CGACGCAACG CGGGGAGGCA GACAAGGTAT AGGGCGGCGC CTACAATCCA TGCCAACCCG 3180
TTCCATGTGC TCGCCGAGGC GGCATAAATC GCCGTGACGA TCAGCGGTCC AGTGATCGAA 3240
GTTAGGCTGG TAAGAGCCGC GAGCGATCCT TGAAGCTGTC CCTGATGGTC GTCATCTACC 3300
TGCCTGGACA GCATGGCCTG CAACGCGGGC ATCCCGATGC CGCCGGAAGC GAGAAGAATC 3360
ATAATGGGGA AGGCCATCCA GCCTCGCGTC GCGAACGCCA GCAAGACGTA GCCCAGCGCG 3420
TCGGCCGCCA TGCCGGCGAT AATGGCCTGC TTCTCGCCGA AACGTTTGGT GGCGGGACCA 3480
GTGACGAAGG CTTGAGCGAG GGCGTGCAAG ATTCCGAATA CCGCAAGCGA CAGGCCGATC 3540
ATCGTCGCGC TCCAGCGAAA GCGGTCCTCG CCGAAAATGA CCCAGAGCGC TGCCGGCACC 3600
TGTCCTACGA GTTGCATGAT AAAGAAGACA GTCATAAGTG CGGCGACGAT AGTCATGCCC 3660
CGCGCCCACC GGAAGGAGCT GACTGGGTTG AAGGCTCTCA AGGGCATCGG TCGACGCTCT 3720
CCCTTATGCG ACTCCTGCAT TAGGAAGCAG CCCAGTAGTA GGTTGAGGCC GTTGAGCACC 3780
GCCGCCGCAA GGAATGGTGC ATGCAAGGAG ATGGCGCCCA ACAGTCCCCC GGCCACGGGG 3840
CCTGCCACCA TACCCACGCC GAAACAAGCG CTCATGAGCC CGAAGTGGCG AGCCCGATCT 3900
TCCCCATCGG TGATGTCGGC GATATAGGCG CCAGCAACCG CACCTGTGGC GCCGGTGATG 3960
CCGGCCACGA TGCGTCCGGC GTAGAGGATC GAGATCTCGA TCCCGCGAAA TTAATACGAC 4020
TCACTATAGG GAGACCACAA CGGTTTCCCT CTAGAAATAA TTTTGTTTAA CTTTAAGAAG 4080
GAGATATACA TATGGAACCG GTCGACCCGC GTCTGGAACC ATGGAAACAC CCCGGGTCCC 4140
AGCCGAAAAC CGCGTTCATC ACCAAAGCCC TAGGTATCTC TTACGGCCGT AAAAAACGTC 4200
GTCAGCGACG TCGTCCGCCG CAGGGATCCC AGACCCACCA GGTTTCTCTG TCTAAACAGT 4260
GATCAGCATT GGCTAGCATG ACTGGTGGAC AGCAAATGGG TCGCGGATCC GGCTGCTAAC 4320
AAAGCCCGAA AGGAAGCTGA GTTGGCTGCT GCCACCGCTG AGCAATAACT AGCATAACCC 4380
CTTGGGGCCT CTAAACGGGT CTTGAGGGGT TTTTTGCTGA AAGGAGGAAC TATATCCGGA 4440
TATCCACAGG ACGGGTGTGG TCGCCATGAT CGCGTAGTCG ATAGTGGCTC CAAGTAGCGA 4500
AGCGAGCAGG ACTGGGCGGC GGCCAAAGCG GTCGGACAGT GCTCCGAGAA CGGGTGCGCA 4560
TAGAAATTGC ATCAACGCAT ATAGCGCTAG CAGCACGCCA TAGTGACTGG CGATGCTGTC 4620
GGAATGGACG ATATCCCGCA AGAGGCCCGG CAGTACCGGC ATAACCAAGC CTATGCCTAC 4680
AGCATCCAGG GTGACGGTGC CGAGGATGAC GATGAGCGCA TTGTTAGATT TCATACACGG 4740

TGCCTGACTG CGTTAGCAAT TTAACTGTGA TAAACTACCG CATTAAAGCT TATCGATGAT 4800

AAGCTGTCAA ACATGAGAA 4819

(2) INFORMATION FOR SEQ ID NO:21:

(i) SEQUENCE CHARACTERISTICS:

(A) LENGTH: 45 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:21:

TTTACGGCCG TAAGAGATAC CTAGGGCTTT GGTGATGAAC GCGGT 45

(2) INFORMATION FOR SEQ ID NO:22:

(i) SEQUENCE CHARACTERISTICS:

(A) LENGTH: 5574 base pairs
(B) TYPE: nucleic acid
(C) STRANDEDNESS: double
(D) TOPOLOGY: circular

(ii) MOLECULE TYPE: DNA (genomic)

(xi) SEQUENCE DESCRIPTION: SEQ ID NO:22:

TTCTTGAAGA CGAAAGGGCC TCGTGATACG CCTATTTTTA TAGGTTAATG TCATGATAAT 60

AATGGTTTCT TAGACGTCAG GTGGCACTTT TCGGGGAAAT GTGCGCGGAA CCCCTATTTG 120

TTTATTTTTC TAAATACATT CAAATATGTA TCCGCTCATG AGACAATAAC CCTGATAAAT 180

GCTTCAATAA TATTGAAAAA GGAAGAGTAT GAGTATTCAA CATTTCCGTG TCGCCCTTAT 240

TCCCTTTTTT GCGGCATTTT GCCTTCCTGT TTTTGCTCAC CCAGAAACGC TGGTGAAAGT 300

AAAAGATGCT GAAGATCAGT TGGGTGCACG AGTGGGTTAC ATCGAACTGG ATCTCAACAG 360

CGGTAAGATC CTTGAGAGTT TTCGCCCCGA AGAACGTTTT CCAATGATGA GCACTTTTAA 420

AGTTCTGCTA TGTGGCGCGG TATTATCCCG TGTTGACGCC GGGCAAGAGC AACTCGGTCG 480

CCGCATACAC TATTCTCAGA ATGACTTGGT TGAGTACTCA CCAGTCACAG AAAAGCATCT 540

TACGGATGGC ATGACAGTAA GAGAATTATG CAGTGCTGCC ATAACCATGA GTGATAACAC 600

TGCGGCCAAC TTACTTCTGA CAACGATCGG AGGACCGAAG GAGCTAACCG CTTTTTTGCA 660

CAACATGGGG GATCATGTAA CTCGCCTTGA TCGTTGGGAA CCGGAGCTGA ATGAAGCCAT 720
ACCAAACGAC GAGCGTGACA CCACGATGCC TGCAGCAATG GCAACAACGT TGCGCAAACT 780
ATTAACTGGC GAACTACTTA CTCTAGCTTC CCGGCAACAA TTAATAGACT GGATGGAGGC 840
GGATAAAGTT GCAGGACCAC TTCTGCGCTC GGCCCTTCCG GCTGGCTGGT TTATTGCTGA 900
TAAATCTGGA GCCGGTGAGC GTGGGTCTCG CGGTATCATT GCAGCACTGG GGCCAGATGG 960
TAAGCCCTCC CGTATCGTAG TTATCTACAC GACGGGGAGT CAGGCAACTA TGGATGAACG 1020
AAATAGACAG ATCGCTGAGA TAGGTGCCTC ACTGATTAAG CATTGGTAAC TGTCAGACCA 1080
AGTTTACTCA TATATACTTT AGATTGATTT AAAACTTCAT TTTTAATTTA AAAGGATCTA 1140
GGTGAAGATC CTTTTTGATA ATCTCATGAC CAAAATCCCT TAACGTGAGT TTTCGTTCCA 1200
CTGAGCGTCA GACCCCGTAG AAAAGATCAA AGGATCTTCT TGAGATCCTT TTTTTCTGCG 1260
CGTAATCTGC TGCTTGCAAA CAAAAAAACC ACCGCTACCA GCGGTGGTTT GTTTGCCGGA 1320
TCAAGAGCTA CCAACTCTTT TTCCGAAGGT AACTGGCTTC AGCAGAGCGC AGATACCAAA 1380
TACTGTCCTT CTAGTGTAGC CGTAGTTAGG CCACCACTTC AAGAACTCTG TAGCACCGCC 1440
TACATACCTC GCTCTGCTAA TCCTGTTACC AGTGGCTGCT GCCAGTGGCG ATAAGTCGTG 1500
TCTTACCGGG TTGGACTCAA GACGATAGTT ACCGGATAAG GCGCAGCGGT CGGGCTGAAC 1560
GGGGGGTTCG TGCACACAGC CCAGCTTGGA GCGAACGACC TACACCGAAC TGAGATACCT 1620
ACAGCGTGAG CATTGAGAAA GCGCCACGCT TCCCGAAGGG AGAAAGGCGG ACAGGTATCC 1680
GGTAAGCGGC AGGGTCGGAA CAGGAGAGCG CACGAGGGAG CTTCCAGGGG GAAACGCCTG 1740
GTATCTTTAT AGTCCTGTCG GGTTTCGCCA CCTCTGACTT GAGCGTCGAT TTTTGTGATG 1800
CTCGTCAGGG GGGCGGAGCC TATGGAAAAA CGCCAGCAAC GCGGCCTTTT TACGGTTCCT 1860
GGCCTTTTGC TGGCCTTTTG CTCACATGTT CTTTCCTGCG TTATCCCCTG ATTCTGTGGA 1920
TAACCGTATT ACCGCCTTTG AGTGAGCTGA TACCGCTCGC CGCAGCCGAA CGACCGAGCG 1980
CAGCGAGTCA GTGAGCGAGG AAGCGGAAGA GCGCCTGATG CGGTATTTTC TCCTTACGCA 2040
TCTGTGCGGT ATTTCACACC GCATATATGG TGCACTCTCA GTACAATCTG CTCTGATGCC 2100
GCATAGTTAA GCCAGTATAC ACTCCGCTAT CGCTACGTGA CTGGGTCATG GCTGCGCCCC 2160
GACACCCGCC AACACCCGCT GACGCGCCCT GACGGGCTTG TCTGCTCCCG GCATCCGCTT 2220
ACAGACAAGC TGTGACCGTC TCCGGGAGCT GCATGTGTCA GAGGTTTTCA CCGTCATCAC 2280
CGAAACGCGC GAGGCAGCTG CGGTAAAGCT CATCAGCGTG GTCGTGAAGC GATTCACAGA 2340

TGTCTGCCTG TTCATCCGCG TCCAGCTCGT TGAGTTTCTC CAGAAGCGTT AATGTCTGGC 2400

TTCTGATAAA GCGGGCCATG TTAAGGGCGG TTTTTTCCTG TTTGGTCACT TGATGCCTCC 2460

GTGTAAGGGG GAATTTCTGT TCATGGGGGT AATGATACCG ATGAAACGAG AGAGGATGCT 2520

CACGATACGG GTTACTGATG ATGAACATGC CCGGTTACTG GAACGTTGTG AGGGTAAACA 2580

ACTGGCGGTA TGGATGCGGC GGGACCAGAG AAAAATCACT CAGGGTCAAT GCCAGCGCTT 2640

CGTTAATACA GATGTAGGTG TTCCACAGGG TAGCCAGCAG CATCCTGCGA TGCAGATCCG 2700

GAACATAATG GTGCAGGGCG CTGACTTCCG CGTTTCCAGA CTTTACGAAA CACGGAAACC 2760

GAAGACCATT CATGTTGTTG CTCAGGTCGC AGACGTTTTG CAGCAGCAGT CGCTTCACGT 2820

TCGCTCGCGT ATCGGTGATT CATTCTGCTA ACCAGTAAGG CAACCCCGCC AGCCTAGCCG 2880

GGTCCTCAAC GACAGGAGCA CGATCATGCG CACCCGTGGC CAGGACCCAA CGCTGCCCGA 2940

GATGCGCCGC GTGCGGCTGC TGGAGATGGC GGACGCGATG GATATGTTCT GCCAAGGGTT 3000

GGTTTGCGCA TTCACAGTTC TCCGCAAGAA TTGATTGGCT CCAATTCTTG GAGTGGTGAA 3060

TCCGTTAGCG AGGTGCCGCC GGCTTCCATT CAGGTCGAGG TGGCCCGGCT CCATGCACCG 3120

CGACGCAACG CGGGGAGGCA GACAAGGTAT AGGGCGGCGC CTACAATCCA TGCCAACCCG 3180

TTCCATGTGC TCGCCGAGGC GGCATAAATC GCCGTGACGA TCAGCGGTCC AGTGATCGAA 3240

GTTAGGCTGG TAAGAGCCGC GAGCGATCCT TGAAGCTGTC CCTGATGGTC GTCATCTACC 3300

TGCCTGGACA GCATGGCCTG CAACGCGGGC ATCCCGATGC CGCCGGAAGC GAGAAGAATC 3360

ATAATGGGGA AGGCCATCCA GCCTCGCGTC GCGAACGCCA GCAAGACGTA GCCCAGCGCG 3420

TCGGCCGCCA TGCCGGCGAT AATGGCCTGC TTCTCGCCGA AACGTTTGGT GGCGGGACCA 3480

GTGACGAAGG CTTGAGCGAG GGCGTGCAAG ATTCCGAATA CCGCAAGCGA CAGGCCGATC 3540

ATCGTCGCGC TCCAGCGAAA GCGGTCCTCG CCGAAAATGA CCCAGAGCGC TGCCGGCACC 3600

TGTCCTACGA GTTGCATGAT AAAGAAGACA GTCATAAGTG CGGCGACGAT AGTCATGCCC 3660

CGCGCCCACC GGAAGGAGCT GACTGGGTTG AAGGCTCTCA AGGGCATCGG TCGACGCTCT 3720

CCCTTATGCG ACTCCTGCAT TAGGAAGCAG CCCAGTAGTA GGTTGAGGCC GTTGAGCACC 3780

GCCGCCGCAA GGAATGGTGC ATGCAAGGAG ATGGCGCCCA ACAGTCCCCC GGCCACGGGG 3840

CCTGCCACCA TACCCACGCC GAAACAAGCG CTCATGAGCC CGAAGTGGCG AGCCCGATCT 3900

TCCCCATCGG TGATGTCGGC GATATAGGCG CCAGCAACCG CACCTGTGGC GCCGGTGATG 3960

CCGGCCACGA TGCGTCCGGC GTAGAGGATC GAGATCTCGA TCCCGCGAAA TTAATACGAC 4020

```
TCACTATAGG GAGACCACAA CGGTTTCCCT CTAGAAATAA TTTTGTTTAA CTTTAAGAAG    4080
GAGATATACA TATGGAACCG GTCGACCCGC GTCTGGAACC ATGGAAACAC CCCGGGTCCC    4140
AGCCGAAAAC CGCGTTCATC ACCAAAGCCC TAGGTATCTC TTACGGCCGT AAAAAACGTC    4200
GTCAGCGACG TCGTCCGCCG CAGGGATCTT CCATGGCCGG TGCTGGACGC ATTTACTATT    4260
CTCGCTTTGG TGACGAGGCA GCCAGATTTA GTACAACAGG GCATTACTCT GTAAGAGATC    4320
AGGACAGAGT GTATGCTGGT GTCTCATCCA CCTCTTCTGA TTTTAGAGAT CGCCCAGACG    4380
GAGTCTGGGT CGCATCCGAA GGACCTGAAG GAGACCCTGC AGGAAAAGAA GCCGAGCCAG    4440
CCCAGCCTGT CTCTTCTTTG CTCGGCTCCC CCGCCTGCGG TCCCATCAGA GCAGGCCTCG    4500
GTTGGGTACG GGACGGTCCT CGCTCGCACC CCTACAATTT TCCTGCAGGC TCGGGGGGCT    4560
CTATTCTCCG CTCTTCCTCC ACCCCGGTGC AGGGCACGGT ACCGGTGGAC TTGGCATCAA    4620
GGCAGGAAGA AGAGGAGCAG TCGCCCGACT CCACAGAGGA AGAACCAGTG ACTCTCCCAA    4680
GGCGCACCAC CAATGATGGA TTCCACCTGT TAAAGGCAGG AGGGTCATGC TTTGCTCTAA    4740
TTTCAGGAAC TGCTAACCAG GTAAAGTGCT ATCGCTTTCG GGTGAAAAAG AACCATAGAC    4800
ATCGCTACGA GAACTGCACC ACCACCTGGT TCACAGTTGC TGACAACGGT GCTGAAAGAC    4860
AAGGACAAGC ACAAATACTG ATCACCTTTG GATCGCCAAG TCAAAGGCAA GACTTTCTGA    4920
AACATGTACC ACTACCTCCT GGAATGAACA TTTCCGGCTT TACAGCCAGC TTGGACTTCT    4980
GATCACTGCC ATTGCCTTTT CTTCATCTGA CTGGTGTACT ATGCCAAATC TATGGTTTCT    5040
ATTGTTCTTG GGACTAGGAA GATCCGGCTG CTAACAAAGC CCGAAAGGAA GCTGAGTTGG    5100
CTGCTGCCAC CGCTGAGCAA TAACTAGCAT AACCCCTTGG GGCCTCTAAA CGGGTCTTGA    5160
GGGGTTTTTT GCTGAAAGGA GGAACTATAT CCGGATATCC ACAGGACGGG TGTGGTCGCC    5220
ATGATCGCGT AGTCGATAGT GGCTCCAAGT AGCGAAGCGA GCAGGACTGG GCGGCGGCCA    5280
AAGCGGTCGG ACAGTGCTCC GAGAACGGGT GCGCATAGAA ATTGCATCAA CGCATATAGC    5340
GCTAGCAGCA CGCCATAGTG ACTGGCGATG CTGTCGGAAT GGACGATATC CCGCAAGAGG    5400
CCCGGCAGTA CCGGCATAAC CAAGCCTATG CCTACAGCAT CCAGGGTGAC GGTGCCGAGG    5460
ATGACGATGA GCGCATTGTT AGATTTCATA CACGGTGCCT GACTGCGTTA GCAATTTAAC    5520
TGTGATAAAC TACCGCATTA AAGCTTATCG ATGATAAGCT GTCAAACATG AGAA          5574
```

(2) INFORMATION FOR SEQ ID NO:23:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(xi) SEQUENCE DESCRIPTION: SEQ ID NO:23:

GATCCCAGAC CCACCAGGTT 20

(2) INFORMATION FOR SEQ ID NO:24:

(i) SEQUENCE CHARACTERISTICS:

(A) LENGTH: 17 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:24:

GAACCTGGTG GGTCTGG 17

(2) INFORMATION FOR SEQ ID NO:25:

(i) SEQUENCE CHARACTERISTICS:

(A) LENGTH: 50 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:25:

CGTCCGCCGC AGGGATCGCA GACCCACCAG GTTTCTCTGT CTAAACAGGC 50

(2) INFORMATION FOR SEQ ID NO:26:

(i) SEQUENCE CHARACTERISTICS:

(A) LENGTH: 58 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:26:

CATGGCCTGT TTAGACAGAG AAACCTGGTG GGTCTGCGAT CCCTGCGGCG GACGACGT 58

(2) INFORMATION FOR SEQ ID NO:27:

(i) SEQUENCE CHARACTERISTICS:

(A) LENGTH: 48 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:27:

CATGTACGGC CGTAAAAAAC GTCGTCAGCG ACGTCGTCCG CCGGACAC 48

(2) INFORMATION FOR SEQ ID NO:28:

(i) SEQUENCE CHARACTERISTICS:

(A) LENGTH: 46 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:28:

CGGTGTCCGG CGGACGACGT CGCTGACGAC GTTTTTTACG GCCGTA 46

(2) INFORMATION FOR SEQ ID NO:29:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:29:

ATCATCGATA AGCTTTAATG CGGTAG 26

(2) INFORMATION FOR SEQ ID NO:30:

(i) SEQUENCE CHARACTERISTICS:

(A) LENGTH: 52 base pairs

(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:30:

ACTTTAAGAA GGAGATATAC ATATGTTCAT CACCAAAGCC CTAGGTATCT CT 52

(2) INFORMATION FOR SEQ ID NO:31:

(i) SEQUENCE CHARACTERISTICS:

(A) LENGTH: 51 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:31:

ACTTTAAGAA GGAGATATAC ATATGTACGG CCGTAAAAAA CGTCGTCAGC G 51

(2) INFORMATION FOR SEQ ID NO:32:

(i) SEQUENCE CHARACTERISTICS:

(A) LENGTH: 52 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:32:

AACGTCGTCA GCGACGTCGT CCGCCGGACA CCGGAAACCC CTGCCACACC AC 52

(2) INFORMATION FOR SEQ ID NO:33:

(i) SEQUENCE CHARACTERISTICS:

(A) LENGTH: 30 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:33:

CGAAAAGTGC CACCTGACGT CTAAGAAACC 30

(2) INFORMATION FOR SEQ ID NO:34:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:34:

CTCCCATGGC TAGCAACACT ACACCC 26

(2) INFORMATION FOR SEQ ID NO:35:

(i) SEQUENCE CHARACTERISTICS:

(A) LENGTH: 10 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:35:

GAAGATCTTC 10

(2) INFORMATION FOR SEQ ID NO:36:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:36:

CAGAGGAAGC CATGGTGACT CTCCCAA 27

(2) INFORMATION FOR SEQ ID NO:37:

(i) SEQUENCE CHARACTERISTICS:

(A) LENGTH: 27 base pairs

(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:37:

AAGGCAATGG ATCCGATCAG AAGTCCA 27

(2) INFORMATION FOR SEQ ID NO:38:

(i) SEQUENCE CHARACTERISTICS:

(A) LENGTH: 134 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:38:

```
Met Tyr Gly Arg Lys Lys Arg Arg Gln Arg Arg Arg Pro Pro Asp Thr
1               5                   10                  15

Gly Asn Pro Cys His Thr Thr Lys Leu Leu His Arg Asp Ser Val Asp
            20                  25                  30

Ser Ala Pro Ile Leu Thr Ala Phe Asn Ser Ser His Lys Gly Arg Ile
        35                  40                  45

Asn Cys Asn Ser Asn Thr Thr Pro Ile Val His Leu Lys Gly Asp Ala
    50                  55                  60

Asn Thr Leu Lys Cys Leu Arg Tyr Arg Phe Lys Lys His Cys Thr Leu
65                  70                  75                  80

Tyr Thr Ala Val Ser Ser Thr Trp His Trp Thr Gly His Asn Val Lys
                85                  90                  95

His Lys Ser Ala Ile Val Thr Leu Thr Tyr Asp Ser Glu Trp Gln Arg
            100                 105                 110
```

Asp Gln Phe Leu Ser Gln Val Lys Ile Pro Lys Thr Ile Thr Val Ser
115 120 125

Thr Gly Phe Met Ser Ile
130

(2) INFORMATION FOR SEQ ID NO:39:

(i) SEQUENCE CHARACTERISTICS:

(A) LENGTH: 55 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:39:

CATGTACGGC CGTAAAAAAC GTCGTCAGCG ACGTCGTCCG CTGAGTCAGG CCCAG 55

(2) INFORMATION FOR SEQ ID NO:40:

(i) SEQUENCE CHARACTERISTICS:

(A) LENGTH: 51 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:40:

CTGGGCCTGA CTCAGCGGAC GACGTCGCTG ACGACGTTTT TTACGGCCGT A 51

(2) INFORMATION FOR SEQ ID NO:41:

(i) SEQUENCE CHARACTERISTICS:

(A) LENGTH: 46 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:41:

TCCTTCCTGT CCGCTGGTCA GCGCCCGCGC CGCCTGTCCA CCTAAG 46

(2) INFORMATION FOR SEQ ID NO:42:

(i) SEQUENCE CHARACTERISTICS:

(A) LENGTH: 54 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:42:

AATTCTTAGG TGGACAGGCG GCGCGGGCGC TGACCAGCGG ACAGGAAGGA CATG 54

(2) INFORMATION FOR SEQ ID NO:43:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:43:

GGGGACTTTC CGCTGGGGAC TTTCCACGGG GGACTTTCC 39

(2) INFORMATION FOR SEQ ID NO:44:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:44:

GGAAAGTCCC CCGTGGAAAG TCCCCAGCGG AAAGTCCCC 39

(2) INFORMATION FOR SEQ ID NO:45:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:45:

GTCTACTTTC CGCTGTCTAC TTTCCACGGT CTACTTTCC 39

(2) INFORMATION FOR SEQ ID NO:46:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:46:

GGAAAGTAGA CCGTGGAAAG TAGACAGCGG AAAGTAGAC 39

(2) INFORMATION FOR SEQ ID NO:47:

(i) SEQUENCE CHARACTERISTICS:

(A) LENGTH: 12 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:47:

```
Tyr Gly Arg Lys Lys Arg Arg Gln Arg Arg Arg Pro
1               5                   10
```

(2) INFORMATION FOR SEQ ID NO:48:

(i) SEQUENCE CHARACTERISTICS:

(A) LENGTH: 26 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:48:

```
Tyr Gly Arg Lys Lys Arg Arg Gln Arg Arg Arg Pro Pro Gln Gly Ser
1               5                   10                  15

Gln Thr His Gln Val Ser Leu Ser Lys Gln
            20                  25
```

(2) INFORMATION FOR SEQ ID NO:49:

(i) SEQUENCE CHARACTERISTICS:

(A) LENGTH: 35 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:49:

```
Phe Ile Thr Lys Ala Leu Gly Ile Ser Tyr Gly Arg Lys Lys Arg Arg
1               5                   10                  15

Gln Arg Arg Arg Pro Pro Gln Gly Ser Gln Thr His Gln Val Ser Leu
            20                  25                  30

Ser Lys Gln
        35
```

(2) INFORMATION FOR SEQ ID NO:50:

(i) SEQUENCE CHARACTERISTICS:

(A) LENGTH: 21 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:50:

```
Phe Ile Thr Lys Ala Leu Gly Ile Ser Tyr Gly Arg Lys Lys Arg Arg
1               5                   10                  15

Gln Arg Arg Arg Pro
            20
```

(2) INFORMATION FOR SEQ ID NO:51:

(i) SEQUENCE CHARACTERISTICS:

(A) LENGTH: 121 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:51:

```
Pro Asp Thr Gly Asn Pro Cys His Thr Thr Lys Leu Leu His Arg Asp
1               5                   10                  15

Ser Val Asp Ser Ala Pro Ile Leu Thr Ala Phe Asn Ser Ser His Lys
            20                  25                  30

Gly Arg Ile Asn Cys Asn Ser Asn Thr Thr Pro Ile Val His Leu Lys
        35                  40                  45

Gly Asp Ala Asn Thr Leu Lys Cys Leu Arg Tyr Arg Phe Lys Lys His
    50                  55                  60

Cys Thr Leu Tyr Thr Ala Val Ser Ser Thr Trp His Trp Thr Gly His
65                  70                  75                  80

Asn Val Lys His Lys Ser Ala Ile Val Thr Leu Thr Tyr Asp Ser Glu
                85                  90                  95

Trp Gln Arg Asp Gln Phe Leu Ser Gln Val Lys Ile Pro Lys Thr Ile
            100                 105                 110

Thr Val Ser Thr Gly Phe Met Ser Ile
        115                 120
```

(2) INFORMATION FOR SEQ ID NO:52:

(i) SEQUENCE CHARACTERISTICS:

(A) LENGTH: 15 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:52:

```
Gly Arg Lys Lys Arg Arg Gln Arg Arg Arg Pro Pro Gln Gly Ser
1               5                   10                  15
```

(2) INFORMATION FOR SEQ ID NO:53:

(i) SEQUENCE CHARACTERISTICS:

(A) LENGTH: 25 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:53:

```
Phe Ile Thr Lys Ala Leu Gly Ile Ser Tyr Gly Arg Lys Lys Arg Arg
1               5                   10                  15

Gln Arg Arg Arg Pro Pro Gln Gly Ser
            20                  25
```

(2) INFORMATION FOR SEQ ID NO:54:

(i) SEQUENCE CHARACTERISTICS:

(A) LENGTH: 85 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:54:

```
Cys Asn Ser Asn Thr Thr Pro Ile Val His Leu Lys Gly Asp Ala Asn
1               5                   10                  15

Thr Leu Lys Cys Leu Arg Tyr Arg Phe Lys Lys His Cys Thr Leu Tyr
            20                  25                  30

Thr Ala Val Ser Ser Thr Trp His Trp Thr Gly His Asn Val Lys His
        35                  40                  45

Lys Ser Ala Ile Val Thr Leu Thr Tyr Asp Ser Glu Trp Gln Arg Asp
    50                  55                  60
```

```
Gln Phe Leu Ser Gln Val Lys Ile Pro Lys Thr Ile Thr Val Ser Thr
65                  70                  75                  80

Gly Phe Met Ser Ile
                85
```

(2) INFORMATION FOR SEQ ID NO:55:

(i) SEQUENCE CHARACTERISTICS:

(A) LENGTH: 121 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:55:

```
Pro Asp Thr Gly Asn Pro Cys His Thr Thr Lys Leu Leu His Arg Asp
1               5                   10                  15

Ser Val Asp Ser Ala Pro Ile Leu Thr Ala Phe Asn Ser Ser His Lys
            20                  25                  30

Gly Arg Ile Asn Cys Asn Ser Asn Thr Thr Pro Ile Val His Leu Lys
        35                  40                  45

Gly Asp Ala Asn Thr Leu Lys Ser Leu Arg Tyr Arg Phe Lys Lys His
    50                  55                  60

Ser Thr Leu Tyr Thr Ala Val Ser Ser Thr Trp His Trp Thr Gly His
65                  70                  75                  80

Asn Val Lys His Lys Ser Ala Ile Val Thr Leu Thr Tyr Asp Ser Glu
                85                  90                  95

Trp Gln Arg Asp Gln Phe Leu Ser Gln Val Lys Ile Pro Lys Thr Ile
            100                 105                 110

Thr Val Ser Thr Gly Phe Met Ser Ile
        115                 120
```

(2) INFORMATION FOR SEQ ID NO:56:

(i) SEQUENCE CHARACTERISTICS:

(A) LENGTH: 161 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:56:

```
Leu Gly Trp Val Arg Asp Gly Pro Arg Ser His Pro Tyr Asn Phe Pro
1               5                   10                  15

Ala Gly Ser Gly Gly Ser Ile Leu Arg Ser Ser Ser Thr Pro Val Gln
            20                  25                  30

Gly Thr Val Pro Val Asp Leu Ala Ser Arg Gln Glu Glu Glu Glu Gln
        35                  40                  45

Ser Pro Asp Ser Thr Glu Glu Glu Pro Val Thr Leu Pro Arg Arg Thr
    50                  55                  60

Thr Asn Asp Gly Phe His Leu Leu Lys Ala Gly Gly Ser Cys Phe Ala
65                  70                  75                  80

Leu Ile Ser Gly Thr Ala Asn Gln Val Lys Cys Tyr Arg Phe Arg Val
                85                  90                  95

Lys Lys Asn His Arg His Arg Tyr Glu Asn Cys Thr Thr Thr Trp Phe
            100                 105                 110

Thr Val Ala Asp Asn Gly Ala Glu Arg Gln Gly Gln Ala Gln Ile Leu
        115                 120                 125

Ile Thr Phe Gly Ser Pro Ser Gln Arg Gln Asp Phe Leu Lys His Val
    130                 135                 140

Pro Leu Pro Pro Gly Met Asn Ile Ser Gly Phe Thr Ala Ser Leu Asp
145                 150                 155                 160

Phe
```

(2) INFORMATION FOR SEQ ID NO:57:

(i) SEQUENCE CHARACTERISTICS:

(A) LENGTH: 249 amino acids

(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:57:

```
Met Ala Gly Ala Gly Arg Ile Tyr Tyr Ser Arg Phe Gly Asp Glu Ala
1               5                   10                  15

Ala Arg Phe Ser Thr Thr Gly His Tyr Ser Val Arg Asp Gln Asp Arg
            20                  25                  30

Val Tyr Ala Gly Val Ser Ser Thr Ser Ser Asp Phe Arg Asp Arg Pro
        35                  40                  45

Asp Gly Val Trp Val Ala Ser Glu Gly Pro Glu Gly Asp Pro Ala Gly
    50                  55                  60

Lys Glu Ala Glu Pro Ala Gln Pro Val Ser Ser Leu Leu Gly Ser Pro
65                  70                  75                  80

Ala Cys Gly Pro Ile Arg Ala Gly Leu Gly Trp Val Arg Asp Gly Pro
                85                  90                  95

Arg Ser His Pro Tyr Asn Phe Pro Ala Gly Ser Gly Gly Ser Ile Leu
            100                 105                 110

Arg Ser Ser Ser Thr Pro Val Gln Gly Thr Val Pro Val Asp Leu Ala
        115                 120                 125

Ser Arg Gln Glu Glu Glu Glu Gln Ser Pro Asp Ser Thr Glu Glu Glu
    130                 135                 140

Pro Val Thr Leu Pro Arg Arg Thr Thr Asn Asp Gly Phe His Leu Leu
145                 150                 155                 160

Lys Ala Gly Gly Ser Cys Phe Ala Leu Ile Ser Gly Thr Ala Asn Gln
                165                 170                 175
```

```
Val Lys Cys Tyr Arg Phe Arg Val Lys Lys Asn His Arg His Arg Tyr
            180                 185                 190

Glu Asn Cys Thr Thr Thr Trp Phe Thr Val Ala Asp Asn Gly Ala Glu
        195                 200                 205

Arg Gln Gly Gln Ala Gln Ile Leu Ile Thr Phe Gly Ser Pro Ser Gln
    210                 215                 220

Arg Gln Asp Phe Leu Lys His Val Pro Leu Pro Pro Gly Met Asn Ile
225                 230                 235                 240

Ser Gly Phe Thr Ala Ser Leu Asp Phe
                245
```

(2) INFORMATION FOR SEQ ID NO:58:

(i) SEQUENCE CHARACTERISTICS:

(A) LENGTH: 385 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:58:

```
Met Tyr Gly Arg Lys Lys Arg Arg Gln Arg Arg Arg Pro Leu Ser Gln
1               5                   10                  15

Ala Gln Leu Met Pro Ser Pro Pro Met Pro Val Pro Pro Ala Ala Leu
            20                  25                  30

Phe Asn Arg Leu Leu Asp Asp Leu Gly Phe Ser Ala Gly Pro Ala Leu
        35                  40                  45

Cys Thr Met Leu Asp Thr Trp Asn Glu Asp Leu Phe Ser Gly Phe Pro
    50                  55                  60

Thr Asn Ala Asp Met Tyr Arg Glu Cys Lys Phe Leu Ser Thr Leu Pro
65                  70                  75                  80

Ser Asp Val Ile Asp Trp Gly Asp Ala His Val Pro Glu Arg Ser Pro
                85                  90                  95
```

Ile Asp Ile Arg Ala His Gly Asp Val Ala Phe Pro Thr Leu Pro Ala
100 105 110

Thr Arg Asp Glu Leu Pro Ser Tyr Tyr Glu Ala Met Ala Gln Phe Phe
115 120 125

Arg Gly Glu Leu Arg Ala Arg Glu Glu Ser Tyr Arg Thr Val Leu Ala
130 135 140

Asn Phe Cys Ser Ala Leu Tyr Arg Tyr Leu Arg Ala Ser Val Arg Gln
145 150 155 160

Leu His Arg Gln Ala His Met Arg Gly Arg Asn Arg Asp Leu Arg Glu
165 170 175

Met Leu Arg Thr Thr Ile Ala Asp Arg Tyr Tyr Arg Glu Thr Ala Arg
180 185 190

Leu Ala Arg Val Leu Phe Leu His Leu Tyr Leu Phe Leu Ser Arg Glu
195 200 205

Ile Leu Trp Ala Ala Tyr Ala Glu Gln Met Met Arg Pro Asp Leu Phe
210 215 220

Asp Gly Leu Cys Cys Asp Leu Glu Ser Trp Arg Gln Leu Ala Cys Leu
225 230 235 240

Phe Gln Pro Leu Met Phe Ile Asn Gly Ser Leu Thr Val Arg Gly Val
245 250 255

Pro Val Glu Ala Arg Arg Leu Arg Glu Leu Asn His Ile Arg Glu His
260 265 270

Leu Asn Leu Pro Leu Val Arg Ser Ala Ala Ala Glu Glu Pro Gly Ala
275 280 285

Pro Leu Thr Thr Pro Pro Val Leu Gln Gly Asn Gln Ala Arg Ser Ser
290 295 300

Gly Tyr Phe Met Leu Leu Ile Arg Ala Lys Leu Asp Ser Tyr Ser Ser
305 310 315 320

Val Ala Thr Ser Glu Gly Glu Ser Val Met Arg Glu His Ala Tyr Ser
325 330 335

Arg Gly Arg Thr Arg Asn Asn Tyr Gly Ser Thr Ile Glu Gly Leu Leu
340 345 350

Asp Leu Pro Asp Asp Asp Asp Ala Pro Ala Glu Ala Gly Leu Val Ala
355 360 365

Pro Arg Met Ser Phe Leu Ser Ala Gly Gln Arg Pro Arg Arg Leu Ser
370 375 380

Thr
385

(2) INFORMATION FOR SEQ ID NO:59:

(i) SEQUENCE CHARACTERISTICS:

(A) LENGTH: 148 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:59:

Met Tyr Gly Arg Lys Lys Arg Arg Gln Arg Arg Arg Pro Pro Gln Gly
1 5 10 15

Ser Gln Thr His Gln Val Ser Leu Ser Lys Gln Pro Asp Thr Gly Asn
20 25 30

Pro Cys His Thr Thr Lys Leu Leu His Arg Asp Ser Val Asp Ser Ala
35 40 45

Pro Ile Leu Thr Ala Phe Asn Ser Ser His Lys Gly Arg Ile Asn Cys
50 55 60

Asn Ser Asn Thr Thr Pro Ile Val His Leu Lys Gly Asp Ala Asn Thr
65 70 75 80

Leu Lys Cys Leu Arg Tyr Arg Phe Lys Lys His Cys Thr Leu Tyr Thr
85 90 95

Ala Val Ser Ser Thr Trp His Trp Thr Gly His Asn Val Lys His Lys
100 105 110

Ser Ala Ile Val Thr Leu Thr Tyr Asp Ser Glu Trp Gln Arg Asp Gln
115 120 125

Phe Leu Ser Gln Val Lys Ile Pro Lys Thr Ile Thr Val Ser Thr Gly
130 135 140

Phe Met Ser Ile
145

(2) INFORMATION FOR SEQ ID NO:60:

(i) SEQUENCE CHARACTERISTICS:

(A) LENGTH: 157 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:60:

Met Phe Ile Thr Lys Ala Leu Gly Ile Ser Tyr Gly Arg Lys Lys Arg
1 5 10 15

Arg Gln Arg Arg Arg Pro Pro Gln Gly Ser Gln Thr His Gln Val Ser
20 25 30

Leu Ser Lys Gln Pro Asp Thr Gly Asn Pro Cys His Thr Thr Lys Leu
35 40 45

Leu His Arg Asp Ser Val Asp Ser Ala Pro Ile Leu Thr Ala Phe Asn
50 55 60

Ser Ser His Lys Gly Arg Ile Asn Cys Asn Ser Asn Thr Thr Pro Ile
65 70 75 80

Val His Leu Lys Gly Asp Ala Asn Thr Leu Lys Cys Leu Arg Tyr Arg
85 90 95

Phe Lys Lys His Cys Thr Leu Tyr Thr Ala Val Ser Ser Thr Trp His
100 105 110

Trp Thr Gly His Asn Val Lys His Lys Ser Ala Ile Val Thr Leu Thr
115 120 125

Tyr Asp Ser Glu Trp Gln Arg Asp Gln Phe Leu Ser Gln Val Lys Ile
130 135 140

Pro Lys Thr Ile Thr Val Ser Thr Gly Phe Met Ser Ile
145 150 155

(2) INFORMATION FOR SEQ ID NO:61:

(i) SEQUENCE CHARACTERISTICS:

(A) LENGTH: 177 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:61:

Met Gly Arg Lys Lys Arg Arg Gln Arg Arg Arg Pro Pro Gln Gly Ser
1 5 10 15

Leu Gly Trp Val Arg Asp Gly Pro Arg Ser His Pro Tyr Asn Phe Pro
20 25 30

Ala Gly Ser Gly Gly Ser Ile Leu Arg Ser Ser Ser Thr Pro Val Gln
35 40 45

Gly Thr Val Pro Val Asp Leu Ala Ser Arg Gln Glu Glu Glu Glu Gln
50 55 60

Ser Pro Asp Ser Thr Glu Glu Glu Pro Val Thr Leu Pro Arg Arg Thr
65 70 75 80

Thr Asn Asp Gly Phe His Leu Leu Lys Ala Gly Gly Ser Cys Phe Ala
85 90 95

```
Leu Ile Ser Gly Thr Ala Asn Gln Val Lys Cys Tyr Arg Phe Arg Val
            100                 105                 110

Lys Lys Asn His Arg His Arg Tyr Glu Asn Cys Thr Thr Thr Trp Phe
        115                 120                 125

Thr Val Ala Asp Asn Gly Ala Glu Arg Gln Gly Gln Ala Gln Ile Leu
    130                 135                 140

Ile Thr Phe Gly Ser Pro Ser Gln Arg Gln Asp Phe Leu Lys His Val
145                 150                 155                 160

Pro Leu Pro Pro Gly Met Asn Ile Ser Gly Phe Thr Ala Ser Leu Asp
                165                 170                 175

Phe
```

(2) INFORMATION FOR SEQ ID NO:62:

(i) SEQUENCE CHARACTERISTICS:

(A) LENGTH: 187 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:62:

```
Met Phe Ile Thr Lys Ala Leu Gly Ile Ser Tyr Gly Arg Lys Lys Arg
1               5                   10                  15

Arg Gln Arg Arg Arg Pro Pro Gln Gly Ser Leu Gly Trp Val Arg Asp
            20                  25                  30

Gly Pro Arg Ser His Pro Tyr Asn Phe Pro Ala Gly Ser Gly Gly Ser
        35                  40                  45

Ile Leu Arg Ser Ser Ser Thr Pro Val Gln Gly Thr Val Pro Val Asp
    50                  55                  60

Leu Ala Ser Arg Gln Glu Glu Glu Glu Gln Ser Pro Asp Ser Thr Glu
65                  70                  75                  80
```

```
Glu Glu Pro Val Thr Leu Pro Arg Arg Thr Thr Asn Asp Gly Phe His
                85                  90                  95

Leu Leu Lys Ala Gly Gly Ser Cys Phe Ala Leu Ile Ser Gly Thr Ala
            100                 105                 110

Asn Gln Val Lys Cys Tyr Arg Phe Arg Val Lys Lys Asn His Arg His
        115                 120                 125

Arg Tyr Glu Asn Cys Thr Thr Thr Trp Phe Thr Val Ala Asp Asn Gly
    130                 135                 140

Ala Glu Arg Gln Gly Gln Ala Gln Ile Leu Ile Thr Phe Gly Ser Pro
145                 150                 155                 160

Ser Gln Arg Gln Asp Phe Leu Lys His Val Pro Leu Pro Pro Gly Met
                165                 170                 175

Asn Ile Ser Gly Phe Thr Ala Ser Leu Asp Phe
            180                 185
```

(2) INFORMATION FOR SEQ ID NO:63:

(i) SEQUENCE CHARACTERISTICS:

(A) LENGTH: 143 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:63:

```
Met Phe Ile Thr Lys Ala Leu Gly Ile Ser Tyr Gly Arg Lys Lys Arg
1               5                   10                  15

Arg Gln Arg Arg Arg Pro Pro Asp Thr Gly Asn Pro Cys His Thr Thr
            20                  25                  30

Lys Leu Leu His Arg Asp Ser Val Asp Ser Ala Pro Ile Leu Thr Ala
        35                  40                  45
```

```
Phe Asn Ser Ser His Lys Gly Arg Ile Asn Cys Asn Ser Asn Thr Thr
    50                  55                  60

Pro Ile Val His Leu Lys Gly Asp Ala Asn Thr Leu Lys Cys Leu Arg
65                  70                  75                  80

Tyr Arg Phe Lys Lys His Cys Thr Leu Tyr Thr Ala Val Ser Ser Thr
                85                  90                  95

Trp His Trp Thr Gly His Asn Val Lys His Lys Ser Ala Ile Val Thr
            100                 105                 110

Leu Thr Tyr Asp Ser Glu Trp Gln Arg Asp Gln Phe Leu Ser Gln Val
        115                 120                 125

Lys Ile Pro Lys Thr Ile Thr Val Ser Thr Gly Phe Met Ser Ile
    130                 135                 140
```